(19) Europäisches Patentamt
European Patent Office
Office européen des brevets

(11) EP 4 458 829 A1

(12) EUROPEAN PATENT APPLICATION

(43) Date of publication:
**06.11.2024 Bulletin 2024/45**

(21) Application number: **24172844.3**

(22) Date of filing: **26.04.2024**

(51) International Patent Classification (IPC):
***C07D 495/04*** *(2006.01)* ***H10K 30/30*** *(2023.01)*
***H10K 39/32*** *(2023.01)* ***H10K 85/60*** *(2023.01)*

(52) Cooperative Patent Classification (CPC):
**C07D 495/04; H10K 30/30; H10K 39/32; H10K 85/322; H10K 85/40; H10K 85/657; H10K 85/6576**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.04.2023 KR 20230056449**

(71) Applicant: **Samsung Electronics Co., Ltd.**
**Suwon-si, Gyeonggi-do 16677 (KR)**

(72) Inventors:
- **LIM, Younhee**
  **16678 Suwon-si (KR)**
- **KIM, Hyeong-Ju**
  **16678 Suwon-si (KR)**
- **FANG, Feifei**
  **16678 Suwon-si (KR)**
- **YI, Jeoung In**
  **16678 Suwon-si (KR)**
- **PARK, Kyung Bae**
  **16678 Suwon-si (KR)**
- **PARK, Jeong Il**
  **16678 Suwon-si (KR)**
- **CHOI, Tae Jin**
  **16678 Suwon-si (KR)**

(74) Representative: **Elkington and Fife LLP**
**Prospect House**
**8 Pembroke Road**
**Sevenoaks, Kent TN13 1XR (GB)**

(54) **4H,8H-THIENO[3',2':5,6]PYRIDO[3,2,1-DE]ACRIDINE DERIVATIVES DERIVATIVES WITH HIGH EXTINCTION COEFFICIENTS FOR USE AS MATERIALS IN PHOTOELECTRONIC DEVICES AND IMAGE SENSORS**

(57) A compound represented by Chemical Formula 1 is provided.

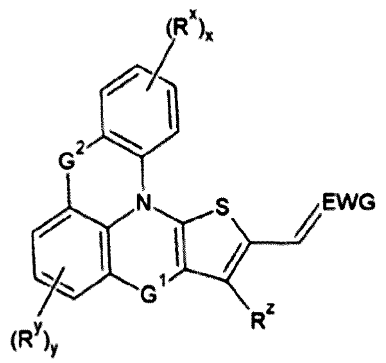

The compound has a reorganization energy of less than about 0.191 eV and a maximum absorption wavelength value calculated by density functional theory (DFT) of less than or equal to about 500 nm.
The compound is for use as materials in photoelectronic devices and image sensors.

FIG. 1

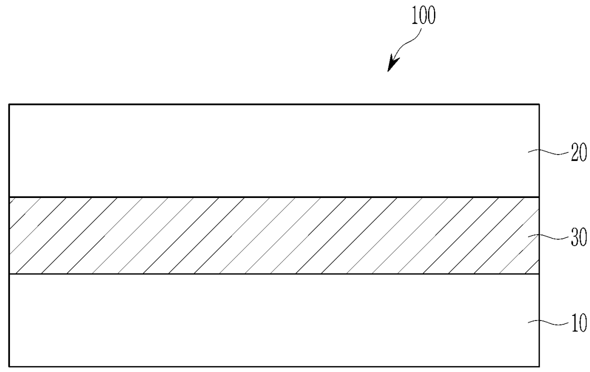


EP 4 458 829 A1

## Description

### FIELD OF THE INVENTION

Example embodiments relate to compounds, photoelectric devices, image sensors, and electronic devices.

### BACKGROUND OF THE INVENTION

A photoelectric device may convert light into an electrical signal using photoelectric effects. A photoelectric device may include a photodiode, a phototransistor, etc., and may be applied to an image sensor, etc.

An image sensor including a photodiode may require high resolution and thus a small pixel. At present, a silicon photodiode is widely used. In some cases, a silicon photodiode exhibits a problem of deteriorated sensitivity because of a relatively small absorption area due to relatively small pixels. Accordingly, an organic material that is capable of replacing silicon has been researched.

An organic material may have a relatively high extinction coefficient and may selectively absorb light in a particular wavelength region depending on a molecular structure, and thus may simultaneously replace a photodiode (e.g., a silicon photodiode) and a color filter and resultantly improve sensitivity and contribute to relatively high integration.

Therefore, interest in organic materials that can be used in these sensors is increasing.

### SUMMARY OF THE INVENTION

Some example embodiments provide a compound that is capable of selectively absorbing light in a green wavelength range and has excellent light absorption characteristics and thermal stability.

Some example embodiments provide a photoelectric device that is configured to selectively absorb light in a green wavelength range and maintain excellent efficiency even in processes under high temperature conditions.

Some example embodiments provide an image sensor including the photoelectric device.

Some example embodiments provide an electronic device including the image sensor.

According to some example embodiments, a compound is represented by Chemical Formula 1, wherein the compound has a reorganization energy of less than or equal to about 0.191 eV, and the compound has a maximum absorption wavelength calculated based on density functional theory (DFT) of less than or equal to about 500 nm.

[Chemical Formula 1]

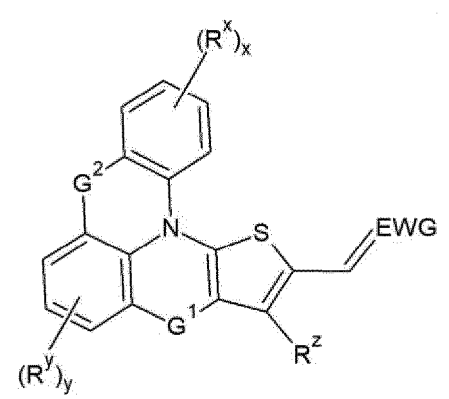

In Chemical Formula 1,

$G^1$ is a single bond,, O, S, Se, Te, S(=O), $S(=O)_2$, $NR^a$, $BR^b$, -$SiR^cR^d$-, - $SiR^{cc}R^{dd}$-, -$GeR^eR^f$-, -$GeR^{ee}R^{ff}$-, -$(CR^gR^h)_{n1}$-, -$(CR^{gg}R^{hh})$-, -(C($R^i$)=C($R^j$))-, or - (C($R^{ii}$)=C($R^{jj}$))- (wherein $R^a$, $R^b$, $R^c$, $R^d$, $R^e$, $R^f$, $R^g$, $R^h$, $R^i$, and $R^j$ are each independently hydrogen, deuterium, a halogen, a C1 to C10 alkyl group, a C1 to C10 haloalkyl group, a C1 to C10 alkoxy group, or a C6 to C20 aryl group, each pair of $R^{cc}$ and $R^{dd}$, $R^{ee}$ and $R^{ff}$, $R^{gg}$ and $R^{hh}$, or $R^{ii}$ and $R^{jj}$ are linked to each other to form a ring structure, and n1 is 1 or 2),

$G^2$ is O, S, Se, Te, S(=O), $S(=O)_2$, $NR^a$, $BR^b$, -$SiR^cR^d$-, -$SiR^{cc}R^{dd}$-, -$GeR^eR^f$-, - $GeR^{ee}R^{ff}$-, -$(CR^gR^h)_{n1}$-, -$(CR^{gg}R^{hh})$-, –(C($R^i$)=C($R^j$))-, or -(C($R^{ii}$)=C($R^{jj}$))- (wherein $R^a$, $R^b$, $R^c$, $R^d$, $R^e$, $R^f$, $R^g$, $R^h$, $R^i$, and $R^j$ are each independently hydrogen, deuterium, a halogen, a C1 to C10 alkyl group, a C1 to C10 haloalkyl group, a C1 to C10 alkoxy group, or a C6 to C20 aryl group, each pair of $R^{cc}$ and $R^{dd}$, $R^{ee}$ and $R^{ff}$, $R^{gg}$ and $R^{hh}$, or $R^{ii}$ and $R^{jj}$ are linked to each other

to form a ring structure, and n1 is 1 or 2),

$R^x$, $R^y$, and $R^z$ are each independently hydrogen, deuterium, a halogen, a C1 to C10 alkyl group, a C1 to C10 haloalkyl group, a C1 to C10 alkoxy group, or a C6 to C20 aryl group, x is an integer of 0 to 4, and y is an integer of 0 to 3, and

EWG means an acceptor moiety including at least one electron withdrawing group.

In Chemical Formula 1, at least one -CH= of a benzene ring of a $G^2$-containing cyclic group may be replaced by -N=.

In Chemical Formula 1, nitrogen (N) may be contained at 1st position of the $G^2$-containing cyclic group.

In Chemical Formula 1, $R^x$, $R^y$, and $R^z$ may each independently be hydrogen or an electron donating group selected from a C1 to C10 alkyl group and a C1 to C10 alkoxy group.

In Chemical Formula 1, the ring structure may be a substituted or unsubstituted C5 to C30 hydrocarbon ring group or a substituted or unsubstituted C2 to C30 heterocyclic group.

In Chemical Formula 1, the ring structure may include a moiety represented by Chemical Formula 2.

[Chemical Formula 2]

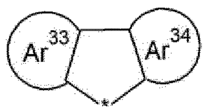

In Chemical Formula 2,

$Ar^{33}$ and $Ar^{34}$ are each independently a substituted or unsubstituted C6 to C30 arene group, a substituted or unsubstituted C3 to C30 heteroarene group, or a condensed ring thereof, and

* is a point linked to Chemical Formula 1.

In Chemical Formula 1, each ring structure may include one of the moieties represented by Chemical Formula 3.

[Chemical Formula 3]

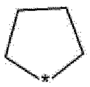
(1)

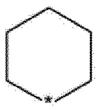
(2)

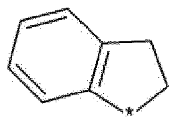
(3)

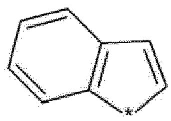
(4)

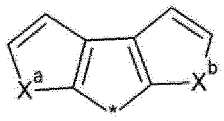
(5)

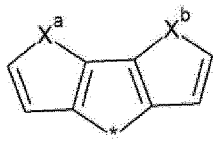
(6)

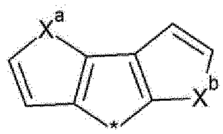
(7)

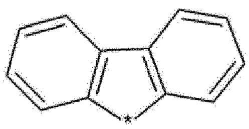
(8)

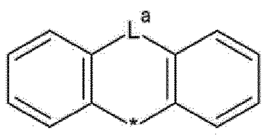
(9)

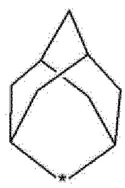
(10)

In Chemical Formula 3,

$X^a$ and $X^b$ are each independently -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$,

-$GeR^dR^e$-, or -$GeR^{dd}R^{ee}$- (wherein $R^{a1}$, $R^{a2}$, $R^b$, $R^c$, $R^d$, and $R^e$ are each independently hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and each pair of $R^{bb}$ and $R^{cc}$ or $R^{dd}$ and $R^{ee}$ may be linked to each other to form a ring structure),

$L^a$ is -O-, -S-, -Se-, -Te-, -$NR^{a1}$-, -$BR^{a2}$-, -$SiR^bR^c$-, -$GeR^dR^e$-, -$(CR^fR^g)_{n1}$-, - ($C(R^p)$=N))-, or a single bond (wherein $R^{a1}$, $R^{a2}$, $R^b$, $R^c$, $R^d$, $R^e$, $R^f$, $R^g$, and $R^p$ are each independently hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and n1 of -$(CR^fR^g)_{n1}$- is 1 or 2),

at least one hydrogen of each ring is optionally replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group, and

* is a linking portion linked to Chemical Formula 1.

In Chemical Formula 3, at least one CH present in at least one aromatic ring of moiety (3), (4), (5), (6), (7), (8), or (9) may be replaced by N.

In Chemical Formula 1, EWG may be a substituted or unsubstituted C6 to C30 hydrocarbon cyclic group having at least one functional group of C=O, C=S, C=Se, C=Te, or C=$CR^pR^q$ (wherein $R^p$ and $R^q$ are each independently hydrogen, deuterium, a substituted or unsubstituted C1 to C10 alkyl group, a cyano group, or a cyano-containing group); a substituted or unsubstituted C2 to C30 heterocyclic group having at least one functional group of C=O, C=S, C=Se, C=Te, or C=$CR^pR^q$ (wherein $R^p$ and $R^q$ are each independently hydrogen, deuterium, a substituted or unsubstituted C1 to C10 alkyl group, a cyano group, or a cyano-containing group); or a fused ring thereof; $CR^pR^q$ (wherein $R^p$ and $R^q$ are each independently hydrogen, deuterium, a substituted or unsubstituted C1 to C10 alkyl group, a cyano group, or a cyano-containing group); or a C2 to C20 alkyl group having at least one functional group of C=O, C=S, C=Se, C=Te, and C=$CR^pR^q$ (wherein $R^p$ and $R^q$ are each independently hydrogen, deuterium, a substituted or unsubstituted C1 to C10 alkyl group, a cyano group, or a cyano-containing group).

In Chemical Formula 1, EWG may be a cyclic group represented by Chemical Formula 4.

[Chemical Formula 4]

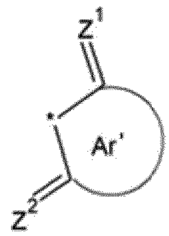

In Chemical Formula 4,

Ar' is a substituted or unsubstituted C6 to C30 aryl group or a substituted or unsubstituted C3 to C30 heteroaryl group,

$Z^1$ and $Z^2$ are each independently O, S, Se, Te, or $CR^aR^b$ (wherein $R^a$ and $R^b$ are each independently hydrogen, deuterium, a substituted or unsubstituted C1 to C10 alkyl group, a cyano group, or a cyano-containing group), and

* is a linking portion linked to Chemical Formula 1.

In Chemical Formula 1, EWG may be a cyclic group represented by any one of Chemical Formula 5A to Chemical Formula 5H.

[Chemical Formula 5A]

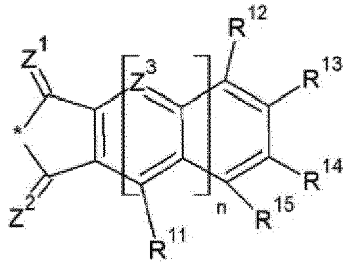

In Chemical Formula 5A,

$Z^1$ and $Z^2$ are each independently O, S, Se, Te, or $CR^aR^b$ (wherein $R^a$ and $R^b$ are each independently hydrogen, deuterium, a substituted or unsubstituted C1 to C10 alkyl group, a cyano group, or a cyano-containing group),
$Z^3$ is N or $CR^c$ (wherein $R^c$ is hydrogen, deuterium, or a substituted or unsubstituted C1 to C10 alkyl group),
$R^{11}$, $R^{12}$, $R^{13}$, $R^{14}$, and $R^{15}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), a cyano-containing group, or any combination thereof, wherein $R^{12}$ and $R^{13}$ and $R^{14}$ and $R^{15}$ are each independently present or are linked to each other to form a fused aromatic ring,
n is 0 or 1, and
* is a linking portion linked to Chemical Formula 1.

[Chemical Formula 5B]

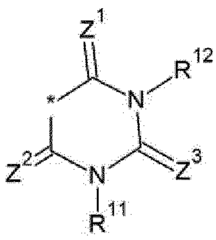

In Chemical Formula 5B,

$Z^1$ and $Z^2$ are each independently O, S, Se, Te, or $CR^aR^b$ (wherein $R^a$ and $R^b$ are each independently hydrogen, deuterium, a substituted or unsubstituted C1 to C10 alkyl group, a cyano group, or a cyano-containing group),
$Z^3$ is O, S, Se, Te, or $CR^aR^b$ (wherein $R^a$ and $R^b$ are each independently hydrogen, deuterium, a substituted or unsubstituted C1 to C10 alkyl group, a cyano group, or a cyano-containing group),
$R^{11}$ and $R^{12}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* is a linking portion linked to Chemical Formula 1.

[Chemical Formula 5C]

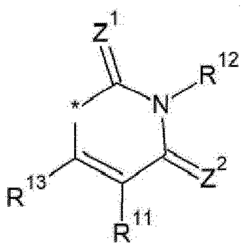

In Chemical Formula 5C,

$Z^1$ and $Z^2$ are each independently O, S, Se, Te, or $CR^aR^b$ (wherein $R^a$ and $R^b$ are each independently hydrogen, deuterium, a substituted or unsubstituted C1 to C10 alkyl group, a cyano group, or a cyano-containing group),
$R^{11}$, $R^{12}$, and $R^{13}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* is a linking portion linked to Chemical Formula 1.

[Chemical Formula 5D]

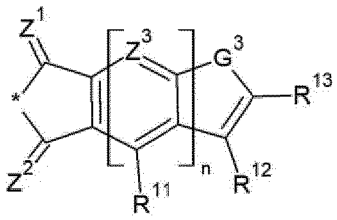

In Chemical Formula 5D,

$Z^1$ and $Z^2$ are each independently O, S, Se, Te, or $CR^aR^b$ (wherein $R^a$ and $R^b$ are each independently hydrogen, deuterium, a substituted or unsubstituted C1 to C10 alkyl group, a cyano group, or a cyano-containing group),
$Z^3$ is N or $CR^c$ (wherein $R^c$ is hydrogen or a substituted or unsubstituted C1 to C10 alkyl group),
$G^3$ is O, S, Se, Te, $SiR^xR^y$, or $GeR^zR^w$ (wherein $R^x$, $R^y$, $R^z$, and $R^w$ are each independently hydrogen, deuterium, a halogen, a cyano group, a substituted or unsubstituted C1 to C20 alkyl group, or a substituted or unsubstituted C6 to C20 aryl group),
$R^{11}$, $R^{12}$, and $R^{13}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof,
n is 0 or 1, and
* is a linking portion linked to Chemical Formula 1.

[Chemical Formula 5E]

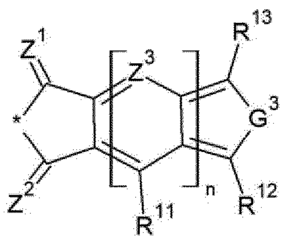

In Chemical Formula 5E,

$Z^1$ and $Z^2$ are each independently O, S, Se, Te, or $CR^aR^b$ (wherein $R^a$ and $R^b$ are each independently hydrogen, deuterium, a substituted or unsubstituted C1 to C10 alkyl group, a cyano group, or a cyano-containing group),
$Z^3$ is N or $CR^c$ (wherein $R^c$ is hydrogen or a substituted or unsubstituted C1 to C10 alkyl group),
$G^3$ is O, S, Se, Te, $SiR^xR^y$, or $GeR^zR^w$ (wherein $R^x$, $R^y$, $R^z$, and $R^w$ are each independently hydrogen, deuterium, a halogen, a cyano group, a substituted or unsubstituted C1 to C20 alkyl group, or a substituted or unsubstituted C6 to C20 aryl group),
$R^{11}$, $R^{12}$, and $R^{13}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or

any combination thereof,
n is 0 or 1, and
* is a linking portion linked to Chemical Formula 1.

[Chemical Formula 5F]

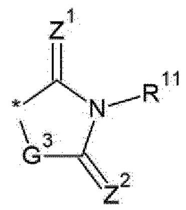

In Chemical Formula 5F,

$Z^1$ and $Z^2$ are each independently O, S, Se, Te, or $CR^aR^b$ (wherein $R^a$ and $R^b$ are each independently hydrogen, deuterium, a substituted or unsubstituted C1 to C10 alkyl group, a cyano group, or a cyano-containing group),
$R^{11}$ is hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof,
$G^3$ is O, S, Se, Te, $SiR^xR^y$, or $GeR^zR^w$, wherein $R^x$, $R^y$, $R^z$, and $R^w$ are each independently hydrogen, deuterium, a halogen, a cyano group, a substituted or unsubstituted C1 to C20 alkyl group, or a substituted or unsubstituted C6 to C20 aryl group, and
* is a linking portion linked to Chemical Formula 1.

[Chemical Formula 5G]

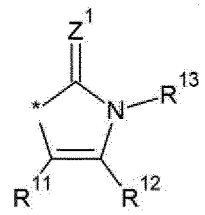

In Chemical Formula 5G,

$Z^1$ is O, S, Se, Te, or $CR^aR^b$, wherein $R^a$ and $R^b$ are each independently hydrogen, deuterium, a substituted or unsubstituted C1 to C10 alkyl group, a cyano group, or a cyano-containing group,
$R^{11}$, $R^{12}$, and $R^{13}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof, and
* is a linking portion linked to Chemical Formula 1.

[Chemical Formula 5H]

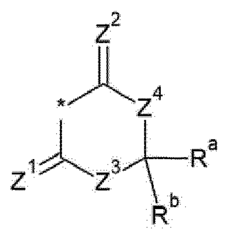

In Chemical Formula 5H,

$R^a$ and $R^b$ are each independently hydrogen, deuterium, a substituted or unsubstituted C1 to C10 alkyl group, a cyano group, or a cyano-containing group,
$Z^1$ to $Z^4$ are each independently O, S, Se, Te, or $CR^cR^d$, wherein $R^c$ and $R^d$ are each independently hydrogen, deuterium, a substituted or unsubstituted C1 to C10 alkyl group, a cyano group, or a cyano-containing group, and
* is a linking portion linked to Chemical Formula 1.

The compound may have a polarizability of less than or equal to about 450 $Bohr^3$.
The compound may have an oscillator strength value of greater than or equal to about 0.65.
The compound may have a dipole moment of greater than or equal to about 5 Debye.
The compound may have a maximum absorption wavelength ($\lambda_{max}$) in a wavelength range of about 520 nm to about 540 nm in a thin film state.
A sublimation temperature of the compound represented by Chemical Formula 1 (wherein, the sublimation temperature refers to a temperature at which a weight loss of 10% compared to the initial weight occurs when thermogravimetric analysis occurs at 10 Pa or less) may be less than or equal to about 250 °C.
The compound may exhibit an absorption curve with a full width at half maximum (FWHM) of less than or equal to about 150 nm in a thin film state.
According to some example embodiments, a photoelectric device (e.g., organic photoelectric device) includes a first electrode and a second electrode facing each other, and

an active layer between the first electrode and the second electrode,

the light absorbing layer includes the compound represented by Chemical Formula 1.

The active layer may include a p-type semiconductor and an n-type semiconductor, the p-type semiconductor includes the compound represented by Chemical Formula 1, and the n-type semiconductor includes fullerene, a fullerene derivative, sub-phthalocyanine or a sub-phthalocyanine derivative, thiophene or a thiophene derivative, or a compound represented by Chemical Formula 6.

[Chemical Formula 6]

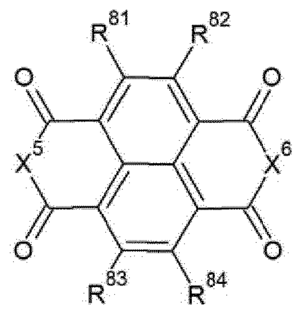

In Chemical Formula 6,

$X^5$ and $X^6$ may each independently be O or $NR^a$, (wherein $R^a$ is hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heterocyclic group, a halogen, or a cyano group), and
$R^{81}$ to $R^{84}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heterocyclic group, a halogen, a cyano group or any combination thereof.

According to some example embodiments, an image sensor including the photoelectric device is provided.
The image sensor may include a semiconductor substrate integrated with a plurality of first photo-sensing devices sensing light in a blue wavelength region and a plurality of second photo-sensing devices sensing light in a red wavelength region, and the photoelectric device may be on the semiconductor substrate and may be configured to selectively sense light in a green wavelength region.
The first photo-sensing device and the second photo-sensing device may be stacked in a vertical direction in

the semiconductor substrate.

The image sensor may further include a color filter layer including a blue filter selectively transmitting light in a blue wavelength region and a red filter selectively transmitting light in a red wavelength region.

The image sensor may include the photoelectric device, wherein the photoelectric device is a green photoelectric device configured to sense light in a green wavelength region, and the image sensor may include a stack of the green photoelectric device, a blue photoelectric device configured to selectively absorb light in a blue wavelength region, and a red photoelectric device configured to selectively absorb light in a red wavelength region.

According to some example embodiments, an electronic device including the photoelectric device or image sensor is provided.

The compound may be configured to selectively absorb light in the visible light region (e.g., green wavelength region) and may have excellent light absorption properties, and thus the compound may be suitably used in photoelectric devices or image sensors.

## BRIEF DESCRIPTION OF THE DRAWINGS

FIG. 1 is a cross-sectional view illustrating a photoelectric device according to some example embodiments,
FIG. 2 is a cross-sectional view showing a photoelectric device according to some example embodiments,
FIG. 3 is a plan view schematically illustrating an organic CMOS image sensor according to some example embodiments,
FIG. 4 is a cross-sectional view of the organic CMOS image sensor of FIG. 3 according to some example embodiments,
FIG. 5 is a cross-sectional view schematically illustrating an organic CMOS image sensor according to some example embodiments,
FIG. 6 is a cross-sectional view schematically illustrating an organic CMOS image sensor according to some example embodiments,
FIG. 7 is a cross-sectional view of an organic CMOS image sensor according to some example embodiments,
FIG. 8 is a schematic view schematically illustrating an organic CMOS image sensor according to some example embodiments,
FIG. 9 is a cross-sectional view of the organic CMOS image sensor of FIG. 8 according to some example embodiments,
FIG. 10 is a perspective view schematically showing an organic CMOS image sensor according to some example embodiments,
FIG. 11 schematically shows a view of cross-section of an example of the organic CMOS image sensor of FIG. 10 taken along line XI-XI' of FIG. 10 according to some example embodiments,
FIG. 12 is a block diagram of a digital camera including an image sensor according to some example embodiments, and
FIG. 13 is a schematic diagram of an electronic device according to some example embodiments.

## DETAILED DESCRIPTION OF THE EMBODIMENTS

Hereinafter, some example embodiments are described in detail so that those of ordinary skill in the art can easily implement them. However, a structure that is actually applied may be implemented in various different forms, and is not limited to the example embodiments described herein.

In the drawings, the thickness of layers, films, panels, regions, etc., are exaggerated for clarity. It will be understood that when an element such as a layer, film, region, or substrate is referred to as being "on" another element, it can be directly on the other element or intervening elements may also be present. In contrast, when an element is referred to as being "directly on" another element, there are no intervening elements present.

In the drawings, parts having no relationship with the description are omitted for clarity of some example embodiments, and the same or similar constituent elements are indicated by the same reference numeral throughout the specification.

Hereinafter, the terms "lower portion" and "'upper portion" are used for better understanding and ease of description, but do not limit the position relationship.

As used herein, "at least one of A, B, or C," "one of A, B, C, or any combination thereof" and "one of A, B, C, and any combination thereof" refer to each constituent element, and any combination thereof (e.g., A; B; C; A and B; A and C; B and C; or A, B and C).

As used herein, when a definition is not otherwise provided, "substituted" refers to replacement of hydrogen of

a compound or a functional group by a substituent selected from a halogen (F, Br, Cl, or I), a hydroxy group, a nitro group, a cyano group, an amine group, an azido group, an amidino group, a hydrazino group, a hydrazono group, a carbonyl group, a carbamyl group, a thiol group, an ester group, a carboxyl group or a salt thereof, a sulfonic acid group or a salt thereof, a phosphoric acid group or a salt thereof, a C1 to C30 alkyl group, a C2 to C30 alkenyl group, a C2 to C30 alkynyl group, a C6 to C30 aryl group, a C7 to C30 arylalkyl group, a C1 to C30 alkoxy group, a C1 to C20 heteroalkyl group, a C3 to C20 heterocyclic group, a C3 to C20 heteroarylalkyl group, a C3 to C30 cycloalkyl group, a C3 to C15 cycloalkenyl group, a C6 to C15 cycloalkynyl group, a C3 to C30 heterocycloalkyl group, Si($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), -Ge($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), or any combination thereof.

As used herein, "aryl group" refers to a cyclic functional group wherein all elements of the cycle (all ring-forming atoms) have p-orbitals which form conjugation, and may be a monocyclic, polycyclic, or fused ring polycyclic (e.g., rings sharing adjacent pairs of carbon atoms) functional group.

As used herein, "arene group" refers to a hydrocarbon cyclic group having an aromatic ring, and includes monocyclic and multicyclic hydrocarbon cyclic groups, and additional rings of the multicyclic hydrocarbon cyclic group may be an aromatic ring or a non-aromatic ring. The arene group may be a C6 to C30 arene group, a C6 to C20 arene group, or a C6 to C10 arene group.

As used herein, "heteroarene group" refers to an arene group including 1 to 3 heteroatoms selected from N, O, S, Se, Te, P, and Si in the ring. The heteroarene group may be a C3 to C30 heteroarene group, a C3 to C20 heteroarene group, or a C3 to C10 heteroarene group.

As used herein, "hydrocarbon cyclic group" may be a C3 to C30 hydrocarbon cyclic group. The hydrocarbon cyclic group may be an aromatic hydrocarbon cyclic group (e.g., C6 to C30 arene group, C6 to C20 arene group, or C6 to C10 arene group or C6 to C30 aryl group, C6 to C20 aryl group, or C6 to C10 aryl group), an alicyclic hydrocarbon cyclic group (e.g., C3 to C30 cycloalkyl group, C5 to C30 cycloalkyl group, C3 to C20 cycloalkyl group, or C3 to C10 cycloalkyl group), or a fused ring group thereof. For example, the fused ring group may refer to a fused ring of an aromatic ring (arene ring) and a non-aromatic ring (alicyclic ring), for example a fused ring in which at least one aromatic ring (arene ring) such as a C6 to C30 arene group, a C6 to C20 arene group, or a C6 to C10 arene group or a C6 to C30 aryl group, a C6 to C20 aryl group, or a C6 to C10 aryl group and at least one non-aromatic ring (alicyclic ring) such as a C3 to C30 cycloalkyl group, a C3 to C20 cycloalkyl group, or a C3 to C10 cycloalkyl group are fused to each other.

As used herein, when a definition is not otherwise provided, "aromatic ring" refers to a C6 to C10 hydrocarbon cyclic group (e.g., a C6 to C10 aryl group) providing a conjugated structure or a C2 to C10 heterocyclic group (e.g., a C2 to C10 heteroaryl group) providing a conjugated structure.

As used herein, when a definition is not otherwise provided, "fused ring" is a fused ring of two or more substituted or unsubstituted C5 to C30 hydrocarbon cyclic groups, a fused ring of two or more substituted or unsubstituted C2 to C30 heterocyclic groups, or a fused ring of a substituted or unsubstituted C5 to C30 hydrocarbon cyclic group and a substituted or unsubstituted C2 to C30 heterocyclic group (e.g., a fluorenyl group). Herein, the hydrocarbon cyclic group and the hetero cyclic group are as defined above.

As used herein, when specific definition is not otherwise provided, "'hetero" refers to one including 1 to 3 heteroatoms selected from N, O, S, Se, Te, P, and Si.

As used herein, "heteroaryl group" refers to an aryl group containing 1 to 3 heteroatoms selected from N, O, S, Se, Te, P, and Si in the ring. The heteroaryl group may be a C3 to C30 heteroaryl group, a C3 to C20 heteroaryl group, or a C3 to C10 heteroaryl group.

As used herein, when a definition is not otherwise provided, "alkyl group" refers to a monovalent linear or branched saturated hydrocarbon group, for example a methyl group, an ethyl group, a propyl group, an isopropyl group, an n-butyl group, an isobutyl group, a t-butyl group, a pentyl group, a hexyl group, and the like.

As used herein, when a definition is not otherwise provided, "alkoxy group" is expressed as -OR, where R may be the alkyl group described above.

As used herein, when a definition is not otherwise provided, "cycloalkyl group" refers to a monovalent saturated hydrocarbon ring group in which the atoms of the cycle are carbon, for example a cyclopropyl group, a cyclobutyl group, a cyclopentyl group, a cyclohexyl group, and the like.

As used herein, when a definition is not otherwise provided, "cyano-containing group" refers to a monovalent group such as a C1 to C30 alkyl group, a C2 to C30 alkenyl group, or a C2 to C30 alkynyl group where at least one hydrogen is substituted with a cyano group. As used herein, when a definition is not otherwise provided, the cyano-containing group also refers to a divalent group such as $=CR^{x'}-(CR^xR^y)_p-CR^{y'}(CN)_2$ wherein $R^x$, $R^y$, $R^{x'}$, and $R^{y'}$ may each independently be hydrogen or a C1 to C10 alkyl group and p is an integer of 0 to 10 (or 1 to 10). As a monovalent functional group, specific examples of the cyano-containing group may be a dicyanomethyl group, a dicyanovinyl group, a cyanoethynyl group, and the like. As used herein, the cyano-containing group does not include a functional group

including a cyano group (-CN) alone.

As used herein, when a definition is not otherwise provided, "combination" refers to a mixture, a stack, or an alloy of constituting components.

As used herein, when a definition is not otherwise provided, "combination thereof" in the definition of chemical formula refers to at least two substituents bound to each other by a single bond or a C1 to C10 alkylene group, or at least two substituents which are fused.

As used herein, when a definition is not otherwise provided, "hydrocarbon ring group" may be a C3 to C30 hydrocarbon ring group. The hydrocarbon ring group may be an arene group (e.g., a C6 to C30 arene group, a C6 to C20 arene group, or a C6 to C10 arene group), an alicyclic hydrocarbon ring group (e.g., a C3 to C30 cycloalkyl group, a C5 to C30 cycloalkyl group, a C3 to C20 cycloalkyl group, or a C3 to C10 cycloalkyl group) or a fused ring thereof. For example, the fused ring thereof may refer to a fused ring of an aromatic ring (arene ring) and a non-aromatic ring (alicyclic ring), for example a fused ring of at least one aromatic ring (arene ring) such as a C6 to C30 arene group, a C6 to C20 arene group, or a C6 to C10 arene group and at least one non-aromatic ring (alicyclic ring) such as a C3 to C30 cycloalkyl group, a C3 to C20 cycloalkyl group, or a C3 to C10 cycloalkyl group.

As used herein, when a definition is not otherwise provided, "heterocyclic group" may be a C2 to C30 heterocyclic group. The heterocyclic group refers to a cyclic group including 1 to 3 heteroatoms selected from N, O, S, Se, Te, P, and Si instead of carbon atom(s) in a cyclic group selected from an arene group (e.g., a C6 to C30 arene group, a C6 to C20 arene group, or a C6 to C10 arene group), an alicyclic hydrocarbon ring group (e.g., a C3 to C30 cycloalkyl group, a C3 to C20 cycloalkyl group, or a C3 to C10 cycloalkyl group), or a fused ring thereof. At least one carbon atom of the heterocyclic group may also be substituted with a thiocarbonyl group (C=S).

As used herein, "arene group" refers to a hydrocarbon group having an aromatic ring, and includes monocyclic and polycyclic hydrocarbon groups, and the additional ring of the polycyclic hydrocarbon group may be an aromatic ring or a nonaromatic ring. "Heteroarene group" refers to an arene group including 1 to 3 heteroatoms selected from N, O, S, Se, Te, P, and Si in a cyclic group.

As used herein, when a definition is not otherwise provided, "aromatic hydrocarbon group" includes a phenyl group, a naphthyl group, a C6 to C30 aryl group, and a C6 to C30 arylene group, but is not limited thereto.

As used herein, when a definition is not otherwise provided, aromatic ring may include a fused ring of an aromatic ring and an alicyclic ring. The "aromatic ring" refers to a C6 to C20 aryl group (e.g., C6 to C10 aryl group) or C2 to C20 heteroaryl group (e.g., C2 to C4 heteroaryl group). The "alicyclic ring" refers to a C3 to C10 cycloalkyl group or a C2 to C10 heterocycloalkyl group.

It will further be understood that when an element is referred to as being "on" another element, it may be above or beneath or adjacent (e.g., horizontally adjacent) to the other element. It will be understood that elements and/or properties thereof (e.g., structures, surfaces, directions, or the like), which may be referred to as being "perpendicular," "parallel," "coplanar," or the like with regard to other elements and/or properties thereof (e.g., structures, surfaces, directions, or the like) may be "perpendicular," "parallel," "coplanar," or the like or may be "substantially perpendicular," "substantially parallel," "substantially coplanar," respectively, with regard to the other elements and/or properties thereof. Elements and/or properties thereof (e.g., structures, surfaces, directions, or the like) that are "substantially perpendicular" with regard to other elements and/or properties thereof will be understood to be "perpendicular" with regard to the other elements and/or properties thereof within manufacturing tolerances and/or material tolerances and/or have a deviation in magnitude and/or angle from "perpendicular," or the like with regard to the other elements and/or properties thereof that is equal to or less than 10% (e.g., a. tolerance of $\pm$10%). Elements and/or properties thereof (e.g., structures, surfaces, directions, or the like) that are "substantially parallel" with regard to other elements and/or properties thereof will be understood to be "parallel" with regard to the other elements and/or properties thereof within manufacturing tolerances and/or material tolerances and/or have a deviation in magnitude and/or angle from "parallel," or the like with regard to the other elements and/or properties thereof that is equal to or less than 10% (e.g., a. tolerance of $\pm$10%). Elements and/or properties thereof (e.g., structures, surfaces, directions, or the like) that are "substantially coplanar" with regard to other elements and/or properties thereof will be understood to be "coplanar" with regard to the other elements and/or properties thereof within manufacturing tolerances and/or material tolerances and/or have a deviation in magnitude and/or angle from "coplanar," or the like with regard to the other elements and/or properties thereof that is equal to or less than 10% (e.g., a. tolerance of $\pm$10%). It will be understood that elements and/or properties thereof may be recited herein as being "identical" to, "the same" or "equal" as other elements and/or properties, and it will be further understood that elements and/or properties thereof recited herein as being "identical" to, "the same" as, or "equal" to other elements and/or properties may be "identical" to, "the same" as, or "equal" to or "substantially identical" to, "substantially the same" as or "substantially equal" to the other elements and/or properties thereof. Elements and/or properties thereof that are "substantially identical" to, "substantially the same" as or "substantially equal" to other elements and/or properties thereof will be understood to include elements and/or properties thereof that are identical to, the same as, or equal to the other elements and/or properties thereof within manufacturing tolerances and/or material tolerances. Elements and/or properties thereof that are identical or substantially identical to and/or the same or substantially the

same as other elements and/or properties thereof may be structurally the same or substantially the same, functionally the same or substantially the same, and/or compositionally the same or substantially the same. While the term "same," "equal" or "identical" may be used in description of some example embodiments, it should be understood that some imprecisions may exist. Thus, when one element or value is referred to as being the same as another element or value, it should be understood that an element or a value is the same as another element or value within a desired manufacturing or operational tolerance range (e.g., ±10%). It will be understood that elements and/or properties thereof described herein as being the "substantially" the same and/or identical encompasses elements and/or properties thereof that have a relative difference in magnitude that is equal to or less than 10%. Further, regardless of whether elements and/or properties thereof are modified as "substantially," it will be understood that these elements and/or properties thereof should be construed as including a manufacturing or operational tolerance (e.g., ±10%) around the stated elements and/or properties thereof. When the terms "about" or "substantially" are used in this specification in connection with a numerical value, it is intended that the associated numerical value include a tolerance of ±10% around the stated numerical value. Moreover, when the words "about" and "substantially" are used in connection with geometric shapes, it is intended that precision of the geometric shape is not required but that latitude for the shape is within the scope of the inventive concepts. Further, regardless of whether numerical values or shapes are modified as "about" or "substantially," it will be understood that these values and shapes should be construed as including a manufacturing or operational tolerance (e.g., ±10%) around the stated numerical values or shapes. When ranges are specified, the range includes all values therebetween such as increments of 0.1%.

As used herein, when a definition is not otherwise provided, the energy level is the highest occupied molecular orbital (HOMO) energy level or the lowest unoccupied molecular orbital (LUMO) energy level.

As used herein, when a definition is not otherwise provided, a work function or energy level is expressed as an absolute value from a vacuum level. In addition, when the work function or the energy level is referred to be deep, high, or large, it may have a large absolute value based on "0 eV" of the vacuum level while when the work function or the energy level is referred to be shallow, low, or small, it may have a small absolute value based on "0 eV" of the vacuum level. In addition, a difference between the work function and/or the energy level may be a value obtained by subtracting a small value of the absolute value from a large value of the absolute value.

As used herein, when a definition is not otherwise provided, the HOMO energy level may be evaluated by the amount of photoelectrons emitted according to energy by irradiating UV light onto a thin film using AC-2 (Hitachi) or AC-3 (Riken Keiki Co., LTD.).

As used herein, when a definition is not otherwise provided, the LUMO energy level is obtained as follow: an energy bandgap is obtained using a UV-Vis spectrometer (Shimadzu Corporation), and then the LUMO energy level is calculated from the energy bandgap and the measured HOMO energy level.

Hereinafter, reorganization energy, dipole moment, and oscillator strength are values calculated at the DFT B3LYP/DGDZVP level using the Gaussian 09 program.

Polarizability refers to an average electric dipole moment created by unit electric field strength per molecule, and can be obtained by calculating at the DFT B3LYP/DGDZVP level using the Gaussian 09 program.

In addition, the sublimation temperature of a compound may be measured by thermogravimetric analysis (TGA), and may be a temperature at which, for example, a weight loss of 10% of the compound compared to an initial weight of the compound occurs when thermogravimetric analysis is performed at a pressure of about 10 Pa or less (e.g., 0 Pa to about 10 Pa, about 0.01 Pa to about 10 Pa, about 0.1 Pa to about 10 Pa, about 1 Pa to about 10 Pa, etc.).

Hereinafter, a compound according to some example embodiments will be described. The compound is represented by Chemical Formula 1 and may have a reorganization energy of less than or equal to about 0.191 eV, and the compound may have a maximum absorption wavelength value calculated by density functional theory (DFT) of less than or equal to about 500 nm.

[Chemical Formula 1]

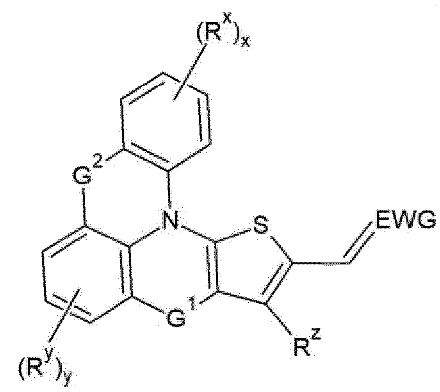

In Chemical Formula 1,

$G^1$ is a single bond, O, S, Se, Te, S(=O), $S(=O)_2$, $NR^a$, $BR^b$, $-SiR^cR^d-$, $-SiR^{cc}R^{dd}-$, $-GeR^eR^f-$, $-GeR^{ee}R^{ff}-$, $-(CR^gR^h)_{n1}-$, $-(CR^{gg}R^{hh})-$, $-(C(R^i)=C(R^j))-$ or $-(C(R^{ii})=C(R^{jj}))-$(wherein $R^a$, $R^b$, $R^c$, $R^d$, $R^e$, $R^f$, $R^g$, $R^h$, $R^i$ and $R^j$ are each independently hydrogen, deuterium, a halogen, a C1 to C10 alkyl group, a C1 to C10 haloalkyl group, a C1 to C10 alkoxy group, or a C6 to C20 aryl group, $R^{cc}$ and $R^{dd}$, $R^{ee}$ and $R^{ff}$, $R^{gg}$ and $R^{hh}$, or $R^{ii}$ and $R^{jj}$ are linked to each other to form a ring structure, and n1 is 1 or 2),

$G^2$ is O, S, Se, Te, S(=O), $S(=O)_2$, $NR^a$, $BR^b$, $-SiR^cR^d-$, $-SiR^{cc}R^{dd}-$, $-GeR^eR^f-$, $-GeR^{ee}R^{ff}-$, $-(CR^gR^h)_{n1}-$, $-(CR^{gg}R^{hh})-$, $-(C(R^i)=C(R^j))-$ or $-(C(R^{ii})=C(R^{jj}))-$ (wherein $R^a$, $R^b$, $R^c$, $R^d$, $R^e$, $R^f$, $R^g$, $R^h$, $R^i$ and $R^j$ are each independently hydrogen, deuterium, a halogen, a C1 to C10 alkyl group, a C1 to C10 haloalkyl group, a C1 to C10 alkoxy group, or a C6 to C20 aryl group, $R^{cc}$ and $R^{dd}$, $R^{ee}$ and $R^{ff}$, $R^{gg}$ and $R^{hh}$, or $R^{ii}$ and $R^{jj}$ are linked to each other to form a ring structure, and n1 is 1 or 2),

$R^x$, $R^y$, and $R^z$ are each independently hydrogen, deuterium, a halogen, a C1 to C10 alkyl group, a C1 to C10 haloalkyl group, a C1 to C10 alkoxy group, or a C6 to C20 aryl group, x is an integer of 0 to 4, and y is an integer of 0 to 3, and

EWG is an acceptor moiety including at least one electron withdrawing group.

In some example embodiments of the inventive concepts, based on a maximum absorption wavelength value and a reorganization energy of a compound calculated by density functional theory being within certain ranges (e.g., a reorganization energy of less than or equal to about 0.191 eV, and a maximum absorption wavelength value calculated based on density functional theory (DFT) of less than or equal to about 500 nm), the compound may have improved absorption intensity in a specific wavelength region of the visible light region (for example, the green wavelength region) and electrical characteristics may be improved. As a result, the photoelectric conversion performance and/or efficiency of a device (e.g., photoelectric device) including the compound (e.g., in an active layer of a photoelectric conversion device) may be improved, thereby improving the photoelectric conversion performance of the device and/or improving the power consumption efficiency of the device without compromising photoelectric conversion performance thereof, thereby improving the functionality of the device.

The compound represented by Chemical Formula 1 includes an electron donor moiety of a $G^2$-containing cyclic group (including a monocyclic group containing $G^2$ and N and two benzene rings fused thereto), a thiophene linker, and an electron acceptor moiety represented by EWG, and the electron donor moiety and thiophene linker are linked by $G^1$. The compound of Chemical Formula 1 has a donor-acceptor structure, and thus the compound has predetermined ranges of reorganization energy and a maximum absorption wavelength value calculated by density functional theory, whereby the absorption wavelength may be adjusted within a predetermined range of the visible light wavelength range (greater than or equal to about 520 nm, for example greater than or equal to about 521 nm, greater than or equal to about 522 nm, greater than or equal to about 522 nm, greater than or equal to about 523 nm, greater than or equal to about 524 nm, or greater than or equal to about 525 nm, and less than or equal to about 540 nm, for example, less than or equal to about 539 nm, less than or equal to about 538 nm, less than or equal to about 537 nm, less than or equal to about 536 nm, or less than or equal to about 535 nm), a deposition temperature (sublimation temperature) may be lowered, and absorption coefficient may be increased.

In Chemical Formula 1, at least one -CH= of at least one benzene ring (e.g., the benzene ring bearing $R^x$) of the $G^2$-containing cyclic group may be replaced by - N=. In Chemical Formula 1, N may be included at the 1st position 1 with respect to the nitrogen (N) of the $G^2$-containing cyclic group (refer to Chemical Formula 1A). In this case, intramolecular interactions increase, which can improve the light absorption characteristics of the compound.

[Chemical Formula 1A]

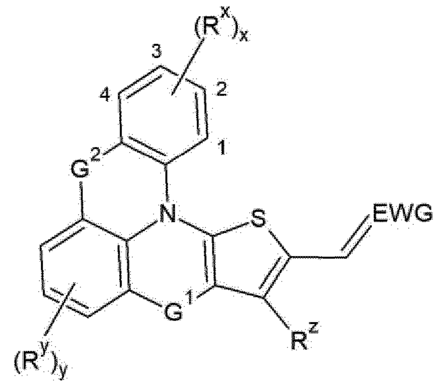

In Chemical Formula 1, $R^x$, $R^y$, and $R^z$ may each independently be hydrogen or an electron donating group selected from a C1 to C10 alkyl group and a C1 to C10 alkoxy group.

In Chemical Formula 1, the ring structure (e.g., formed, in at least one of $G^1$ or $G^2$, by $R^{cc}$ and $R^{dd}$, $R^{ee}$ and $R^{ff}$, $R^{gg}$ and $R^{hh}$, or $R^{ii}$ and $R^{jj}$ being linked to each other) may be a substituted or unsubstituted C5 to C30 hydrocarbon ring group or a substituted or unsubstituted C2 to C30 heterocyclic group.

The substituted or unsubstituted C5 to C30 hydrocarbon ring group may be, for example, a substituted or unsubstituted C5 to C30 cycloalkyl group (e.g., a substituted or unsubstituted C5 to C20 cycloalkyl group or a substituted or unsubstituted C3 to C10 cycloalkyl group); or a fused ring of at least one substituted or unsubstituted C5 to C30 cycloalkyl group (e.g., a substituted or unsubstituted C5 to C20 cycloalkyl group or a substituted or unsubstituted C3 to C10 cycloalkyl group) and at least one substituted or unsubstituted C6 to C30 aryl group (e.g., a substituted or unsubstituted C6 to C20 aryl group or a substituted or unsubstituted C3 to C10 aryl group). Examples of the fused ring include a fluorenyl group, an indanyl group, etc.

The substituted or unsubstituted C2 to C30 heterocyclic group may be, for example, a substituted or unsubstituted C2 to C30 heterocycloalkyl group (e.g., a substituted or unsubstituted C2 to C20 heterocycloalkyl group or a substituted or unsubstituted C2 to C10 heterocycloalkyl group). In addition, the substituted or unsubstituted C2 to C30 heterocyclic group may be a substituted or unsubstituted C2 to C30 heteroaryl group including at least one heteroatom. The substituted or unsubstituted C2 to C30 heterocyclic group may be a fused ring including a heterocycloalkyl group and/or a heteroaryl group. For example, the substituted or unsubstituted C2 to C30 heterocyclic group may be a fused ring of at least one of a substituted or unsubstituted C5 to C30 heterocycloalkyl group (e.g., a substituted or unsubstituted C3 to C20 heterocycloalkyl group or a substituted or unsubstituted C3 to C10 heterocycloalkyl group) and at least one of a substituted or unsubstituted C6 to C30 aryl group (e.g., a substituted or unsubstituted C6 to C20 aryl group or a substituted or unsubstituted C6 to C10 aryl group); a fused ring of at least one of a substituted or unsubstituted C5 to C30 cycloalkyl group (e.g., a substituted or unsubstituted C5 to C20 cycloalkyl group or a substituted or unsubstituted C5 to C10 cycloalkyl group) and at least one of a substituted or unsubstituted C2 to C30 heteroaryl group (e.g., a substituted or unsubstituted C2 to C20 heteroaryl group or a substituted or unsubstituted C3 to C10 heteroaryl group); or a fused ring of at least one of a substituted or unsubstituted C5 to C30 heterocycloalkyl group (e.g., a substituted or unsubstituted C3 to C20 heterocycloalkyl group or a substituted or unsubstituted C3 to C10 heterocycloalkyl group) and at least one of a substituted or unsubstituted C2 to C30 heteroaryl group (e.g., a substituted or unsubstituted C2 to C20 heteroaryl group or a substituted or unsubstituted C3 to C10 heteroaryl group).

In Chemical Formula 1, $G^1$ or $G^2$ may be the same or different from each other.

In Chemical Formula 1, the ring structure (a ring structure formed by linking $R^{cc}$ and $R^{dd}$, $R^{ee}$ and $R^{ff}$, $R^{gg}$ and $R^{hh}$, or $R^{ii}$ and $R^{jj}$ in at least one of $G^1$ or $G^2$) may include a moiety represented by Chemical Formula 2.

[Chemical Formula 2]

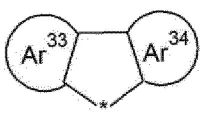

In Chemical Formula 2,

$Ar^{33}$ and $Ar^{34}$ are each independently a substituted or unsubstituted C6 to C30 arene group, a substituted or unsubstituted C3 to C30 heteroarene group, or a condensed ring thereof, and
* means a point linked to Chemical Formula 1.

In Chemical Formula 1, each ring structure (a ring structure formed, in at least one of $G^1$ or $G^2$, by $R^{cc}$ and $R^{dd}$, $R^{ee}$ and $R^{ff}$, $R^{gg}$ and $R^{hh}$, or $R^{ii}$ and $R^{jj}$ being linked to each other) may include one of moieties represented by Chemical Formula 3.

[Chemical Formula 3]

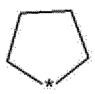
(1)

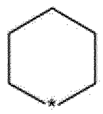
(2)

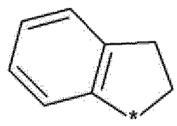
(3)

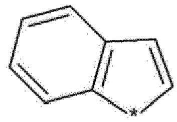
(4)

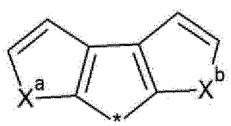
(5)

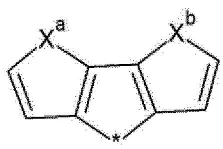
(6)

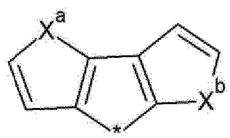
(7)

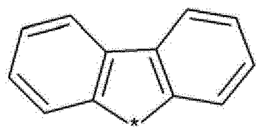
(8)

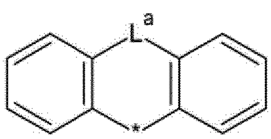
(9)

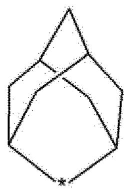
(10)

In Chemical Formula 3,

$X^a$ and $X^b$ may each independently be -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$ or $-GeR^{dd}R^{ee}-$, (wherein $R^{a1}$, $R^{a2}$, $R^b$, $R^c$, $R^d$, and $R^e$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and each pair of $R^{bb}$ and $R^{cc}$ or $R^{dd}$ and $R^{ee}$ may be linked to each other to form a ring structure),
$L^a$ may be -O-, -S-, -Se-, -Te-, $-NR^{a1}-$, $-BR^{a2}-$, $-SiR^bR^c-$, $-GeR^dR^e-$, $-(CR^fR^g)_{n1}-$, $-(C(R^p)=N)-$, or a single bond (wherein $R^{a1}$, $R^{a2}$, $R^b$, $R^c$, $R^d$, $R^e$, $R^f$, $R^g$, and $R^p$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and n1 of $-(CR^fR^g)_{n1}-$ is 1 or 2),
at least one hydrogen of each ring may be optionally replaced (i.e., may be present as hydrogen or may be replaced) by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group, and
* is a linking portion linked to Chemical Formula 1.

In Chemical Formula 3, at least one CH present in at least one aromatic ring of the moiety (3), (4), (5), (6), (7), (8), or (9) may be replaced by nitrogen (N).

In Chemical Formula 1, EWG may be a substituted or unsubstituted C6 to C30 hydrocarbon ring group including

at least one functional group selected from C=O, C=S, C=Se, C=Te, and $C=CR^pR^q$ (wherein $R^p$ and $R^q$ may each independently be hydrogen, deuterium, a substituted or unsubstituted C1 to C10 alkyl group, a cyano group, or a cyano-containing group); a substituted or unsubstituted C2 to C30 heterocyclic group including at least one functional group selected from C=O, C=S, C=Se, C=Te, and $C=CR^pR^q$ (wherein $R^p$ and $R^q$ may each independently be hydrogen, deuterium, a substituted or unsubstituted C1 to C10 alkyl group, a cyano group, or a cyano-containing group); or a fused ring thereof; $CR^pR^q$ (wherein $R^p$ and $R^q$ are each independently hydrogen, deuterium, a substituted or unsubstituted C1 to C10 alkyl group, a cyano group, or a cyano-containing group); or a C2 to C20 alkyl group including at least one functional group selected from C=O, C=S, C=Se, C=Te, and $C=CR^pR^q$ (wherein $R^p$ and $R^q$ may each independently be hydrogen, deuterium, a substituted or unsubstituted C1 to C10 alkyl group, a cyano group, or a cyano-containing group). In some example embodiments, in $C=CR^pR^q$ or $CR^pR^q$, at least one of $R^p$ and $R^q$ may be a cyano group or a cyano-containing group.

In Chemical Formula 1, EWG may be a cyclic group represented by Chemical Formula 4.

[Chemical Formula 4]

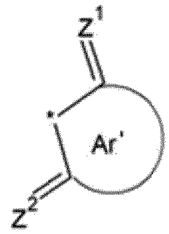

In Chemical Formula 4,

Ar' may be a substituted or unsubstituted C6 to C30 aryl group or a substituted or unsubstituted C3 to C30 heteroaryl group,
$Z^1$ and $Z^2$ may each independently be O, S, Se, Te, or $CR^aR^b$, wherein $R^a$ and $R^b$ may each independently be hydrogen, deuterium, a substituted or unsubstituted C1 to C10 alkyl group, a cyano group, or a cyano-containing group, and
* is a linking portion linked to Chemical Formula 1.

In some example embodiments, when both $Z^1$ and $Z^2$ are $CR^aR^b$, at least one of $Z^1$ and $Z^2$ may include a cyano group or a cyano-containing group.

In Chemical Formula 1, EWG may be a cyclic group represented by Chemical Formula 5A.

[Chemical Formula 5A]

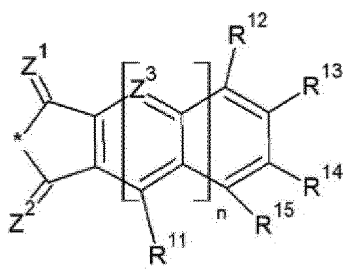

In Chemical Formula 5A,

$Z^1$ and $Z^2$ may each independently be O, S, Se, Te, or $CR^aR^b$, wherein $R^a$ and $R^b$ may each independently be hydrogen, deuterium, a substituted or unsubstituted C1 to C10 alkyl group, a cyano group, or a cyano-containing group,
$Z^3$ may be N or $CR^c$ (wherein $R^c$ may be hydrogen, deuterium, or a substituted or unsubstituted C1 to C10 alkyl group),
$R^{11}$, $R^{12}$, $R^{13}$, $R^{14}$, and $R^{15}$ may each independently be hydrogen, deuterium, a substituted or unsubstituted C1 to C30 alkyl group, a substituted or unsubstituted C1 to C30 alkoxy group, a substituted or unsubstituted C6 to C30

aryl group, a substituted or unsubstituted C4 to C30 heteroaryl group, a halogen, a cyano group (-CN), a cyano-containing group, or any combination thereof, wherein $R^{12}$ and $R^{13}$ and $R^{14}$ and $R^{15}$ are each independently present or are linked to each other to form a fused aromatic ring,
n may be 0 or 1, and
* is a linking portion linked to Chemical Formula 1.

In some example embodiments, when both $Z^1$ and $Z^2$ of Chemical Formula 5A are $CR^aR^b$, at least one of $Z^1$ or $Z^2$ may include a cyano group or a cyano-containing group.

The cyclic group represented by Chemical Formula 5A may be a cyclic group represented by Chemical Formula 5A-1 or Chemical Formula 5A-2.

[Chemical Formula 5A-1]

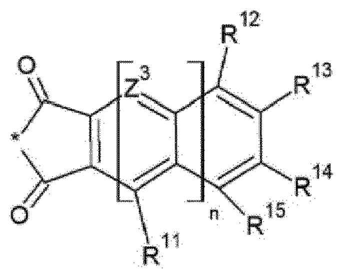

[Chemical Formula 5A-2]

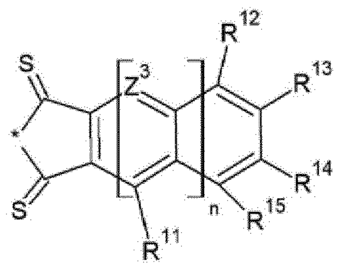

In Chemical Formula 5A-1 and Chemical Formula 5A-2,

$Z^3$, n, $R^{11}$, $R^{12}$, $R^{13}$, $R^{14}$, and $R^{15}$ are the same as $Z^3$, n, $R^{11}$, $R^{12}$, $R^{13}$, $R^{14}$, and $R^{15}$, respectively, in Chemical Formula 5A, and
* is a linking portion linked to Chemical Formula 1.

In Chemical Formula 1, EWG may be a cyclic group represented by Chemical Formula 5B.

[Chemical Formula 5B]

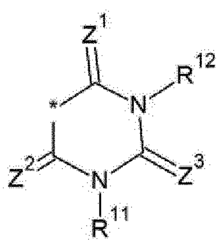

In Chemical Formula 5B,

$Z^1$ and $Z^2$ may each independently be O, S, Se, Te, or $CR^aR^b$, wherein $R^a$ and $R^b$ may each independently be hydrogen, deuterium, a substituted or unsubstituted C1 to C10 alkyl group, a cyano group, or a cyano-containing

group,

$Z^3$ may be O, S, Se, Te, or $CR^aR^b$ (wherein $R^a$ and $R^b$ are each independently hydrogen, deuterium, a substituted or unsubstituted C1 to C10 alkyl group, a cyano group, or a cyano-containing group),

$R^{11}$ and $R^{12}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and

* is a linking portion linked to Chemical Formula 1.

In some example embodiments, when both $Z^1$ and $Z^2$ of Chemical Formula 5B are $CR^aR^b$, at least one of $Z^1$ or $Z^2$ may include a cyano group or a cyano-containing group.

The cyclic group represented by Chemical Formula 5B may be, for example, a cyclic group represented by Chemical Formula 5B-1, Chemical Formula 5B-2, or Chemical Formula 5B-3.

[Chemical Formula 5B-1] [Chemical Formula 5B-2] [Chemical Formula 5B-3]

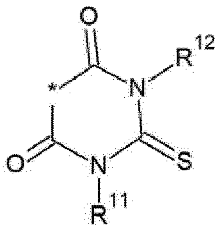

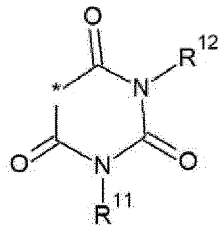

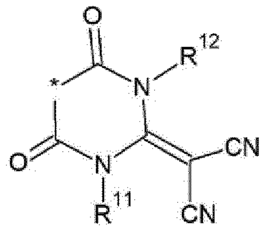

In Chemical Formulas 5B-1, 5B-2, and 5B-3,

$R^{11}$ and $R^{12}$ are the same as $R^{11}$ and $R^{12}$, respectively, in Chemical Formula 5B, and

* is a linking portion linked to Chemical Formula 1.

In Chemical Formula 1, EWG may be a cyclic group represented by Chemical Formula 5C.

[Chemical Formula 5C]

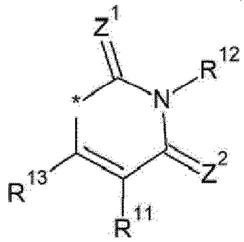

In Chemical Formula 5C,

$Z^1$ and $Z^2$ may each independently be O, S, Se, Te, or $CR^aR^b$ (wherein $R^a$ and $R^b$ are each independently hydrogen, deuterium, a substituted or unsubstituted C1 to C10 alkyl group, a cyano group, or a cyano-containing group),

$R^{11}$, $R^{12}$, and $R^{13}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and

* is a linking portion linked to Chemical Formula 1.

In some example embodiments, when both $Z^1$ and $Z^2$ of Chemical Formula 5C are $CR^aR^b$, at least one of $Z^1$ or $Z^2$ may include a cyano group or a cyano-containing group.

The cyclic group represented by Chemical Formula 5C may be, for example, a cyclic group represented by Chemical Formula 5C-1 or Chemical Formula 5C-2.

[Chemical Formula 5C-1]

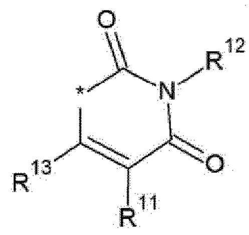

[Chemical Formula 5C-2]

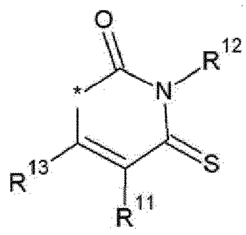

In Chemical Formulas 5C-1 and 5C-2,

$R^{11}$ to $R^{13}$ are the same as $R^{11}$ to $R^{13}$, respectively, in Chemical Formula 5C, and
* is a linking portion linked to Chemical Formula 1.

In Chemical Formula 1, EWG may be a cyclic group represented by Chemical Formula 5D.

[Chemical Formula 5D]

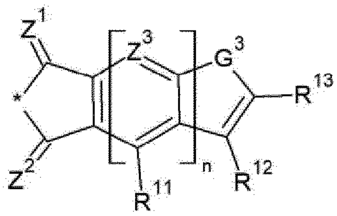

In Chemical Formula 5D,

$Z^1$ and $Z^2$ may each independently be O, S, Se, Te, or $CR^aR^b$ (wherein $R^a$ and $R^b$ may each independently be hydrogen, deuterium, a substituted or unsubstituted C1 to C10 alkyl group, a cyano group, or a cyano-containing group),
$Z^3$ may be N or $CR^c$ (wherein $R^c$ may be hydrogen or a substituted or unsubstituted C1 to C10 alkyl group),
$G^3$ may be O, S, Se, Te, $SiR^xR^y$, or $GeR^zR^w$, (wherein $R^x$, $R^y$, $R^z$, and $R^w$ may each independently be hydrogen, deuterium, a halogen, a cyano group, a substituted or unsubstituted C1 to C20 alkyl group, or a substituted or unsubstituted C6 to C20 aryl group),
$R^{11}$, $R^{12}$, and $R^{13}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof,
n may be 0 or 1, and
* is a linking portion linked to Chemical Formula 1.

In some example embodiments, when both $Z^1$ and $Z^2$ of Chemical Formula 5D are $CR^aR^b$, at least one of $Z^1$ or $Z^2$ may include a cyano group or a cyano-containing group.

In Chemical Formula 1, EWG may be a cyclic group represented by Chemical Formula 5E.

[Chemical Formula 5E]

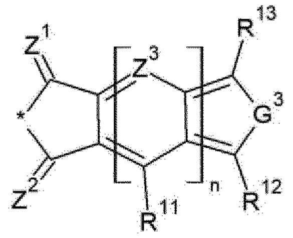

In Chemical Formula 5E,

$Z^1$ and $Z^2$ may each independently be O, S, Se, Te, or $CR^aR^b$ (wherein $R^a$ and $R^b$ are each independently hydrogen, deuterium, a substituted or unsubstituted C1 to C10 alkyl group, a cyano group, or a cyano-containing group),
$Z^3$ may be N or $CR^c$ (wherein $R^c$ may be hydrogen or a substituted or unsubstituted C1 to C10 alkyl group),
$G^3$ may be O, S, Se, Te, $SiR^xR^y$, or $GeR^zR^w$, (wherein $R^x$, $R^y$, $R^z$, and $R^w$ may each independently be hydrogen, deuterium, a halogen, a cyano group, a substituted or unsubstituted C1 to C20 alkyl group, or a substituted or unsubstituted C6 to C20 aryl group),
$R^{11}$, $R^{12}$, and $R^{13}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof,
n may be 0 or 1,
* is a linking portion linked to Chemical Formula 1.

In some example embodiments, when both $Z^1$ and $Z^2$ of Chemical Formula 5E are $CR^aR^b$, at least one of $Z^1$ or $Z^2$ may include a cyano group or a cyano-containing group.
In Chemical Formula 1, EWG may be a cyclic group represented by Chemical Formula 5F.

[Chemical Formula 5F]

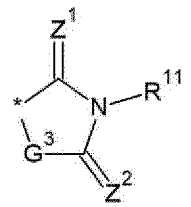

In Chemical Formula 5F,

$Z^1$ and $Z^2$ may each independently be O, S, Se, Te, or $CR^aR^b$, wherein $R^a$ and $R^b$ may each independently be hydrogen, deuterium, a substituted or unsubstituted C1 to C10 alkyl group, a cyano group, or a cyano-containing group,
$R^{11}$ may be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), a cyano-containing group, or any combination thereof,
$G^2$ may be O, S, Se, Te, $SiR^xR^y$, or $GeR^zR^w$, wherein $R^x$, $R^y$, $R^z$, and $R^w$ may each independently be hydrogen, deuterium, a halogen, a cyano group, a substituted or unsubstituted C1 to C20 alkyl group, or a substituted or unsubstituted C6 to C20 aryl group, and
* is a linking portion linked to Chemical Formula 1.

In some example embodiments, when both $Z^1$ and $Z^2$ of Chemical Formula 5F are $CR^aR^b$, at least one of $Z^1$ and $Z^2$ may include a cyano group or a cyano-containing group.
In Chemical Formula 1, EWG may be a cyclic group represented by Chemical Formula 5G.

[Chemical Formula 5G]

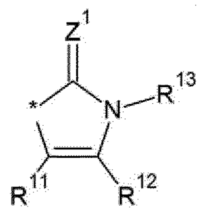

In Chemical Formula 5G,

$Z^1$ may be O, S, Se, Te, or $CR^aR^b$, wherein $R^a$ and $R^b$ may each independently be hydrogen, deuterium, a substituted or unsubstituted C1 to C10 alkyl group, a cyano group, or a cyano-containing group,
$R^{11}$, $R^{12}$, and $R^{13}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof, and
* is a linking portion linked to Chemical Formula 1.

In Chemical Formula 1, EWG may be a cyclic group represented by Chemical Formula 5H.

[Chemical Formula 5H]

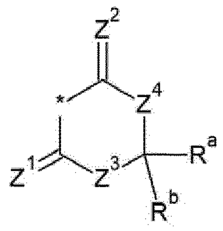

In Chemical Formula 5H,

$R^a$ and $R^b$ may each independently be hydrogen, deuterium, a substituted or unsubstituted C1 to C10 alkyl group, a cyano group, or a cyano-containing group,
$Z^1$ to $Z^4$ may each independently be O, S, Se, Te, or $CR^cR^d$, wherein $R^c$ and $R^d$ may each independently be hydrogen, deuterium, a substituted or unsubstituted C1 to C10 alkyl group, a cyano group, or a cyano-containing group, and
* is a linking portion linked to Chemical Formula 1.

In some example embodiments, when all $Z^1$ to $Z^4$ of Chemical Formula 5F are $CR^aR^b$, at least one of $Z^1$ to $Z^4$ may include a cyano group or a cyano-containing group.

Specific examples of the compound of Chemical Formula 1 may include compounds of Group 1-1 to Group 1-5, but are not limited thereto.

[Group 1-1]

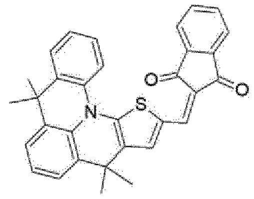
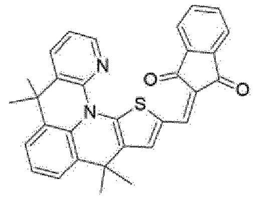
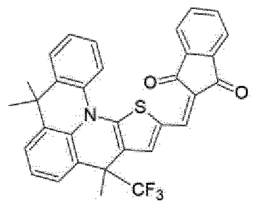
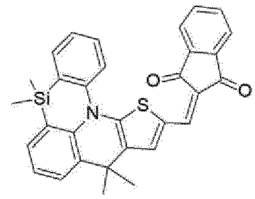
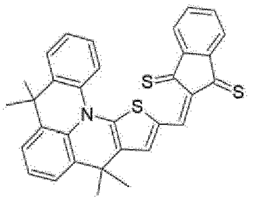
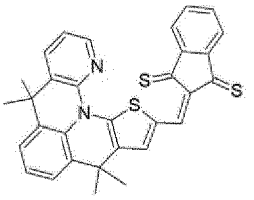
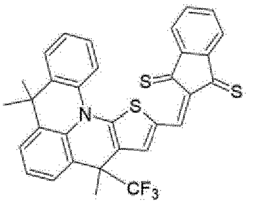
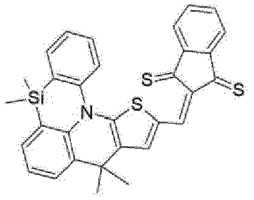
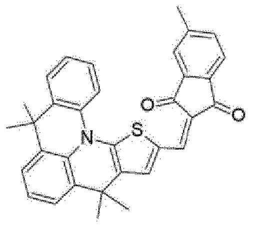
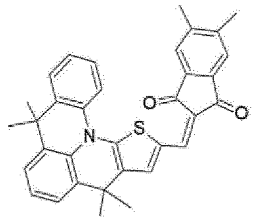
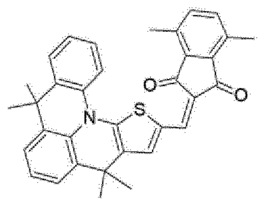
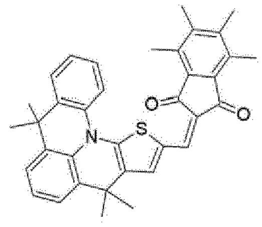
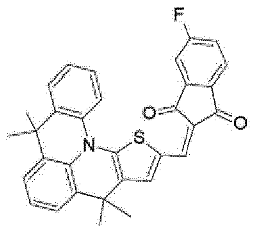
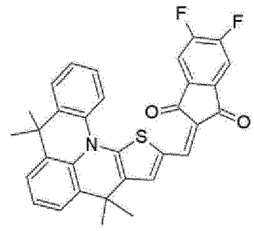
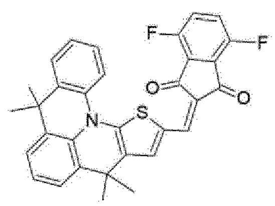
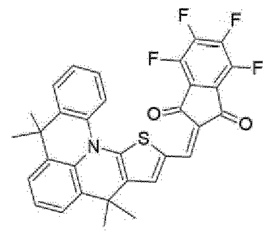
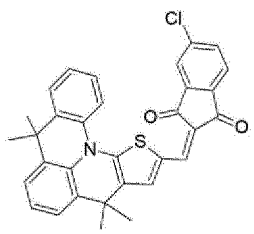
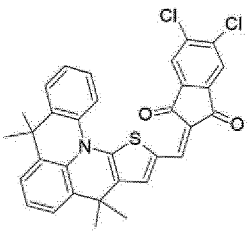
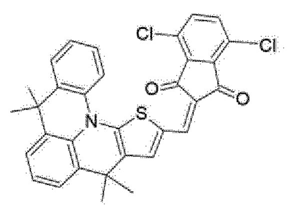
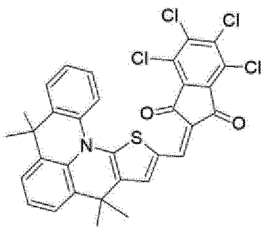

[Group 1-2]

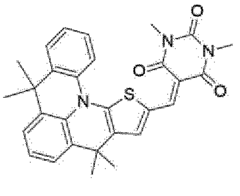
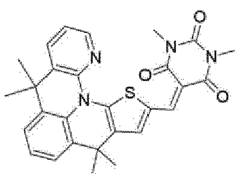
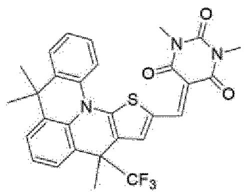
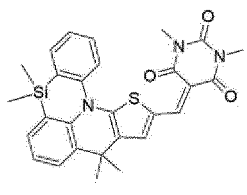
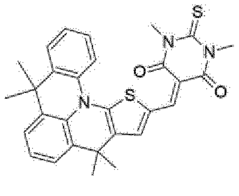
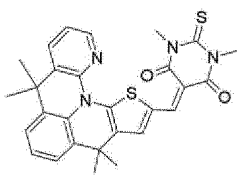
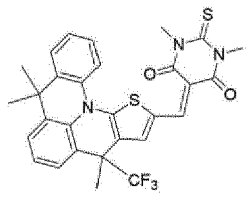
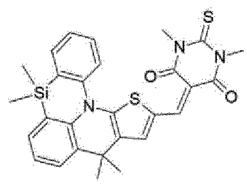
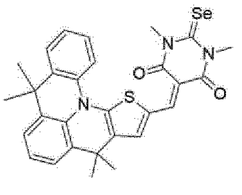
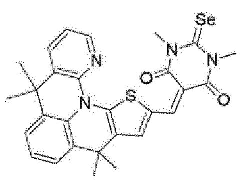
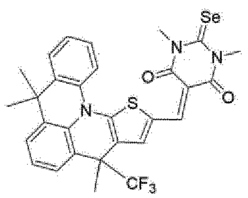
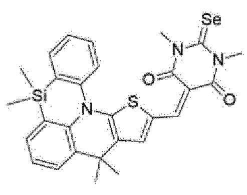
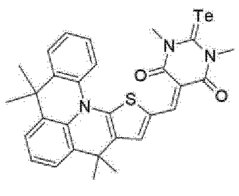
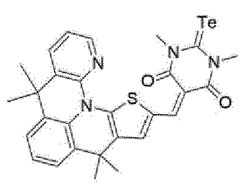
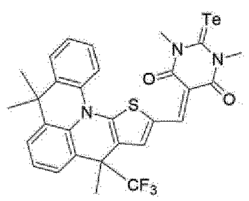
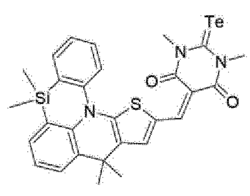
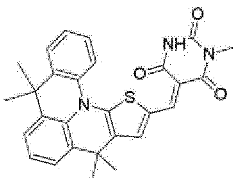
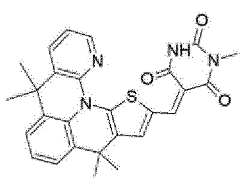
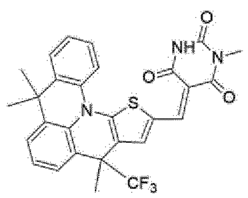
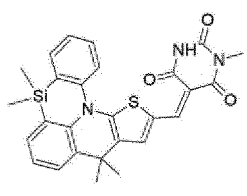
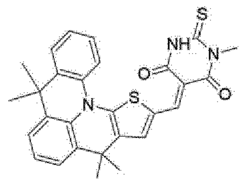
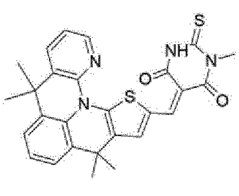
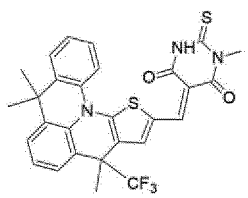
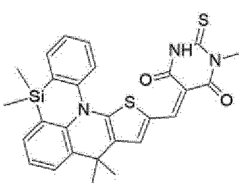
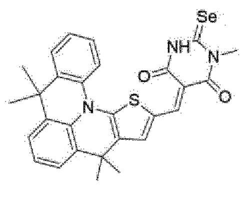
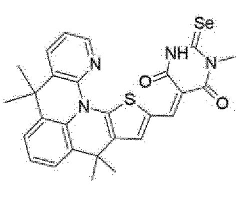
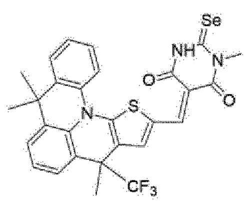
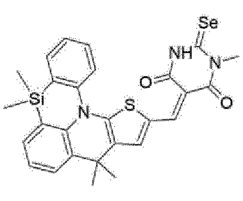
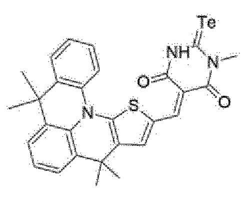
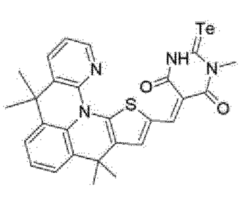
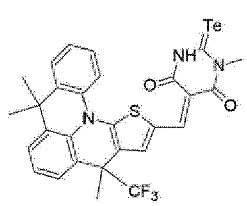
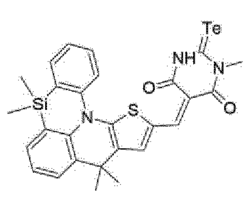

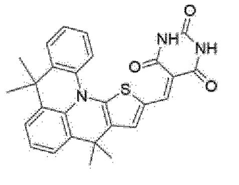
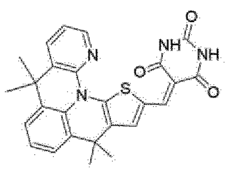
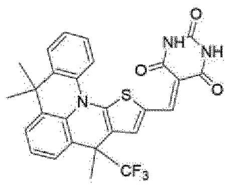
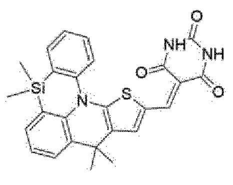

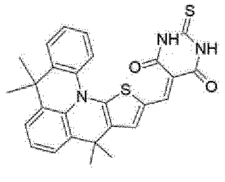
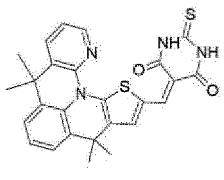
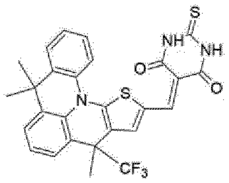
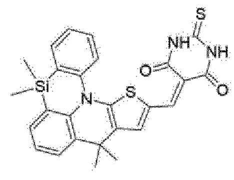

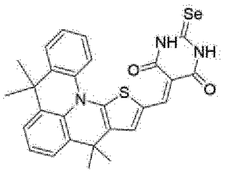
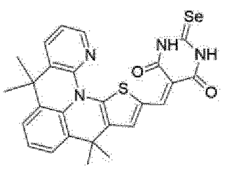
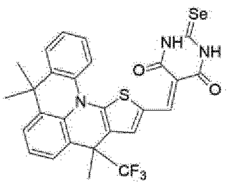
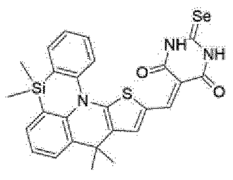

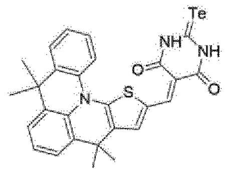
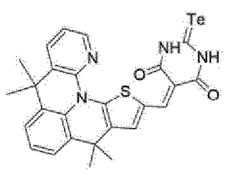
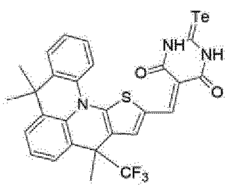
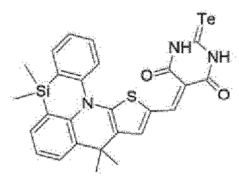

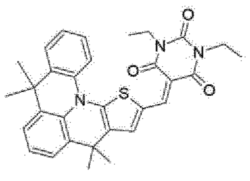
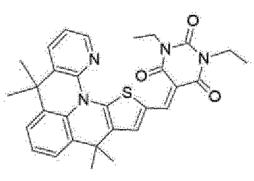
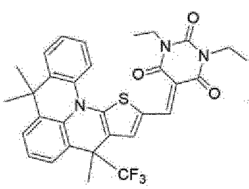
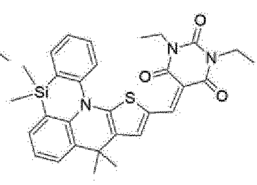

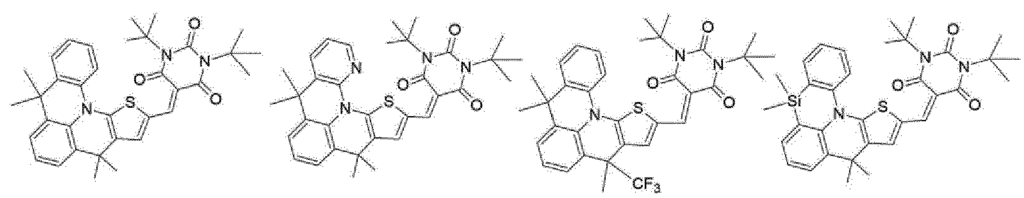

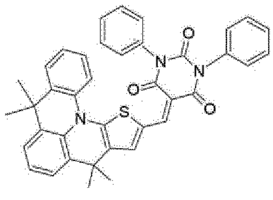
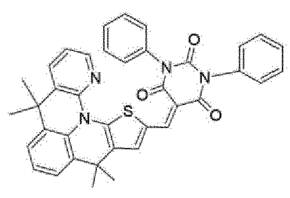
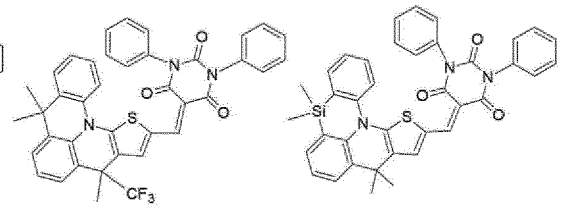

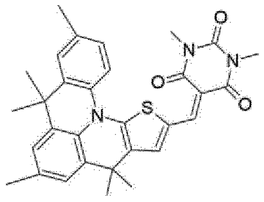 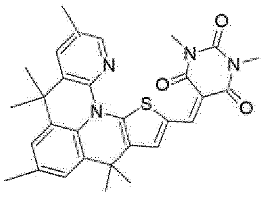 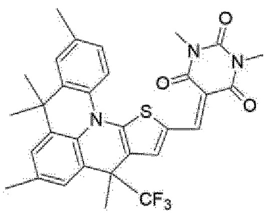 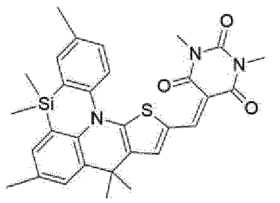

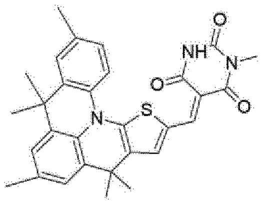 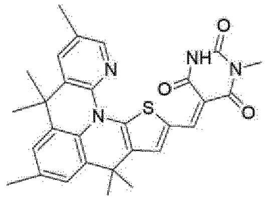 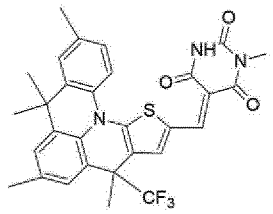 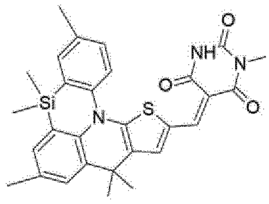

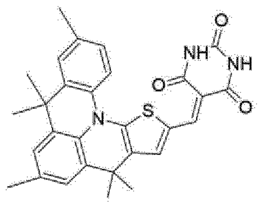 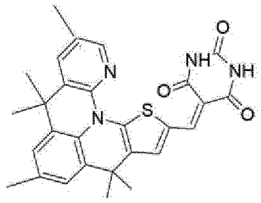 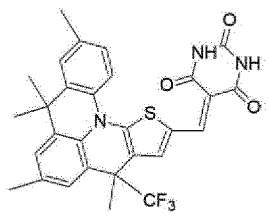 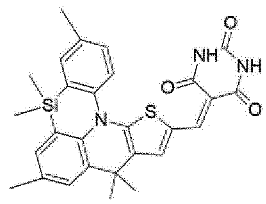

[Group 1-3]

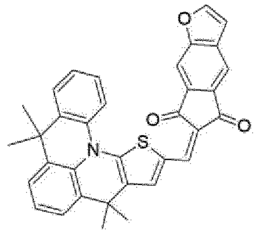
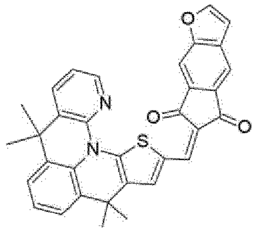
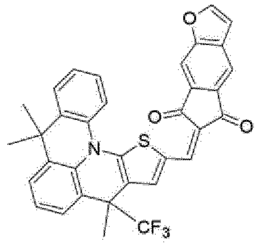
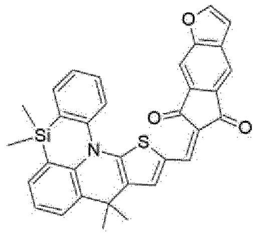
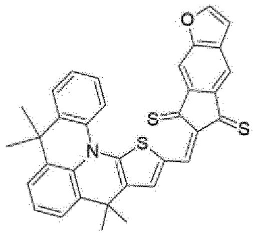
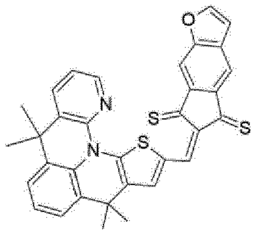
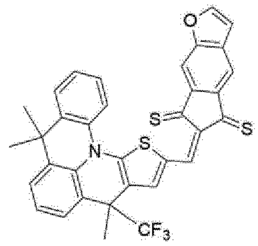
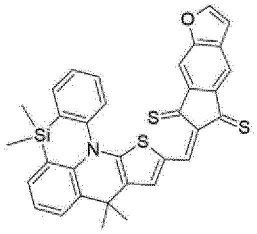
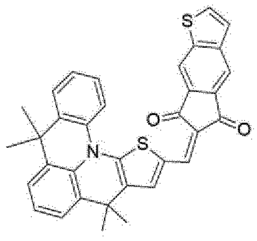
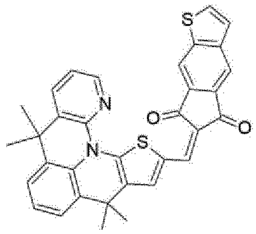
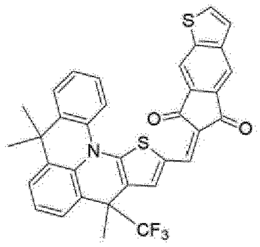
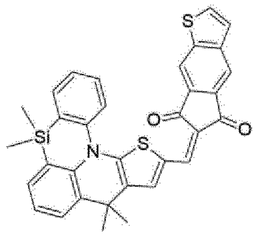
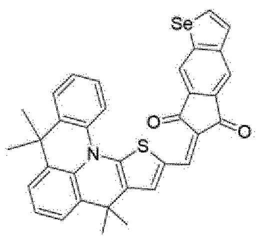
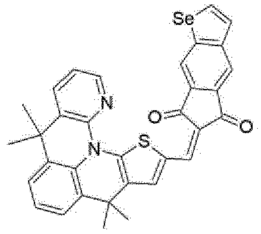
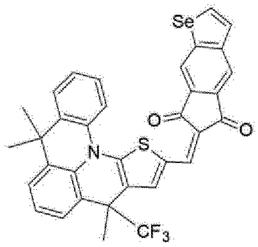
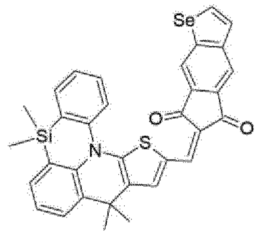
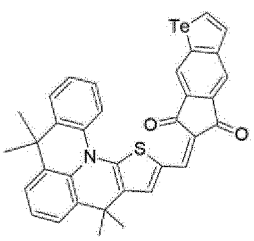
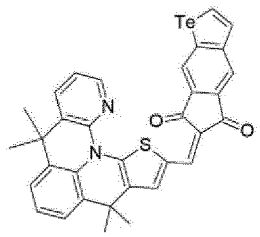
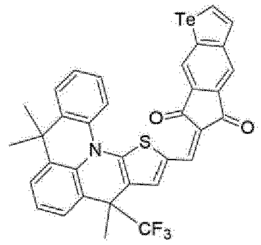
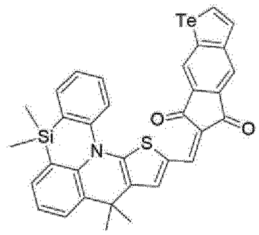

[Group 1-4]

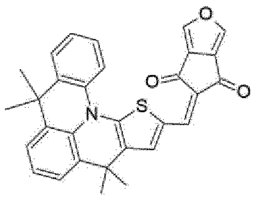
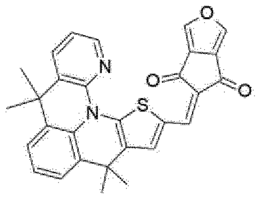
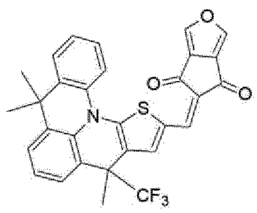
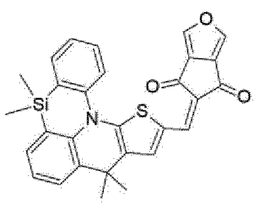

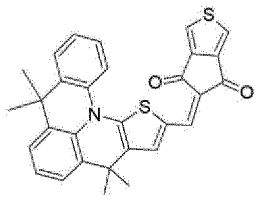
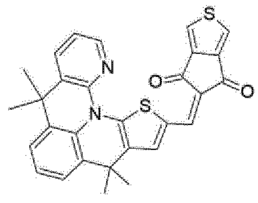
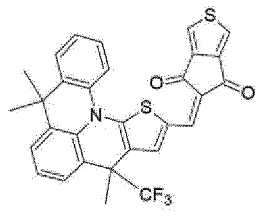
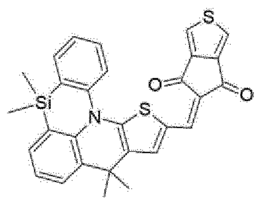

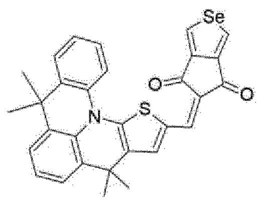
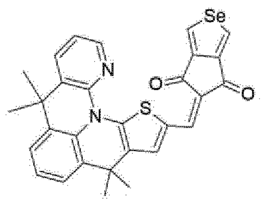
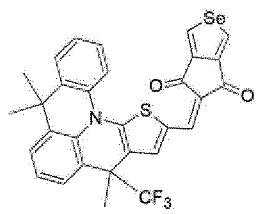
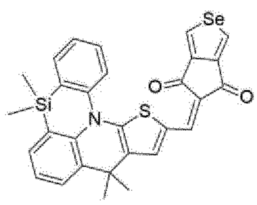

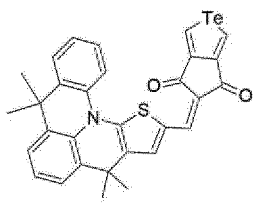
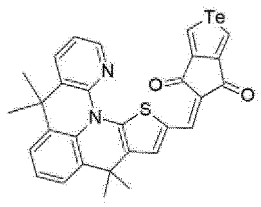
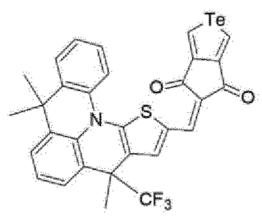
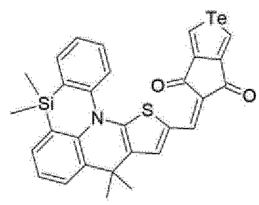

[Group 1-5]

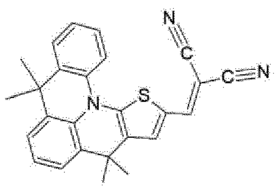
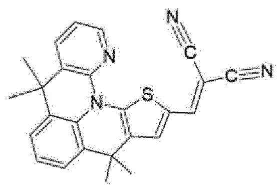
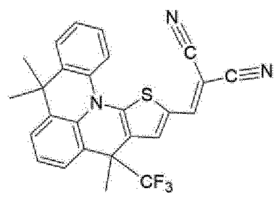
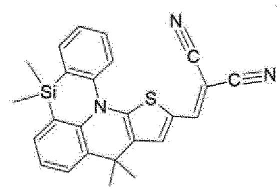

In Groups 1-1 to 1-5,
at least one hydrogen present in one or more rings, or each ring, may be present or may be replaced by a substituent selected from a C1 to C10 alkyl group, a C1 to C10 alkoxy group, a C6 to C10 aryl group, a C4 to C10 heteroaryl group, a halogen (F, Cl, Br, or I), a cyano group (-CN), a cyano-containing group, or any combination thereof.

In Groups 1-1 to 1-5, compounds in which $G^1$ is $-C(CH_3)_2-$, -S-, or $-Si(CH_3)_2$-are shown but compounds in which $G^1$ is a single bond, O, Se, Te, S(=O), $S(=O)_2$, $NR^a$, $BR^b$, $-SiR^cR^d-$, $-SiR^{cc}R^{dd}-$, $-GeR^eR^f-$, $-GeR^{ee}R^{ff}-$, $-(CR^gR^h)_{n1}-$, $-(CR^{gg}R^{hh})-$, - $(C(R^i)=(C(R^j))-$, or $-(C(R^{ii})=(C(R^{jj}))-$ may also be provided in the same way.

In addition, in Groups 1-1 to 1-5, compounds in which EWG is a cyclic group represented by Chemical Formula 5A, Chemical Formula 5B, Chemical Formula 5D, or Chemical Formula 5E or an alkyl group having an electron-withdrawing group are shown but compounds in which EWG is a cyclic group represented by Chemical Formula 5C, Chemical Formula 5F, Chemical Formula 5G, or Chemical Formula 5H may also be provided in the same way.

The compound may have a reorganization energy of less than or equal to about 0.191 eV. For example, the reorganization energy of the compound may be greater than or equal to about 0.05 eV and less than or equal to about 0.191 eV, for example greater than or equal to about 0.06 eV and less than or equal to about 0.191 eV, or greater than

or equal to about 0.07 eV and less than or equal to about 0.191 eV. When the compound has a reorganization energy in the above range, the mobility of the compound can be improved, such that a photoelectric conversion device and/or photoelectric conversion layer including the compound may have improved photoelectric conversion performance and/or efficiency.

Additionally, the compound may have a maximum absorption wavelength calculated by density functional theory (DFT) of less than or equal to about 500 nm. The calculated maximum absorption wavelength value may be, for example, less than about 500 nm and greater than or equal to about 450 nm, for example less than about 500 nm and greater than or equal to about 455 nm, less than about 500 nm and greater than or equal to about 460 nm, less than about 500 nm and greater than or equal to about 465 nm, less than about 500 nm and greater than or equal to about 470 nm, less than about 500 nm and greater than or equal to about 475 nm, or less than about 500 nm and greater than or equal to about 480 nm. When the calculated maximum absorption wavelength value is in the above range, the absorption wavelength (corrected maximum absorption wavelength, also referred to as corrected absorption wavelength) of the thin film including the compound may be in the range of about 520 nm to about 540 nm. If the maximum absorption wavelength value calculated by the DFT exceeds about 500 nm, the wavelength absorption selectivity may be reduced because the corrected maximum absorption wavelength is in the yellow wavelength region rather than the green wavelength region. The corrected maximum absorption wavelength of the thin film including the compound may be obtained by using Equation 2 as in the Examples.

The compound may have a polarizability of less than or equal to about 450 $Bohr^3$, for example less than or equal to about 445 $Bohr^3$, less than or equal to about 440 $Bohr^3$, less than or equal to about 435 $Bohr^3$, or less than or equal to about 430 $Bohr^3$, and greater than or equal to about 340 $Bohr^3$, for example greater than or equal to about 345 $Bohr^3$. When the compound has a polarizability within the above range, the light absorption characteristics of the compound may be improved, such that a photoelectric conversion device and/or photoelectric conversion layer including the compound may have improved photoelectric conversion performance and/or efficiency.

The compound may have a dipole moment of greater than or equal to about 5 Debye, for example greater than or equal to about 6 Debye and less than or equal to about 11 Debye. When the compound has a dipole moment in the above range, it is desirable for charge separation between the donor moiety and the acceptor moiety of the compound, and this allows electrons to be transferred well, thereby improving device performance such that a photoelectric conversion device and/or photoelectric conversion layer including the compound may have improved photoelectric conversion performance and/or efficiency.

The compound may have an oscillator strength value of greater than or equal to about 0.65, for example, greater than or equal to about 0.70, or greater than or equal to about 0.75 and less than or equal to about 1.8, for example less than or equal to about 1.7, or less than or equal to about 1.6. When the oscillator strength is in the above range, the absorption coefficient of the compound may be increased such that a photoelectric conversion device and/or photoelectric conversion layer including the compound may have improved photoelectric conversion performance and/or efficiency.

In some example embodiments, the compound represented by Chemical Formula 1 is a compound that is configured (e.g., based on the compound being in a thin film state) to selectively absorb light in the visible light wavelength region (e.g., green wavelength region), and may have a maximum absorption wavelength ($\lambda_{max}$) in a wavelength range of greater than or equal to about 520 nm, for example greater than or equal to about 521 nm, greater than or equal to about 522 nm, greater than or equal to about 523 nm, greater than or equal to about 524 nm, or greater than or equal to about 525 nm, and less than or equal to about 540 nm, for example, less than or equal to about 539 nm, less than or equal to about 538 nm, less than or equal to about 537 nm, less than or equal to about 536 nm, or less than or equal to about 535 nm.

In some example embodiments, the compound represented by Chemical Formula 1 has an absorption curve having a full width at half maximum (FWHM) of less than or equal to about 150 nm, for example about 20 nm to about 150 nm, about 20 nm to about 120 nm, about 20 nm to about 110 nm, or about 20 nm to about 100 nm. By having the FWHM in the above range, absorption selectivity for light of a specific wavelength in the visible light wavelength range (for example, green wavelength range) can be increased such that a photoelectric conversion device and/or photoelectric conversion layer including the compound may have improved absorption selectivity and thus photoelectric conversion selectivity in the visible light wavelength range (for example, green wavelength range), and thus a photoelectric conversion device and/or photoelectric conversion layer including the compound may have improved photoelectric conversion performance and/or efficiency with regard to light in the visible light wavelength range (for example, green wavelength range). The thin film may be a thin film deposited under vacuum conditions.

In some example embodiments, the sublimation temperature (temperature formed by vacuum deposition, also referred to as "deposition temperature") of the compound represented by Chemical Formula 1 may be less than or equal to about 250 °C, for example, less than or equal to about 240 °C, or less than or equal to about 235 °C, for example about 100 °C to about 250 °C. Due to the sublimation temperature in the above range, there is little possibility of impurity mixing when forming a thin film by deposition. The sublimation temperature may be confirmed by thermogravimetric analysis (TGA), and may be, for example, a temperature at which a weight loss of 10% relative to an initial weight occurs

during thermogravimetric analysis at a pressure of 10 Pa or less.

In addition, a micro lens array (MLA) needs to be formed to concentrate light after manufacturing an organic photoelectric device during manufacture of an image sensor. Formation of this micro lens array requires a relatively high temperature (greater than or equal to about 160 °C, for example greater than or equal to about 170 °C, greater than or equal to about 180 °C, or greater than or equal to about 190 °C). The performance of the photoelectric devices (e.g., organic photoelectric devices) is required not to be deteriorated in these heat-treatment processes. The performance deterioration of the organic photoelectric device during the heat treatment of MLA may be caused not by chemical decomposition of an organic material but its morphology change. The morphology change is in general caused, when a material starts a thermal vibration due to a heat treatment, but even a material having a firm molecule structure may not have the thermal vibration and be prevented from the deterioration by the heat treatment. The compound represented by Chemical Formula 1 has a ring structure linked by $G^2$ in the donor moiety and thus may be stably maintained during the MLA heat treatment and secure process stability and thereby reducing the likelihood of process defects in a layer, film, and/or device including the compound, and thereby improving the reliability of a device including the compound and/or a layer including the compound, a film including the compound, or the like.

The compound represented by Chemical Formula 1 may be a p-type semiconductor. The compound may have a HOMO energy level in the range of about 4.5 eV to about 6.5 eV, and an energy bandgap of greater than or equal to about 2.0 eV, for example, about 2.0 eV to about 3.0 eV. In this case, the LUMO energy level is located between about 2.5 eV and about 4.5 eV. As the HOMO and LUMO energy levels of the compound of Chemical Formula 1 are controlled, the compound represented by Chemical Formula 1 may be used as a p-type semiconductor.

The n-type semiconductor that can be used with the compound represented by Chemical Formula 1 may include fullerene, a fullerene derivative, sub-phthalocyanine or a sub-phthalocyanine derivative, thiophene or a thiophene derivative, or the compound represented by Chemical Formula 6, or any combination thereof.

A composition including a p-type semiconductor including the compound represented by Chemical Formula 1 and an n-type semiconductor including a fullerene, a fullerene derivative, sub-phthalocyanine or a sub-phthalocyanine derivative, thiophene or a thiophene derivative, or the compound represented by Chemical Formula 6 has excellent absorption in the entire green wavelength range, and the photoelectric conversion efficiency of photoelectric devices and image sensors including the composition may be improved and dark current and remaining charges thereof may be greatly reduced.

The compound represented by Chemical Formula 6 may include a planar core having an imide group or an anhydride group.

[Chemical Formula 6]

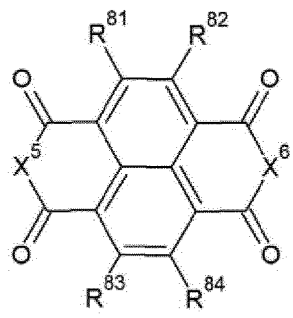

In Chemical Formula 6,

$X^5$ and $X^6$ may each independently be O or $NR^a$, (wherein $R^a$ is hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heterocyclic group, a halogen, or a cyano group), and

$R^{81}$ to $R^{84}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heterocyclic group, a halogen, a cyano group, or any combination thereof.

Examples of the fullerene may include C60, C70, C76, C78, C80, C82, C84, C90, C96, C240, C540, a mixture thereof, a fullerene nanotube, and the like. The fullerene derivative may refer to compounds of these fullerenes having a substituent thereof. The fullerene derivative may include a substituent such as an alkyl group (e.g., C1 to C30 alkyl group), an aryl group (e.g., C6 to C30 aryl group), a heterocyclic group (e.g., C3 to C30 heterocycloalkyl group), and

the like. Examples of the aryl groups and heterocyclic groups may be a benzene ring, a naphthalene ring, an anthracene ring, a phenanthrene ring, a fluorene ring, a triphenylene ring, a naphthacene ring, a biphenyl ring, a pyrrole ring, a furan ring, a thiophene ring, an imidazole ring, an oxazole ring, a thiazole ring, a pyridine ring, a pyrazine ring, a pyrimidine ring, a pyridazine ring, an indolizine ring, an indole ring, a benzofuran ring, a benzothiophene ring, a isobenzofuran ring, a benzimidazole ring, a imidazopyridine ring, a quinolizidine ring, a quinoline ring, a phthalazine ring, a naphthyridine ring, a quinoxaline ring, an isoquinoline ring, a carbazole ring, a phenanthridine ring, an acridine ring, a phenanthroline ring, a thianthrene ring, a chromene ring, an xanthene ring, a phenoxazine ring, a phenoxathiin ring, a phenothiazine ring, or a phenazine ring.

The subphthalocyanine or subphthalocyanine derivative may be represented by Chemical Formula 7.

[Chemical Formula 7]

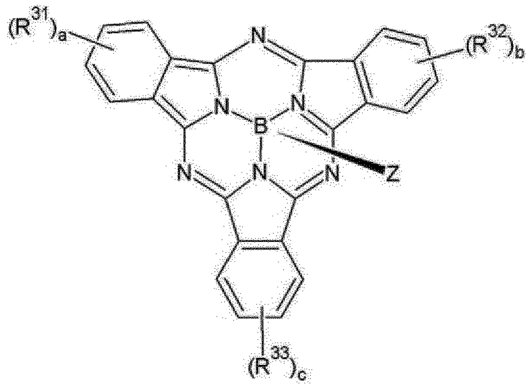

In Chemical Formula 7,

$R^{31}$ to $R^{33}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a halogen, a halogen-containing group, or any combination thereof,

a, b, and c are integers ranging from 1 to 3, and

Z is a monovalent substituent.

For example, Z may be a halogen or a halogen-containing group, for example F, Cl, an F-containing group, or a Cl-containing group.

The halogen refers to F, Cl, Br, or I and the halogen-containing group refers to alkyl group (C1 to C30 alkyl group) where at least one hydrogen of the alkyl group may be replaced by F, Cl, Br, or I.

The thiophene derivative may be for example represented by Chemical Formula 8 or Chemical Formula 9, but is not limited thereto.

[Chemical Formula 8]

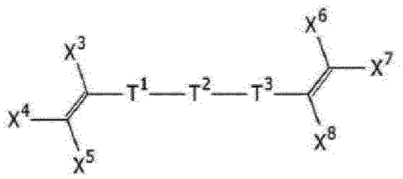

[Chemical Formula 9] $EWG^1\text{-}T^1\text{-}T^2\text{-}T^3\text{-}EWG^2$

In Chemical Formulas 8 and 9,

$T^1$, $T^2$, and $T^3$ may be aromatic rings including substituted or unsubstituted thiophene moieties,

$T^1$, $T^2$, and $T^3$ may each independently be present or may be fused to each other,

$X^3$ to $X^8$ may each independently be hydrogen, deuterium, a substituted or unsubstituted C1 to C30 alkyl group, a

substituted or unsubstituted C1 to C30 alkoxy group, a substituted or unsubstituted C6 to C30 aryl group, a substituted or unsubstituted C3 to C30 heterocyclic group, a cyano group, or any combination thereof, and
$EWG^1$ and $EWG^2$ may each independently be electron withdrawing groups, which may be the same as any example embodiment of EWG as described herein with regard to Chemical Formula 1.

For example, in Chemical Formula 8, at least one of $X^3$ to $X^8$ may be an electron withdrawing group, for example, a cyano group or a cyano-containing group.

For example, specific examples of the compound represented by Chemical Formula 6 include compounds represented by Chemical Formula 6A or 6B.

[Chemical Formula 6A]

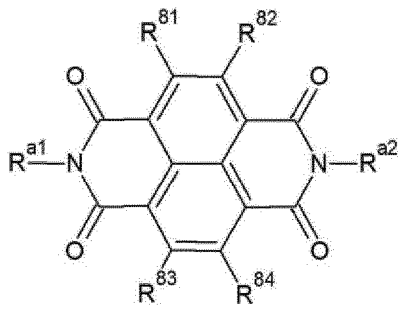

[Chemical Formula 6B]

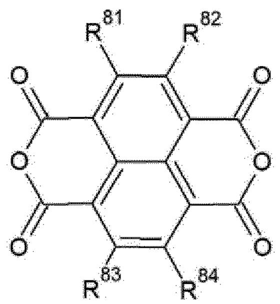

In Chemical Formulas 6A and 6B,
$R^{81}$ to $R^{84}$, $R^{a1}$, and $R^{a2}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heterocyclic group, a halogen, a cyano group, or any combination thereof.

For example, at least one of $R^{a1}$ or $R^{a2}$ may include an electron withdrawing group. For example, $R^{a1}$ and $R^{a2}$ may each include an electron withdrawing group, which may be the same as any example embodiment of EWG as described herein with regard to Chemical Formula 1.

For example, at least one of $R^{a1}$ or $R^{a2}$ may be a halogen; a cyano group; a halogen-substituted C1 to C30 alkyl group; a halogen-substituted C6 to C30 aryl group; a halogen-substituted C3 to C30 heterocyclic group; a cyano-substituted C1 to C30 alkyl group; a cyano-substituted C6 to C30 aryl group; a cyano-substituted C3 to C30 heterocyclic group; a substituted or unsubstituted pyridinyl group; a substituted or unsubstituted pyrimidinyl group; a substituted or unsubstituted triazinyl group; a substituted or unsubstituted pyrazinyl group; a substituted or unsubstituted quinolinyl group; a substituted or unsubstituted isoquinolinyl group; a substituted or unsubstituted quinazolinyl group; a C1 to C30 alkyl group substituted with a substituted or unsubstituted pyridinyl group; a C6 to C30 aryl group substituted with a substituted or unsubstituted pyridinyl group; a C1 to C30 alkyl group substituted with a substituted or unsubstituted pyrimidinyl group; a C6 to C30 aryl group substituted with a substituted or unsubstituted pyrimidinyl group; a C1 to C30 alkyl group substituted with a substituted or unsubstituted triazinyl group; a C6 to C30 aryl group substituted with a substituted or unsubstituted triazinyl group; a C1 to C30 alkyl group substituted with a substituted or unsubstituted pyrazinyl group; a C6 to C30 aryl group substituted with a substituted or unsubstituted pyrazinyl group; a C1 to C30 alkyl group substituted with a substituted or unsubstituted quinolinyl group; a C6 to C30 aryl group substituted with a substituted or unsubstituted quinolinyl group; a C1 to C30 alkyl group substituted with a substituted or unsubstituted isoquinolinyl group; a C6 to C30 aryl group substituted with a substituted or unsubstituted isoquinolinyl group; a C1 to C30 alkyl group substituted with a substituted or unsubstituted quinazolinyl group; a C6 to C30 aryl group substituted with a substituted or unsubstituted quinazolinyl group; or any combination thereof.

For example, $R^{a1}$ and $R^{a2}$ may each independently be a halogen; a cyano group; a halogen-substituted C1 to C30 alkyl group; a halogen-substituted C6 to C30 aryl group; a halogen-substituted C3 to C30 heterocyclic group; a cyano-substituted C1 to C30 alkyl group; a cyano-substituted C6 to C30 aryl group; a cyano-substituted C3 to C30 heterocyclic group; a substituted or unsubstituted pyridinyl group; a substituted or unsubstituted pyrimidinyl group; a substituted or unsubstituted triazinyl group; a substituted or unsubstituted pyrazinyl group; a substituted or unsubstituted quinolinyl group; a substituted or unsubstituted isoquinolinyl group; a substituted or unsubstituted quinazolinyl group; a C1 to C30 alkyl group substituted with a substituted or unsubstituted pyridinyl group; a C6 to C30 aryl group substituted with a substituted or unsubstituted pyridinyl group; a C1 to C30 alkyl group substituted with a substituted or unsubstituted pyrimidinyl group; a C6 to C30 aryl group substituted with a substituted or unsubstituted pyrimidinyl group; a C1 to C30 alkyl group substituted with a substituted or unsubstituted triazinyl group; a C6 to C30 aryl group substituted with a substituted or unsubstituted triazinyl group; a C1 to C30 alkyl group substituted with a substituted or unsubstituted

pyrazinyl group; a C6 to C30 aryl group substituted with a substituted or unsubstituted pyrazinyl group; a C1 to C30 alkyl group substituted with a substituted or unsubstituted quinolinyl group; a C6 to C30 aryl group substituted with a substituted or unsubstituted quinolinyl group; a C1 to C30 alkyl group substituted with a substituted or unsubstituted isoquinolinyl group; a C6 to C30 aryl group substituted with a substituted or unsubstituted isoquinolinyl group; a C1 to C30 alkyl group substituted with a substituted or unsubstituted quinazolinyl group; a C6 to C30 aryl group substituted with a substituted or unsubstituted quinazolinyl group; or any combination thereof.

As an example, $R^{a1}$ and $R^{a2}$ may be the same or different from each other and in some example embodiments, $R^{a1}$ and $R^{a2}$ may be the same.

The compound represented by Chemical Formula 6 may be selected from, for example, the compounds listed in Group 2, but is not limited thereto.

[Group 2]

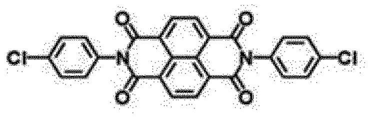
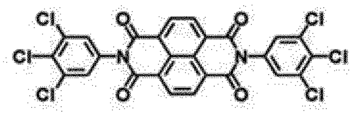

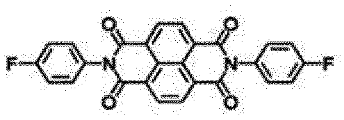
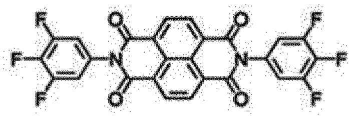

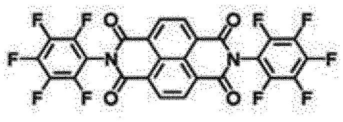
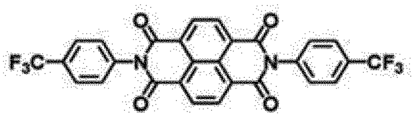

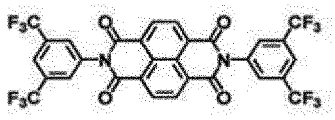
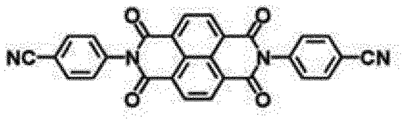

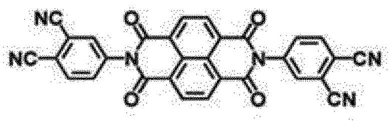
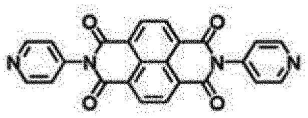

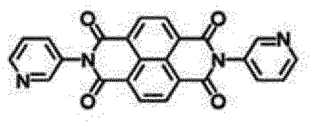
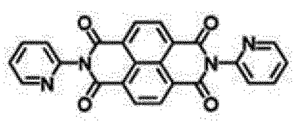

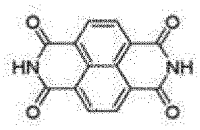
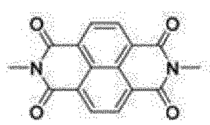
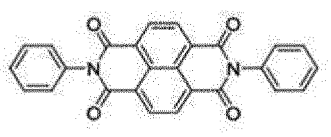

In Group 2,

at least one hydrogen of each aromatic ring or heteroaromatic ring may be optionally replaced by substituent selected from deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heterocyclic group, a halogen, a cyano group, or any combination thereof.

Hereinafter, a photoelectric device according to some example embodiments including the compound will be described with reference to the drawings.

FIG. 1 is a cross-sectional view showing a photoelectric device according to some example embodiments.

Referring to FIG. 1, a photoelectric device 100 according to some example embodiments includes a first electrode 10 and a second electrode 20 (e.g., a first electrode 10 and a second electrode 20 facing each other), and an active layer (also referred to as a light absorbing layer) 30 between the first electrode 10 and the second electrode 20.

One of the first electrode 10 or the second electrode 20 is an anode and the other is a cathode. At least one of the first electrode 10 or the second electrode 20 may be a light-transmitting electrode, and the light-transmitting electrode may be made of, for example, a transparent conductor such as indium tin oxide (ITO) or indium zinc oxide (IZO), or a metal thin layer of a single layer or multilayer. When one of the first electrode 10 or the second electrode 20 is a non-light-transmitting electrode, it may include (e.g., may be made of), for example, an opaque conductor such as aluminum (Al).

In some example embodiments, the active layer 30 may be a layer including a p-type semiconductor and an n-type semiconductor that form (e.g., establish, define, etc.) a pn junction, and absorbs external (e.g., incident) light to generate excitons and then separates the generated excitons into holes and electrons.

The active layer 30 may include the compound represented by Chemical Formula 1.

The active layer 30 (which may, in some example embodiments, be a thin film) may have a maximum absorption wavelength ($\lambda_{max}$) in a wavelength range of greater than or equal to about 520 nm, for example, greater than or equal to about 521 nm, greater than or equal to about 522 nm, greater than or equal to about 523 nm, greater than or equal to about 524 nm, or greater than or equal to about 525 nm, and less than or equal to about 540 nm, for example, less than or equal to about 539 nm, less than or equal to about 538 nm, less than or equal to about 537 nm, less than or equal to about 536 nm, or less than or equal to about 535 nm.

The active layer 30 may exhibit an absorption curve with a relatively small full width at half maximum (FWHM) of about 50 nm to about 200 nm, for example about 50 nm to about 150 nm, about 50 nm to about 120 nm or about 50 nm to about 100 nm. Accordingly, the active layer 30 can have high selectivity for light in the green wavelength range such that the photoelectric conversion device 100 and any device including same (e.g., an image sensor, an electronic device, etc.) may have improved absorption selectivity and thus photoelectric conversion selectivity in the green wavelength range, and thus the photoelectric conversion device 100 and any device including same may have improved photoelectric conversion performance and/or efficiency with regard to light in the green wavelength range.

The active layer 30 may be a single layer or multiple layers. The active layer 30 may be, for example, an intrinsic layer (I layer), a p-type layer/I layer, an I layer/n-type layer, a p-type layer/I layer/n-type layer, a p-type layer/n-type layer, and the like.

The intrinsic layer (I layer) may include the semiconductor compound represented by Chemical Formula 1 and the n-type semiconductor compound according to any of the example embodiments in a volume ratio of about 1:100 to about 100:1. Within the above range, they may be included in the active layer 30 in a volume ratio of about 1:50 to about 50:1, about 1:10 to about 10:1, about 1:5 to about 5: 1, about 1:3 to about 3:1, about 1:2 to about 2:1, about 1:1.5 to about 1.5:1, about 1:1.2 to about 1.2:1, or about 1:1. By being included in the active layer 30 in a volume ratio within the above range, effective exciton generation and pn junction formation may be provided such that the photoelectric conversion device 100 and any device including same may have improved photoelectric conversion performance and/or efficiency.

The p-type layer may include the semiconductor compound of Chemical Formula 1, and the n-type layer may include the n-type semiconductor compound according to any of the example embodiments.

The active layer 30 may have a thickness of about 1 nm to about 500 nm, for example, about 20 nm to about 250 nm, about 30 nm to about 200 nm, about 50 nm to about 150 nm, about 70 nm to about 130 nm, about 80 nm to about 120 nm, or about 5 nm to about 300 nm. Based on the active layer 30 having a thickness within the above range, photoelectric conversion efficiency can be effectively improved by effectively absorbing light and effectively separating and transferring holes and electrons such that the photoelectric conversion device 100 and any device including same may have improved photoelectric conversion performance and/or efficiency. The optimal film thickness of the active layer 30 can be determined, for example, by considering the absorption coefficient of the active layer 30, and for example, it may have a thickness capable of absorbing about 70% or more, for example, about 80% or more, or for example, about 90% of the light.

In the photoelectric device 100, when light enters from the first electrode 10 and/or second electrode 20, and when the active layer 30 absorbs light in a desired and/or alternatively particular (or, alternatively, predetermined) wavelength region, excitons may be produced from the inside. The excitons are separated into holes and electrons in the active layer 30, and the separated holes are transported to an anode that is one of the first electrode 10 or the second electrode 20 and the separated electrons are transported to the cathode that is the other of the first electrode 10 or the second electrode 20 so as to flow a current in (e.g., induce a current through at least a portion of) the photoelectric device 100.

Hereinafter, a photoelectric device according to some example embodiments is described with reference to FIG. 2.

FIG. 2 is a cross-sectional view showing a photoelectric device according to some example embodiments.

Referring to FIG. 2, a photoelectric device 200 according to some example embodiments includes a first electrode 10 and a second electrode 20 facing each other, and an active layer 30 between the first electrode 10 and the second electrode 20, like some example embodiments, including the example embodiments shown in FIG. 1.

However, the photoelectric device 200 according to some example embodiments, including the example embodiments shown in FIG. 2 further includes charge auxiliary layers 40 and 45 between the first electrode 10 and the active layer 30, and the second electrode 20 and the active layer 30, unlike some example embodiments, including the example embodiments shown in FIG. 1. The charge auxiliary layers 40 and 45 may facilitate the transfer of holes and electrons separated from the active layer 30, so as to increase efficiency.

The charge auxiliary layers 40 and 45 may be at least one selected from a hole injection layer (HIL) for facilitating hole injection, a hole transport layer (HTL) for facilitating hole transport, an electron blocking layer (EBL) for preventing electron transport, an electron injection layer (EIL) for facilitating electron injection, an electron transport layer (ETL) for facilitating electron transport, and a hole blocking layer (HBL) for preventing hole transport.

The charge auxiliary layers 40 and 45 may include, for example, an organic material, an inorganic material, or an organic/inorganic material. The organic material may be an organic compound having hole or electron characteristics, and the inorganic material may be, for example, a metal oxide such as molybdenum oxide, tungsten oxide, nickel oxide, and the like.

The hole injection layer (HIL) and/or hole transport layer (HTL) may include one selected from, for example, poly(3,4-ethylenedioxythiophene):poly(styrenesulfonate) (PEDOT:PSS), polyarylamine, poly(N-vinylcarbazole), polyaniline, polypyrrole, N,N,N',N'-tetrakis(4-methoxyphenyl)-benzidine (TPD), 4,4'-bis[N-(1-naphthyl)-N-phenyl-amino]biphenyl ($\alpha$-NPD), m-MTDATA (4,4',4"-tris[phenyl(m-tolyl)amino]triphenylamine), 4,4'4"-tris(N-carbazolyl)-triphenylamine (TCTA), and any combination thereof, but is not limited thereto.

The electron blocking layer (EBL) may include one selected from, for example, poly(3,4-ethylenedioxythiophene):poly(styrenesulfonate) (PEDOT:PSS), polyarylamine, poly(N-vinylcarbazole), polyaniline, polypyrrole, N,N,N',N'-tetrakis(4-methoxyphenyl)-benzidine (TPD), 4,4'-bis[N-(1-naphthyl)-N-phenyl-amino]biphenyl ($\alpha$-NPD), m-MTDATA, 4,4',4"-tris(N-carbazolyl)-triphenylamine (TCTA), and any combination thereof, but is not limited thereto.

The electron injection layer (EIL) and/or electron transport layer (ETL) may include one selected from, for example, 1,4,5,8-naphthalene-tetracarboxylic dianhydride (NTCDA), bathocuproine (BCP), LiF, $Alq_3$, $Gaq_3$, $Inq_3$, $Znq_2$, $Zn(BTZ)_2$, $BeBq_2$, and any combination thereof, but is not limited thereto.

The hole blocking layer (HBL) may include one selected from, for example, 1,4,5,8-naphthalene-tetracarboxylic dianhydride (NTCDA), bathocuproine (BCP), LiF, $Alq_3$, $Gaq_3$, $Inq_3$, $Znq_2$, $Zn(BTZ)_2$, $BeBq_2$, and any combination thereof, but is not limited thereto.

Either one of the charge auxiliary layers 40 or 45 may be omitted.

The photoelectric device may be applied to various fields, for example a solar cell, an image sensor, a photodetector, a photo-sensor, and an organic light emitting diode (OLED), but is not limited thereto.

Hereinafter, an example of an image sensor including the organic photoelectric device is described referring to drawings. As an example of an image sensor, an organic CMOS image sensor is described.

FIG. 3 is a schematic top plan view showing an organic CMOS image sensor according to some example embodiments, and FIG. 4 is a cross-sectional view showing the organic CMOS image sensor of FIG. 3 according to some example embodiments.

Referring to FIGS. 3 and 4, an organic CMOS image sensor 300 according to some example embodiments, including the example embodiments shown in FIGS. 3 and 4 includes a semiconductor substrate 310 integrated with a photo-sensing device 50, a transmission transistor (not shown), a charge storage 55, a lower insulation layer 60, a color filter layer 70, an upper insulation layer 80 (also referred to herein as an insulation layer), and a photoelectric device 100. The photoelectric device 100 may be the photoelectric device according to any of the example embodiments and is on (e.g., directly or indirectly on) the semiconductor substrate 310.

The semiconductor substrate 310 may be a silicon substrate, and is integrated with a photo-sensing device 50, the transmission transistor (not shown), and the charge storage 55. The photo-sensing device 50 may include a blue photo-sensing device 50B and a red photo-sensing device 50R. The blue photo-sensing device 50B and the red photo-sensing device 50R may be photodiodes.

The photo-sensing device 50, the transmission transistor, and/or the charge storage 55 may be integrated in each pixel, for example may be integrated in the semiconductor substrate 310 such that the photo-sensing device 50 (e.g., photo-sensing devices 50B and 50R) is located within a volume space defined by one or more outermost surfaces of the semiconductor substrate 310 and may be at least partially exposed by the semiconductor substrate 310 or may be enclosed within an interior of the semiconductor substrate 310, and as shown in the drawing, the photo-sensing device 50 may be respectively included in a blue pixel and a red pixel and the charge storage 55 may be included in a green pixel. The blue photo-sensing device 50B may be configured to sense (e.g., selectively sense, including selectively absorbing and photoelectrically converting) blue light which is light in a blue wavelength region, and the red photo-sensing device 50R may be configured to sense (e.g., selectively sense, including selectively absorbing and photoelectrically converting) red light which is light in a red wavelength region.

The blue photo-sensing device 50B may be configured to sense (e.g., selectively sense, including selectively absorbing and photoelectrically converting) blue light which is light in a blue wavelength region, and the red photo-sensing device 50R may be configured to sense (e.g., selectively sense, including selectively absorbing and photoelectrically converting) red light which is light in a red wavelength region.

The photo-sensing device 50 may be configured to sense (e.g., selectively sense) light, the information sensed by the photo-sensing device 50 may be transferred by the transmission transistor, the charge storage 55 is electrically connected to the photoelectric device 100, and the information of the charge storage 55 may be transferred by the transmission transistor.

In the drawings, the blue photo-sensing device 50B and the red photo-sensing device 50R are, for example, arranged in parallel without limitation, and the blue photo-sensing device 50B and the red photo-sensing device 50R may be stacked in a vertical direction.

A metal wire (not shown) and a pad (not shown) are formed on the semiconductor substrate 310. In order to decrease signal delay, the metal wire and pad may be made of a metal having low resistivity, for example, aluminum (Al), copper (Cu), silver (Ag), and alloys thereof, but are not limited thereto. Further, it is not limited to the structure, and the metal wire and pad may be positioned under the photo-sensing device 50.

The lower insulation layer 60 is formed on the metal wire and the pad. The lower insulation layer 60 may be made of an inorganic insulating material such as a silicon oxide and/or a silicon nitride, or a low dielectric constant (low K) material such as SiC, SiCOH, SiCO, and/or SiOF. The lower insulation layer 60 has a trench exposing the charge storage 55. The trench may be filled with fillers.

A color filter layer 70 is formed on the lower insulation layer 60. In some example embodiments, the color filter layer 70 may be located between the photoelectric device 100 and the semiconductor substrate 310. The color filter layer 70 includes a blue filter 70B formed in the blue pixel and configured to selectively transmit blue light and a red filter 70R formed in the red pixel and configured to selectively transmit red light. In some example embodiments, a cyan filter and a yellow filter may be disposed instead of the blue filter 70B and red filter 70R. In some example embodiments, including the example embodiments shown in FIGS. 3 and 4, a green filter is not included, but a green filter may be further included.

The color filter layer 70 may be omitted. For example, when the blue photo-sensing device 50B and the red photo-sensing device 50R are stacked in a vertical direction, the blue photo-sensing device 50B and the red photo-sensing device 50R may selectively absorb and/or sense light in each wavelength region depending on their stack depth, and the color filter layer 70 may not be equipped.

The upper insulation layer 80 is formed on the color filter layer 70. The upper insulation layer 80 eliminates a step caused by the color filter layer 70 and smoothens the surface. The upper insulation layer 80 and the lower insulation layer 60 may include a contact hole (not shown) exposing a pad, and a through-hole 85 exposing the charge storage 55 of the green pixel.

The aforementioned photoelectric device 100 is formed on (e.g., directly or indirectly on) the upper insulation layer 80. The photoelectric device 100 includes the first electrode 10, the active layer 30, and the second electrode 20 as described above.

The first electrode 10 and the second electrode 20 may be transparent electrodes, and the active layer 30 is the same as described above. The active layer 30 may selectively absorb and/or sense light in a green wavelength region and replaces a color filter of a green pixel. Accordingly, the photoelectric device 100 may be on the semiconductor substrate 310 and may be configured to selectively sense light in the green wavelength region.

When light enters from the second electrode 20, the light in a green wavelength region may be mainly absorbed in the active layer 30 and photoelectrically converted, while the light in the rest of the wavelength regions passes through first electrode 10 and may be sensed in the photo-sensing device 50.

As described above, the photoelectric devices configured to selectively absorb light in a green wavelength region are stacked and thereby a size of an image sensor may be decreased and a down-sized image sensor may be realized.

As described above, the compound represented by the Chemical Formula 1 may be used as a p-type semiconductor compound, aggregation between compounds in a thin film state is inhibited, and thereby light absorption characteristics depending on a wavelength may be maintained. Thereby, green wavelength selectivity may be maintained, crosstalk caused by unnecessary absorption of other light except a green wavelength region may be decreased and sensitivity may be increased such that the image sensor 300 and any device including same may have improved photoelectric conversion performance and/or efficiency.

In some example embodiments, in FIG. 4, additional color filters may be further disposed on the photoelectric device 100. The additional color filters may include a blue filter 70B and a red filter 70R or a cyan filter and a yellow filter.

The organic CMOS image sensor with the color filters disposed on the photoelectric device is shown in FIG. 5.

FIG. 5 is a schematic cross-sectional view showing an organic CMOS image sensor according to some example embodiments. Referring to FIG. 5, an organic CMOS image sensor 400 has the same structure as FIG. 4 except that a color filter layer 72 including the blue filter 72B and the red filter 72R is disposed on the photoelectric device 100 such that the photoelectric device 100 is between the color filter layer 72 and the semiconductor substrate 310. Instead of the blue filter 72B and the red filter 72R, the cyan filter and the yellow filter may be disposed respectively.

In FIGS. 4 and 5, the photoelectric device 100 of FIG. 1 is included, but example embodiments are not limited thereto, and thus the photoelectric device 200 of FIG. 2 may be applied in the same manner.

FIG. 6 is a cross-sectional view showing an organic CMOS image sensor 500 to which the photoelectric device 200 is applied according to some example embodiments.

Referring to FIG. 6, the organic CMOS image sensor 500 includes a semiconductor substrate 310 integrated with photo-sensing devices 50B and 50R, a transmission transistor (not shown), a charge storage 55, a lower insulation layer 60, an upper insulation layer 80, and a photoelectric device 200, like some example embodiments, including the example embodiments shown in FIG. 4. However, the organic CMOS image sensor 500 according to some example embodiments, including the example embodiments shown in FIG. 6, includes the photoelectric device 200, unlike some example embodiments, including the example embodiments shown in FIG. 4, which include the photoelectric device 100.

FIG. 7 is a schematic view showing an organic CMOS image sensor according to some example embodiments.

Referring to FIG. 7, the organic CMOS image sensor 600 includes a semiconductor substrate 310 integrated with photo-sensing devices 50B and 50R, a transmission transistor (not shown), and a charge storage 55, an insulation layer 80, and a photoelectric device 100, like some example embodiments, including the example embodiments illustrated in FIG. 4.

However, the organic CMOS image sensor 600 according to some example embodiments includes the blue photo-sensing device 50B and the red photo-sensing device 50R that are stacked in a vertical direction (e.g., perpendicular to a direction in which the upper surface of the semiconductor substrate 310 extends as shown in FIG. 7) in the semiconductor substrate 310 and does not include a color filter layer 70, unlike some example embodiments, including the example embodiments shown in FIG. 4. For example, each red photo-sensing device 50R of a plurality of red photo-sensing devices 50R integrated in the semiconductor substrate 310 may be stacked in a vertical direction with a separate blue photo-sensing device 50B of a plurality of blue photo-sensing devices 50B in the semiconductor substrate 310. The blue photo-sensing device 50B and the red photo-sensing device 50R are electrically connected with the charge storage 55, and the information of the charge storage 55 may be transferred by the transmission transistor (not shown). The blue photo-sensing device 50B and the red photo-sensing device 50R may selectively absorb light in each wavelength region depending on a stack depth.

As described above, the photoelectric devices selectively absorbing light in a green wavelength region are stacked and the red photo-sensing device and the blue photo-sensing device are stacked, and thereby a size of an image sensor may be decreased and a down-sized image sensor may be realized. As described above, the photoelectric device 100 has improved green wavelength selectivity, and crosstalk caused by unnecessary absorption of light in a wavelength region except green may be decreased while increasing sensitivity.

In FIG. 7, the photoelectric device 100 of FIG. 1 is included, but example embodiments are not limited thereto, and thus the photoelectric device 200 of FIG. 2 may be applied in the same manner.

FIG. 8 is a schematic view showing an organic CMOS image sensor according to some example embodiments and FIG. 9 is a cross-sectional view of the organic CMOS image sensor of FIG. 8 according to some example embodiments.

Referring to FIGS. 8 and 9, the organic CMOS image sensor 700 according to some example embodiments includes a green photoelectric device (G) configured to selectively absorb light in a green wavelength region, a blue photoelectric device (B) configured to selectively absorb light in a blue wavelength region, and a red photoelectric device (R) configured to selectively absorb light in a red wavelength region that are stacked. For example, the organic CMOS image sensor 700 may include a green photoelectric device configured to selectively sense light in a green wavelength region, a blue photoelectric device configured to selectively sense light in a blue wavelength region, and a red photoelectric device configured to selectively sense light in a red wavelength region, where the green photoelectric device, the blue photoelectric device, and the red photoelectric device are stacked as shown in at least FIG. 8. As shown, the photoelectric devices 100a to 100c may be stacked in a vertical direction on the semiconductor substrate 310, such that the photoelectric devices 100a to 100c at least partially overlap each other in a vertical direction that is perpendicular to an upper surface 40s of the semiconductor substrate 310, but example embodiments are not limited thereto.

The organic CMOS image sensor 700 according to some example embodiments includes a semiconductor substrate 310, a lower insulation layer 60, an intermediate insulation layer 65, an upper insulation layer 80, a first device (i.e., photoelectric device, the same below) 100a, a second device 100b, and a third device 100c. Herein, the device refers to a photoelectric device.

The semiconductor substrate 310 may be a silicon substrate, and a transmission transistor (not shown) and charge storages 155a, 155b, and 155c are integrated therein.

Metal wires (not shown) and pads (not shown) are formed on the semiconductor substrate 310, and the lower insulation layer 60 is formed on the metal wires and the pads.

The first device 100a, the second device 100b, and the third device 100c are sequentially formed on the lower insulation layer 60.

Any one of the first, second, and third devices 100a, 100b, and 100c may be the photoelectric devices 100 and 200 (green photoelectric device according to example embodiments) of FIG. 1 or 2, and the other two of the first, second, and third devices 100a, 100b, and 100c (a red photoelectric device and a blue photoelectric device) may have the same structure as the photoelectric devices 100 and 200, but an active layer 30 therein selectively absorbs light in a red or blue wavelength region to photoelectrically convert the light. Detailed descriptions of the photoelectric devices 100 and 200 are the same as described above. The first electrode 10 or the second electrode 20 of the photoelectric devices 100 and 200, the red photoelectric device and the blue photoelectric device may be connected to the charge storages 155a, 155b, and 155c.

The active layer 30 of the first device 100a may selectively absorb light in any one wavelength region of red, blue, or green to photoelectrically convert the light. For example, the first device 100a may be a red photoelectric conversion device configured to selectively sense light in a red wavelength region. The first electrode 10 and the second electrode 20 of the first device 100a may be electrically connected to the first charge storage 155a. A "photoelectric conversion device" may be interchangeably referred to herein as a "photoelectric device."

The intermediate insulation layer 65 may be formed on the first device 100a and the second device 100b may be formed on the intermediate insulation layer 65.

The active layer 30 of the second device 100b may selectively absorb light in any one wavelength region of red, blue, or green to photoelectrically convert the light. For example, the second device 100b may be a green photoelectric conversion device configured to selectively sense light in a green wavelength region. In another example, the second device 100b may be a blue photoelectric conversion device configured to selectively sense light in a blue wavelength region. The first electrode 10 and the second electrode 20 of the second device 100b may be electrically connected to the second charge storage 155b.

The upper insulation layer 80 is formed on the second device 100b. The lower insulation layer 60, the intermediate insulation layer 65, and the upper insulation layer 80 have a plurality of through-holes 85a, 85b, and 85c exposing the charge storages 155a, 155b, and 155c.

The third device 100c is formed on the upper insulation layer 80. The active layer 30 of the third device 100c may selectively absorb light in any one wavelength region of red, blue, and green to photoelectrically convert the light. For example, the third device 100c may be a blue photoelectric conversion device configured to selectively sense light in a blue wavelength region. In another example, the third device 100c may be a green photoelectric conversion device configured to selectively sense light in a green wavelength region. The first electrode 10 and the second electrode 20 of the third device 100c may be electrically connected to the third charge storage 155c.

A focusing lens (not shown) may be further formed on the third device 100c. The focusing lens may control the direction of incident light and gather the light in one region. The focusing lens may have the shape of, for example, a cylinder or a hemisphere, but is not limited thereto.

In the drawing, a structure in which the first device 100a, the second device 100b, and the third device 100c are sequentially stacked, but example embodiments are not limited thereto, and the stacking order may be variously changed.

As described above, the first device 100a, the second device 100b, and the third device 100c that absorb light in different wavelength regions have a stacked structure, further reducing a size of the image sensor, implementing a down-sized image sensor, and simultaneously increasing sensitivity and reducing a crosstalk.

FIG. 10 is a perspective view schematically showing an example of an organic CMOS image sensor according to some example embodiments, and FIG. 11 schematically shows a view of a cross-section of an example of the organic CMOS image sensor of FIG. 10 taken along line XI-XI' of FIG. 10 according to some example embodiments.

Referring to FIGS. 10 and 11, the organic CMOS image sensor 800 according to some example embodiments, as in some example embodiments, including the example embodiments shown in at least FIG. 4, includes the semiconductor substrate 310 and the insulation layer 80. The organic CMOS image sensor 800 may include an optical filter 250 which may be configured to selectively transmit light of various wavelength regions (e.g., selectively transmit visible light). In some example embodiments, the optical filter 250 may be omitted. The organic CMOS image sensor 800 includes multiple photoelectric devices 100aa, 100bb, 100cc, and 100dd which are stacked horizontally on the semiconductor substrate 310, for example stacked in a direction that is parallel to the upper surface 40s of the semiconductor substrate 310 such that the photoelectric devices 100aa to 100dd at least partially overlap each other in a horizontal direction that is parallel to the upper surface 40s of the semiconductor substrate 310.

As shown, for example, in FIG. 10, photoelectric devices 100aa and 100cc may be blue photoelectric devices configured to selectively sense light in a blue wavelength region, photoelectric device 100bb may be a green photoelectric device configured to selectively sense light in a green wavelength region, and photoelectric device 100dd may be a red

photoelectric device configured to selectively sensor light in a red wavelength region, but example embodiments are not limited thereto, and each of the photoelectric devices 100aa to 100dd may be configured to selectively sense light of any wavelength region, including visible or non-visible (e.g., infrared or ultraviolet) light, provided that at least one of the photoelectric devices 100aa to 100dd is the green photoelectric device according to the example embodiments, such as the photoelectric device 100 or 200 as shown in FIG. 1 or 2.

As shown, the photoelectric devices 100aa to 100dd may be aligned side by side along a plane direction (e.g., XY direction) of the semiconductor substrate 310 and respectively connected, via respective through-holes 85, to respective charge storages 155a to 155d integrated in the semiconductor substrate 310. As shown, the respective first electrodes 210a to 210d, active layers 230a to 230d, and second electrodes 220a to 220d of the photoelectric devices 100aa to 100dd may be aligned side by side along a plane direction (e.g., XY direction) of the semiconductor substrate 310 (e.g., overlap in the horizontal direction).

At least one of the photoelectric devices 100aa to 100dd may each independently be the photoelectric devices 100 or 200 (green photoelectric device according to example embodiments) of FIG. 1 or 2, and the others (including a red photoelectric device and a blue photoelectric device) may have the same structure as the photoelectric devices 100 and 200, but an active layer 30 therein selectively absorbs light in a red or blue wavelength region to photoelectrically convert the light. Detailed descriptions of the photoelectric devices 100 and 200 are the same as described above. The first electrode 10 or the second electrode 20 of the photoelectric devices 100 and 200, the red photoelectric device, and the blue photoelectric device may be connected to the charge storages 155a, 155b, and 155c.

The photoelectric device 100aa includes a first electrode 210a, an active layer 230a, and a second electrode 220a. The photoelectric device 100bb includes a first electrode 210b, an active layer 230b, and a second electrode 220b. The photoelectric device 100cc includes a first electrode 210c, an active layer 230c, and a second electrode 220c. The photoelectric device 100dd includes a first electrode 210d, an active layer 230d, and a second electrode 220d.

The first electrodes 210a to 210d may each be the same as the first electrodes 10 of the photoelectric devices 100 and 200 of FIG. 1 or 2. The second electrodes 220a to 220d may each be the same as the second electrodes 20 of the photoelectric devices 100 and 200 of FIG. 1 or 2. The active layers 230a to 230d may each be the same as the active layers 30 of the photoelectric devices 100 and 200 of FIG. 1 or 2. At least some of the active layers 230a to 230d may have different material compositions and may be configured to sense (e.g., selectively absorb and photoelectrically convert) light in different wavelength regions. The active layers 230a and 230c may each be configured to selectively absorb light in the blue wavelength region and photoelectrically convert the absorbed light, the active layer 230b may be configured to selectively absorb light in the green wavelength region and photoelectrically convert the absorbed light, and the active layer 230d may be configured to absorb light in the red wavelength region and photoelectrically convert the absorbed light.

In some example embodiments, some or all of the second electrodes 220a to 220d may be separate portions of a single, continuous piece of material that extends between each of the photoelectric devices 100aa to 100dd to establish a common electrode, while the first electrodes 210a to 210d may be separate pieces of material in separate photoelectric devices 100aa to 100dd. In some example embodiments, some or all of the first electrodes 210a to 210d may be separate portions of a single, continuous piece of material that extends between each of the photoelectric devices 100aa to 100dd to establish a common electrode, while the second electrodes 220a to 220d may be separate pieces of material in separate photoelectric devices 100aa to 100dd.

The image sensor absorbs light in an appropriate wavelength region and may show all improved sensitivity (YSNR10) and color reproducibility ($\Delta E^*ab$) despite a stacked structure.

Herein, the YSNR10 indicates sensitivity of the image sensor, which is measured in a method described in Juha Alakarhu's "Image Sensors and Image Quality in Mobile Phones" printed in 2007 International Image Sensor Workshop (Ogunquit Maine, USA) but minimum illuminance expressed by lux at a ratio of 10 between signal and noise. Accordingly, the smaller the YSNR10 is, the higher sensitivity is.

On the other hand, the color reproducibility ($\Delta E^*ab$) shows a difference from standard colors in an X-Rite chart, and the $\Delta E^*ab$ (also indicated as simply $\Delta E$) is defined as a distance between two points on a L*a*b* color space by CIE (Commission International de L' Eclairage) in 1976. For example, the color difference may be calculated according to Equation 1.

[Equation 1]

$$\Delta E = \sqrt{(\Delta L^*)^2 + (\Delta a^*)^2 + (\Delta b^*)^2}$$

In Equation 1,

ΔE denotes the color reproducibility (also denoted as ΔE*ab herein),
ΔL* denotes a change of a color coordinate L* compared with the color coordinate L* at room temperature (about 20 °C to about 25 °C),
Δa* denotes a change of a color coordinate a* compared with the color coordinate a* at room temperature (about 20 °C to about 25 °C), and
Δb* denotes a change of a color coordinate b* compared with the color coordinate b* at room temperature (about 20 °C to about 25 °C).

In order to manufacture an image sensor having high sensitivity and high color reproducibility, YSNR10≤100lux at ΔE*ab≤3, and herein, the compound may realize YSNR10≤100 lux of sensitivity and color reproducibility at ΔE*ab≤3.
The image sensor may be applied to (e.g., included in) various electronic devices, for example, a mobile phone, a digital camera, and the like but is not limited thereto.
FIG. 12 is a block diagram of a digital camera including an image sensor according to some example embodiments.
Referring to FIG. 12, a digital camera 1000 includes a lens 1010, an image sensor 1020, a motor 1030, and an engine 1040. The image sensor 1020 may be one of image sensors according to some example embodiments, including any of the example embodiments shown in FIGS. 3 to 11.
The lens 1010 concentrates incident light on the image sensor 1020. The image sensor 1020 generates RGB data for received light through the lens 1010.
In some example embodiments, the image sensor 1020 may interface with the engine 1040.
The motor 1030 may adjust the focus of the lens 1010 or perform shuttering in response to a control signal received from the engine 1040. The engine 1040 may control the image sensor 1020 and the motor 1030.
The engine 1040 may be connected to a host/application 1050.
FIG. 13 is a schematic view showing an electronic device according to some example embodiments.
Referring to FIG. 13, an electronic device 1100 may include a processor 1120, a memory 1130, and an image sensor 1140 that are electrically coupled together via a bus 1110.
The image sensor 1140 may be an image sensor, organic CMOS image sensor, or the like according to any of the example embodiments, including any of the example embodiments shown in FIGS. 3 to 11 of the present application. The memory 1130 may be a non-transitory computer readable medium and may store a program of instructions. The memory 1130 may be a nonvolatile memory, such as a flash memory, a phase-change random access memory (PRAM), a magneto-resistive RAM (MRAM), a resistive RAM (ReRAM), or a ferro-electric RAM (FRAM), or a volatile memory, such as a static RAM (SRAM), a dynamic RAM (DRAM), or a synchronous DRAM (SDRAM). The processor 1120 may execute the stored program of instructions to perform one or more functions. For example, the processor 1120 may be configured to process electrical signals generated by the image sensor 1140. The processor 1120 may include processing circuitry such as hardware including logic circuits; a hardware/software combination such as a processor executing software; or any combination thereof. For example, the processing circuitry more specifically may include, but is not limited to, a central processing unit (CPU), an arithmetic logic unit (ALU), a digital signal processor, a microcomputer, a field programmable gate array (FPGA), a System-on-Chip (SoC), a programmable logic unit, a microprocessor, application-specific integrated circuit (ASIC), etc. The processor 1120 may be configured to generate an output (e.g., an image to be displayed on a display interface) based on such processing.
One or more of the processor 1120, memory 1130, motor 1030, engine 1040, or host/application 1050 may be included in, include, and/or implement one or more instances of processing circuitry such as hardware including logic circuits, a hardware/software combination such as a processor executing software; or any combination thereof. In some example embodiments, said one or more instances of processing circuitry may include, but are not limited to, a central processing unit (CPU), an application processor (AP), an arithmetic logic unit (ALU), a graphic processing unit (GPU), a digital signal processor, a microcomputer, a field programmable gate array (FPGA), a System-on-Chip (SoC), a programmable logic unit, a microprocessor, or an application-specific integrated circuit (ASIC), etc. In some example embodiments, any of the memories, memory units, or the like as described herein may include a non-transitory computer readable storage device, for example a solid state drive (SSD), storing a program of instructions, and the one or more instances of processing circuitry may be configured to execute the program of instructions to implement the functionality of some or all of any of the processor 1120, memory 1130, motor 1030, engine 1040, or host/application 1050, or the like according to any of the example embodiments as described herein.
Hereinafter, some example embodiments are illustrated in more detail with reference to examples. However, the scope of the example embodiments of the inventive concepts is not limited to these examples.

**Synthesis Examples**

**Synthesis Example** 1-1: **Synthesis of Compound Represented by Chemical** Formula 1-1

[Chemical Formula 1-1]

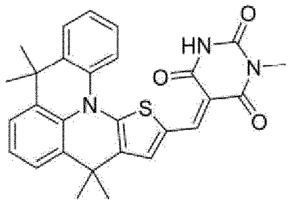

[Reaction Scheme 1-1]

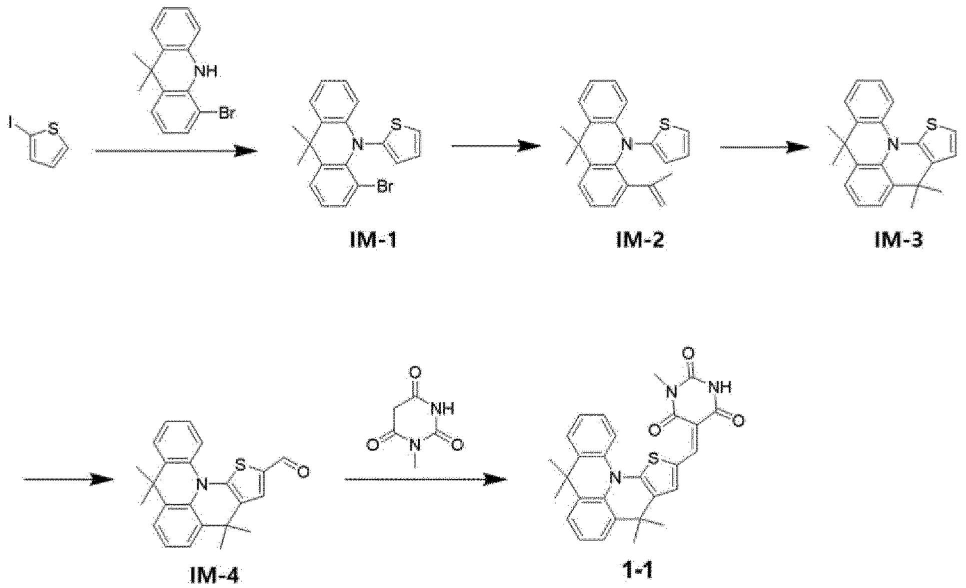

IM-1 IM-2 IM-3

IM-4 1-1

(i) Synthesis of Compound IM-1

19.7 g (93.7 mmol) of 2-iodothiophene, 9.00 g (31.2 mmol) of 4-bromo-9,9-dimethyl-9,10)-dihydroacridine, 0.99 g (5.2 mmol) of copper(I) iodide, 0.16 ml (1.4) mmol) of trans-1,2-diaminocyclohexane, and 2.95 g (13.9 mmol) of potassium phosphate ($K_3PO_4$) are transferred to a round-bottomed flask, and then 80 ml of 1,4-dioxane is added thereto, and refluxed for 12 hours. Then, the product is filtered through Celite, the filtrate is concentrated, and the product is separated and purified through silica gel column chromatography (hexane:dichloromethane = volume ratio of 19:1). Thereby, 9.0 g of compound IM-1 (yield: 78%) is obtained.

(ii) Synthesis of Compound IM-2

compound IM-1 8.00 g (21.6 mmol), 2.5 g (2.2 mmol) of palladium-tetrakis(triphenylphosphine) ($Pd(PPh_3)_4$), and 7.46 g (54.0 mmol) of potassium carbonate ($K_2CO_3$) are transferred to a round-bottomed flask and stirred in 100 ml of tetrahydrofuran and 25 ml of water ($H_2O$). 12.2 ml (64.8 mmol) of 2-isopropenylboronic acid pinacol ester is added thereto and refluxed for 5 hours. The product is extracted with ethyl acetate and dried with anhydrous magnesium sulfate. The product obtained at this time is separated and purified through silica gel column chromatography (hexane: dichloromethane = volume ratio of 4:1), and 9.00 g of Compound IM-2 (yield: 89%) is obtained.

(iii) Synthesis of Compound IM-3

5.00 g (15.1 mmol) of Compound IM-2 is dissolved in 500 ml of toluene. 9.7 ml (150 mmol) of methanesulfonic acid is added dropwise and stirred for 12 hours. The product is poured into ice water, neutralized by adding 2 M aqueous sodium hydroxide solution, and the organic layer extracted with toluene is washed with aqueous sodium chloride solution. Anhydrous magnesium sulfate is added to the organic layer, dried, concentrated, and separated and purified through silica gel column chromatography (hexane:dichloromethane = volume ratio of 9:1) to obtain 2.0 g (yield: 40%) of Compound IM-3.

(iv) Synthesis of Compound IM-4

1.5 ml (16.0 mmol) of phosphoryl chloride ($POCl_3$) is added dropwise to 6.5 ml (84.0 mmol) of N,N-dimethylformamide (DMF) at 0 °C, and then stirred at room temperature (24 °C) for 2 hours. This solution is slowly dropped into a solution of 1.00 g (4.08 mmol) of Compound IM-3 in 50 ml of dichloromethane at 0 °C, and then stirred at room temperature for 1 hour. Then, water is added to the product, 2 M aqueous sodium hydroxide solution is added until the pH value reached 14, and then stirred at room temperature for 2 hours. The organic layer extracted with dichloromethane is washed with aqueous sodium chloride solution, dried with anhydrous magnesium sulfate, and then concentrated. The product obtained through this is separated and purified through silica gel column chromatography (volume ratio changed from hexane: dichloromethane = 3:2 to dichloromethane 100%) to obtain 1.1 g (yield 75%) of Compound IM-4.

(v) Synthesis of Compound Represented by Chemical Formula 1-1

1.00 g (2.78 mmol) of Compound IM-4 is suspended in 100 ml of ethanol, and 0.44 g (3.10 mmol) of 1-methylpyrimidine-2,4,6(1H,3H,5H)-trione is added, stirred at 50 °C for 4 hours, and concentrated under reduced pressure. After silica gel filtering, recrystallization is performed using chloroform and ethanol to obtain 1.05 g (yield: 78%) of the compound represented by Chemical Formula 1-1. The compound is purified by sublimation to a purity of 99.9%.
$^1$H-NMR (500 MHz, $CD_2Cl_2$): δ 8.56 (s,1H), 8.54 (s, 1H), 8.23 (s, 1H), 8.06-8.05 (m, 2H), 8.04 (s, 1H), 7.88 (s, 2H), 7.60 (t, 2H), 7.43-7.26 (m, 10H), 3.31 (s, 3H), 3.27 (s, 3H), 1.58 (s, 12H), 1.55 (s, 12H).

**Synthesis Example 1-2: Synthesis of Compound Represented by Chemical Formula 1-2**

[Chemical Formula 1-2]

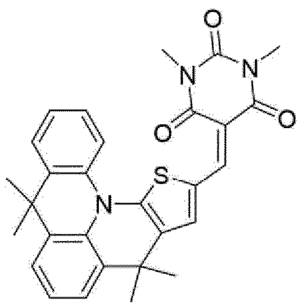

The compound represented by Chemical Formula 1-2 is obtained in the same manner as in Synthesis Example 1-1, except that 1,3-dimethylbarbituric acid is used instead of 1-methylpyrimidine-2,4,6(1H,3H,5H)-trione in step (v) of Synthesis Example 1-1. The obtained compound (represented by Chemical Formula 1-2) is purified by sublimation to a purity of 99.9%.
$^1$H-NMR (500 MHz, $CD_2Cl_2$): δ 8.71 (s, 1H), 8.23 (d, 1H), 8.15 (s, 1H), 7.62 (d, 1H), 7.49-7.24 (m, 5H), 3.37 (s, 3H), 3.30 (s, 3H), 1.61 (s, 12H).

**Synthesis Example 1-3: Synthesis of Compound Represented by Chemical** Formula 1-3

[Chemical Formula 1-3]

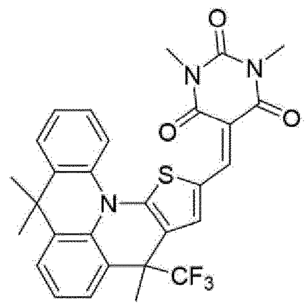

[Reaction Scheme 1-3]

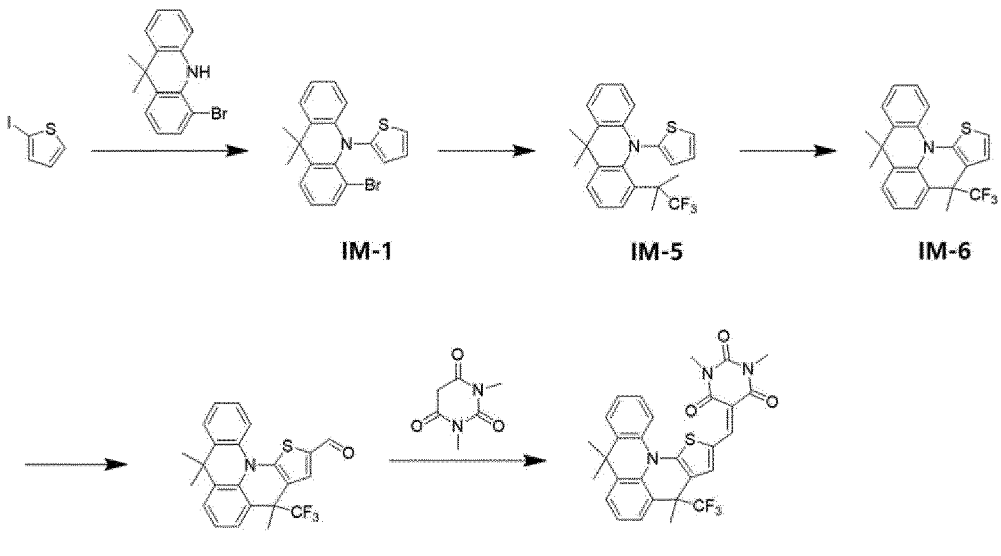

IM-1 IM-5 IM-6

IM-7 1-3

(i) Synthesis of Compound IM-5

8.00 g (21.7 mmol) of Compound IM-1 is dissolved in 150 ml of dehydrated diethyl ether. 9.5 ml (23.8 mmol) of 2.5 M n-BuLi in hexane solution (2.5 M) is slowly added at -70 °C and stirred for 1 hour. 2.3 ml (25.7 mmol) of 1,1,1-trifluoroacetone is added at -70 °C and stirred at room temperature for 1 hour. The product is washed with aqueous sodium chloride solution, extracted with ethyl acetate, and dried with anhydrous magnesium sulfate. The product obtained at this time is separated and purified through silica gel column chromatography (hexane: dichloromethane = volume ratio of 2:3), and 4.8 g (yield: 42%) of Compound IM-5 is obtained.

(ii) Synthesis of Compound IM-6

Compound IM-6 is synthesized in the same manner as the synthesis of Compound IM-3 in step (iii) of Synthesis Example 1-1, except that Compound IM-5 is used instead of Compound IM-2.

(iii) Synthesis of Compound IM-7

Compound IM-7 is synthesized in the same manner as the synthesis of Compound IM-4 in step (iv) of Synthesis Example 1-1, except that Compound IM-6 is used instead of Compound IM-3.

(iv) Synthesis of Compound represented by Chemical Formula 1-3

The compound represented by Chemical Formula 1-3 is obtained in the same manner as in Synthesis Example 1, except that 1,3-dimethylbarbituric acid and Compound IM-7 are used instead of 1-methylpyrimidine-2,4,6(1H,3H,5H)-trione and Compound IM-4 in step (v) of Synthesis Example 1-1, respectively. The obtained compound is purified by sublimation to a purity of 99.9%.

$^{1}$H-NMR (500 MHz, $CD_2Cl_2$): δ 8.19 (s, 1H), 8.14 (m, 1H), 7.59 (m, 2H), 7.54 (d, 1H), 7.50 (d, 1H), 7.35-7.31 (m, 3H), 3.35 (s, 3H), 3.30 (s, 3H), 2.10 (s, 3H), 1.94 (s, 3H), 1.34 (s, 3H).

**Synthesis Example 1-4: Synthesis of Compound Represented by Chemical Formula 1-4**

[Chemical Formula 1-4]

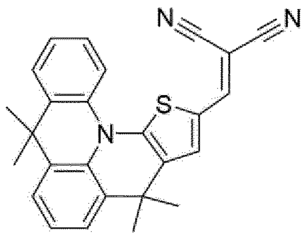

The compound represented by Chemical Formula 1-4 is obtained in the same manner as in Synthesis Example 1, except that malononitrile is used instead of 1-methylpyrimidine-2,4,6(1H,3H,5H)-trione in step (v) of Synthesis Example 1-1. The obtained compound is purified by sublimation to a purity of 99.9%.

$^{1}$H-NMR (500 MHz, $CD_2Cl_2$): δ 8.11 (s, 1H), 7.52-7.44 (m, 3H), 7.34 (d, 1H), 7.29 (d, 1H), 7.24-7.11 (m, 2H), 6.41 (s, 1H), 1.61 (s, 12H).

**Comparative Synthesis Example 1-1C: Synthesis of Compound Represented by Chemical Formula 1-1C**

[Chemical Formula 1-1C]

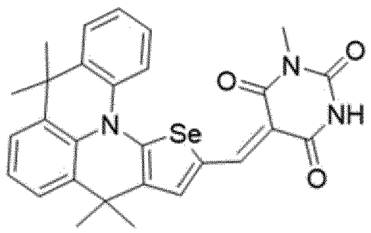

[Reaction Scheme 1-1C]

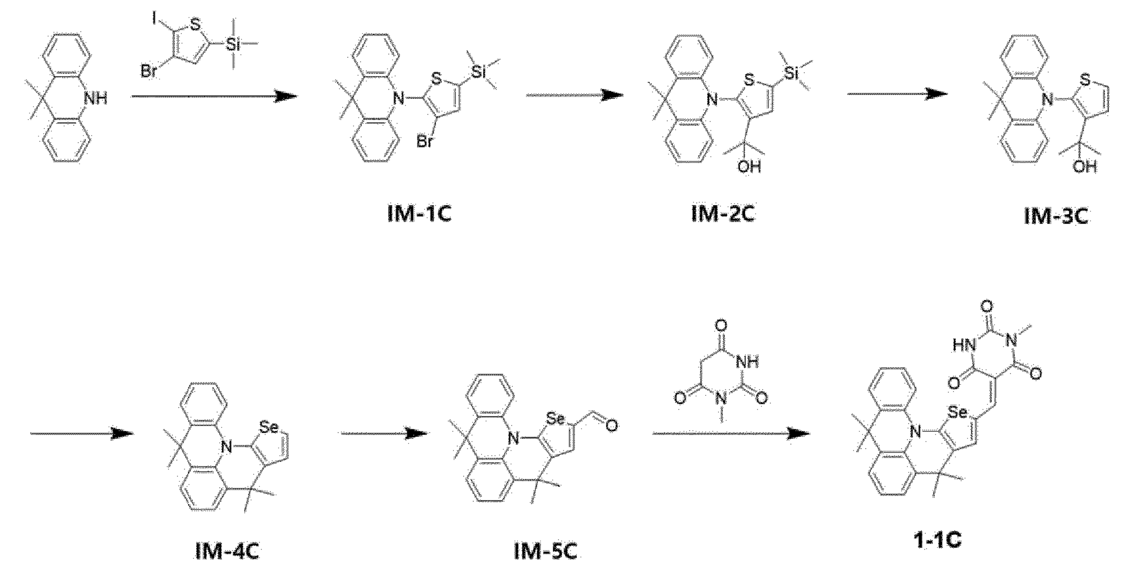

IM-1C IM-2C IM-3C

IM-4C IM-5C 1-1C

(i) Synthesis of Compound IM-1C

2.10 g (5.16 mmol) of (4-bromo-5-iodoselenophen-2-yl)trimethylsilane) and 0.900 g (4.30 mmol) of 9,9-dimethyl-9,10-dihydroacridine are transferred to a microwave vial and dissolved in 12 ml of toluene. 0.247 g (0.43 mmol) of bis(dibenzylideneacetone)palladium ($Pd(dba)_2$), 0.34 ml (0.86 mmol) of 50 wt% triphenylphosphine ($P(PPh)_3$) solution, and 1.24 g (12.9 mmol) of sodium tert-butoxide (NaOtBu) are added and reacted in a microwave reactor at 100 °C for 30 minutes. Then, the product is filtered through Celite, the filtrate is concentrated, and the product is separated and purified through silica gel column chromatography (hexane:dichloromethane = volume ratio of 19:1). Thereby, 1.2 g of Compound IM-1C (yield: 57%) is obtained.

(ii) Synthesis of Compound IM-2C

Compound IM-2C is obtained by using the same method as the synthesis method of Compound IM-5 except that dehydrated acetone is used instead of 1,1,1-trifluoroacetone in step (i) of Synthesis Example 1-3.

(iii) Synthesis of Compound IM-3C

5.43 g (12.1 mmol) of Compound IM-2C is dissolved in 120 ml of tetrahydrofuran. 13.3 ml (13.3 mmol) of tetra-n-butylammonium fluoride (TBAF) is added dropwise and stirred for 1 hour. The reaction product is washed with aqueous sodium chloride solution, and then anhydrous magnesium sulfate is added to the organic layer and dried. The product obtained at this time is separated and purified through silica gel column chromatography (hexane: dichloromethane = volume ratio of 9: 1), resulting in 4.2 g of Compound IM-3C (yield: 92%).

(iv) Synthesis of compound IM-4C

2.16 g (5.71 mmol) of Compound IM-3C is dissolved in 250 ml of toluene. 3.71 ml (57.1 mmol) of methanesulfonic acid is added dropwise and stirred for 12 hours. The product is poured into ice water, neutralized by adding 2M aqueous sodium hydroxide solution, and the organic layer extracted with toluene is washed with aqueous sodium chloride solution. Anhydrous magnesium sulfate is added to the organic layer, dried, concentrated, and the product is separated and purified through silica gel column chromatography (hexane:dichloromethane = volume ratio of 9:1) to obtain 1.4 g (yield: 63%) of Compound IM-4C.

(v) Synthesis of Compound IM-5C

Compound IM-5C is synthesized in the same manner as the synthesis of Compound IM-4 in step (iv) of Synthesis Example 1-1, except that Compound IM-4C is used instead of Compound IM-3.

(vi) Synthesis of Compound Represented by Chemical Formula 1-1C

The compound represented by Chemical Formula 1-1C is synthesized using the same method as the synthesis of Compound 1-1 in step (v) of Synthesis Example 1-1, except that Compound IM-5C is used instead of Compound IM-4. $^1$H-NMR (500 MHz, $CD_2Cl_2$): δ 8.61 (s,1H), 8.58 (s, 1H), 8.22-8.16 (m, 2H), 8.13 (s, 1H), 7.48 (s, 1H), 7.49-7.28 (m, 8H), 7.05 (t, 2H), 6.99 (t, 2H), 6.61 (d, 2H), 3.30 (s, 3H), 3.26 (s, 3H), 1.67 (s, 12H), 1.63 (s, 12H).

**Comparative Synthesis Example 1-2C: Synthesis of Compound Represented by Chemical Formula 1-2C**

[Chemical Formula 1-2C]

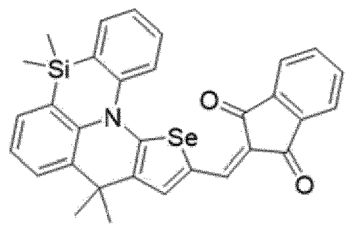

(i) Synthesis of Compound Represented by Chemical Formula 1-2C

The compound represented by Chemical Formula 1-2C is synthesized in the same manner as Comparative Synthesis Example 1-1C, except that 10,10-dimethyl-5,10-dihydrodibenzo[b,e][1,4]azasiline) is used instead of 9,9-dimethyl-9,10-dihydroacridine in step (i) of Comparative Synthesis Example 1-1C, and 1H-indene-1,3(2H)-dione is used instead of 1-methylpyrimidine-2,4,6(1H,3H,5H)-trione in step (vi) of Comparative Synthesis Example 1-1C.

$^1$H-NMR (500 MHz, $CD_2Cl_2$): δ 8.04 (d, 1H), 7.98 (s, 2H), 7.83-7.81 (m, 1H), 7.78-7.76 (m, 1H), 7.70-7.67 (m, 3H), 7.52 (dd, 1H), 7.47 (dd, 1H), 7.44 (td, 1H), 7.35 (td, 1H), 7.28 (t, 1H), 1.92 (s, 3H), 1.37 (s, 3H), 0.72 (s, 3H), 0.27 (s, 3H).

**Synthesis Example 2-1: Synthesis of Compound Represented by Chemical Formula 2-1**

[Chemical Formula 2-1]

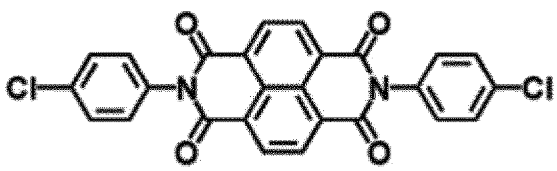

A mixture of 1,4,5,8-naphthalenetetracarboxylic dianhydride (1 eq.) and 4-chloroaniline (2.2 eq.) is dissolved in a dimethyl formamide (DMF) solvent and placed in a two-necked round-bottomed flask at 180 °C for 24 hours. Then, the temperature is lowered to room temperature, methanol is added thereto, the product is precipitated, and the product is filtered to obtain a powder-like material. The material is then washed with methanol several times and purified by recrystallization using ethyl acetate and dimethyl sulfoxide (DMSO). The obtained product is then placed in an oven and dried in vacuum at a temperature of 80 °C for 24 hours to obtain a compound represented by Chemical Formula 2-1. A yield is 50% or more.

$^1$H NMR (300 MHz, $CDCl_3$ with Hexafluoroisopropanol): δ = 8.85 (s, 4H), 7.63 (s, 4H), 7.60 (s, 4H).

**Synthesis Example 2-2: Synthesis of Compound Represented by Chemical Formula 2-2**

[Chemical Formula 2-2]

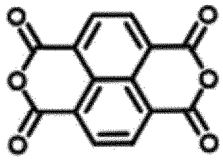

The compound represented by Chemical Formula 2-2 (manufactured by Tokyo Chemical Industry) is prepared by sublimation purification so that it can be used as an n-type semiconductor in photoelectric devices.

**Synthesis Example 2-3**

Fullerene ($C_{60}$, nanom purple ST, manufactured by Frontier Carbon Corporation) is prepared so that it can be used as an n-type semiconductor in photoelectric devices.

**Evaluation I: Reorganization Energy and Maximum Absorption Wavelength of Compounds**

The reorganization energy and DFT calculated maximum absorption wavelength of the compounds according to Synthesis Examples 1-1 to 1-4 and Comparative Synthesis Examples 1-1C and 1-2C are calculated at DFT B3LYP/DGDZVP level using the Gaussian 09 program. Then, corrected maximum absorption wavelength of a thin film including each of the compounds is calculated. The results are shown in Table 1.

(Table 1)

| | Reorganization energy (eV) | DFT calculated maximum absorption wavelength (nm) | Corrected maximum absorption wavelength (nm)[1] |
|---|---|---|---|
| Synthesis Example 1-1 | 0.188 | 499.76 | 532.76 |
| Synthesis Example 1-2 | 0.191 | 497.59 | 530.30 |
| Synthesis Example 1-3 | 0.187 | 489.29 | 523.22 |
| Synthesis Example 1-4 | 0.122 | 491.34 | 520.90 |
| Comparative Synthesis Example 1-1C | 0.195 | 509.47 | 543.75 |
| Comparative Synthesis Example 1-2C | 0.234 | 521.07 | 556.89 |
| 1) The corrected maximum absorption wavelength is a value calculated using Equation 2. | | | |

corrected maximum absorption wavelenth (nm) = 1.1323 X (DFT calculated maximum absorption wavelength (nm)) - 33.12 [equation 2]

Referring to Table 1, the compounds of Synthesis Examples 1-1 to 1-4 exhibit low reorganization energies of 0.122 to 0.191 eV, the DFT calculated maximum absorption wavelength of 489.29 nm to 499.76 nm, and the corrected maximum absorption wavelength of 520.90 nm to 532.76 nm, showing absorption characteristics in the green wavelength range. In comparison, the compounds of Comparative Synthesis Example 1-1C and Comparative Synthesis Example 1-2C exhibit higher reorganization energy values and maximum absorption wavelength at a longer wavelength compared to the compounds according to Synthesis Examples 1-1 to 1-4.

**Evaluation II: Polarizability, Dipole Moment and Oscillator Strength of Compounds**

Polarizability, a dipole moment, and oscillator strength according to a wavelength of the compounds according to Synthesis Examples 1-1 to 1-4 are obtained at the DFT B3LYP/DGDZVP level by using the Gaussian 09 program. The results are shown in Table 2.

(Table 2)

| | Polarizability ($Bohr^3$) | Dipole moment (Debye) | Oscillator Strength (a.u.) |
|---|---|---|---|
| Synthesis Example 1-1 | 427.51 | 7.4 | 0.81 |
| Synthesis Example 1-2 | 439.86 | 6.9 | 0.82 |
| Synthesis Example 1-3 | 436.47 | 6.4 | 0.79 |
| Synthesis Example 1-4 | 371.50 | 10.6 | 0.69 |

Referring to Table 2, the compounds of Synthesis Examples 1-1 to 1-4 have excellent electrical properties and absorption coefficients because they exhibit a polarization ratio of 450 $Bohr^3$ or less, a dipole moment of 5 Debye to 11 Debye, and an oscillator strength of 0.65 or more.

**Evaluation III: Energy Level and Energy Bandgap of Compounds**

The compounds according to Synthesis Examples 1-1 to 1-4 are respectively deposited on a glass substrate, and energy levels of the deposited thin films are measured. HOMO energy levels are evaluated with an amount of photoelectrons emitted by energy when irradiating UV light to a thin film using AC-2 (Hitachi) or AC-3 (Riken Keiki Co., Ltd.). Energy bandgaps are obtained by using a UV-Vis spectrometer (Shimadzu Corp.). Then, LUMO energy levels are calculated by using the energy bandgaps and the HOMO energy levels. The results are shown in Table 3.

(Table 3)

| | HOMO (eV) | LUMO (eV) | Energy bandgap (eV) |
|---|---|---|---|
| Synthesis Example 1-1 | 5.60 | 2.71 | 2.89 |
| Synthesis Example 1-2 | 5.55 | 2.71 | 2.84 |
| Synthesis Example 1-3 | 5.74 | 2.78 | 2.96 |
| Synthesis Example 1-4 | 5.76 | 2.84 | 2.92 |
| * HOMO, LUMO: absolute value | | | |

Referring to Table 3, the compounds according to Synthesis Example 1-1 to Synthesis Example 1-4 can be used as a p-type semiconductor.

**Evaluation IV: Sublimation Temperature of Compounds**

The sublimation temperatures of the compounds obtained in Synthesis Examples and Comparative Synthesis Examples are evaluated.

The sublimation temperature is evaluated through thermogravimetric analysis (TGA) from a temperature where the weight of a sample decreases by 10% relative to the initial weight by increasing the temperature under high vacuum (about 10 Pa or less).

The results are shown in Table 4.

(Table 4)

| | $T_{s\ (10,}$ °C) |
|---|---|
| Synthesis Example 1-1 | 210 |
| Synthesis Example 1-2 | 200 |
| Comparative Synthesis Example 1-1C | 253 |
| * $T_{s(10)}$ (°C): A temperature (sublimation temperature) where a weight of a sample decreases by 10% relative to the initial weight of the sample | |

Referring to Table 4, the sublimation temperatures of the compounds according to Synthesis Examples 1-1

and 1-2 is lower than that of the compound according to Comparative Synthesis Example 1-1C, showing that deposition stability thereof is improved.

## Example A

### Example 1-1A: Manufacture of Photoelectric Device

Al (10 nm), ITO (100 nm), and Al (8 nm) are sequentially deposited on a glass substrate to form a lower electrode with an Al/ITO/Al structure (work function: 4.9 eV). Next, N,N'-bis(3-methylphenyl)-N,N'-diphenyl-[1,1-biphenyl]-4,4'-diamine is deposited on the lower electrode to form a hole auxiliary layer (HOMO: 5.30 to 5.70 eV, LUMO: 2.00 to 2.30 eV). Then, the compound represented by Chemical Formula 1-1 obtained in Synthesis Example 1-1 is deposited on the hole auxiliary layer at a rate of 0.25 Å/s to form a p-type layer (10 nm), and the compound represented by Chemical Formula 2-1 according to Synthesis Example 2-1 is deposited at a rate of 0.25 Å/s to form an n-type layer (40 nm), to form an active layer. Then, 4,7-diphenyl-1,10-phenanthroline is deposited on the active layer to form an electron auxiliary layer (HOMO: 6.10 to 6.40 eV, LUMO: 2.90 to 3.20 eV). Then, magnesium and silver are deposited on the electron auxiliary layer to form a Mg:Ag (in a volume ratio of 1:10) upper electrode to manufacture a photoelectric device.

### Examples 1-2A to 1-4A: Manufacture of Photoelectric Device

Each photoelectric device according to Examples 1-2A to 1-4A is manufactured in the same manner as in Example 1-1A, except that each of the compounds represented by Chemical Formulas 1-2 to 1-4 according to Synthesis Examples 1-2 to 1-4 is used, instead of the compound represented by Chemical Formula 1-1 according to Synthesis Example 1-1.

### Comparative Examples 1-1CA and 1-2CA: Manufacture of Photoelectric Device

Each photoelectric device according to Comparative Examples 1-1CA and 1-2CA is manufactured in the same manner as in Example 1-1A, except that the compound represented by Chemical Formula 1-1C or 1-2C according to Comparative Synthesis Example 1-1C or 1-2C is used, instead of the compound represented by Chemical Formula 1-1 according to Synthesis Example 1-1.

## Example B

### Example 1-1B: Manufacture of Photoelectric Device

ITO is laminated on a glass substrate through sputtering to form an about 150 nm-thick anode, and the ITO glass substrate is ultrasonic wave-cleaned with acetone/isopropyl alcohol/pure water respectively for 15 minutes and then, UV ozone-cleaned. Subsequently, the compound according to Synthesis Example 1-1 and C60 are codeposited on the anode in a volume ratio of 1:1 to form a 100 nm-thick active layer, and ITO is vacuum-deposited on the active layer to be 7 nm thick to manufacture a photoelectric device having a structure of ITO (150 nm)/active layer (100 nm)/ITO (7 nm).

### Examples 1-2B to 1-4B: Manufacture of Photoelectric Device

Each photoelectric device (sensor) according to Examples 1-2B to 1-4B is manufactured in the same manner as in Example 1-1B, except that each of the compounds represented by Chemical Formulas 1-2 to 1-4 according to Synthesis Examples 1-2 to 1-4 is used, respectively, instead of the compound represented by Chemical Formula 1-1 according to Synthesis Example 1-1.

### Comparative Examples 1-1CB and 1-2CB: Manufacture of Photoelectric Device

Each photoelectric device according to Comparative Examples 1-1CB and 1-2CB is manufactured in the same manner as in Example 1-1B, except that the compound represented by Chemical Formula 1-1C or 1-2C according to Comparative Synthesis Example 1-1C or 1-2C is used, instead of the compound represented by Chemical Formula 1-1 according to Synthesis Example 1-1.

**Evaluation IV: Evaluation of External Quantum Efficiency of Photoelectric Devices**

External quantum efficiency ($EQE_{max}$) of the photoelectric devices according to Example 1-1A and Comparative Example 1-1CA at room temperature (25 °C, "pristine") and high temperature (left at 85 °C for 1 hour) are evaluated, and the external quantum efficiency of the photoelectric devices according to Example 1-1A and Comparative Example 1-1CA at 450 nm (blue, B), 530 nm (green, G), and 630 nm (red, R) wavelengths are evaluated. External quantum efficiency is evaluated by Incident Photon to Current Efficiency (IPCE) equipment. The results are shown in Table 5.

(Table 5)

| | $EQE_{max}$ (%) | | EQE (3V, B/G/R, %) (85 °C, 1h) |
|---|---|---|---|
| | pristine | 85 °C, 1h | |
| Example 1-1A | 54.7 | 55.6 | 2.3/54.5/0.1 |
| Comparative Example 1-1CA | 37.3 | 35.2 | 0.9/31.5/0.1 |

Referring to Table 5, the photoelectric device according to Example 1-1A exhibits improved external quantum efficiency (photoelectric conversion efficiency) at room temperature and high temperature in the green wavelength spectrum compared to the photoelectric device according to Comparative Example 1-1CA.

For the photoelectric devices according to Example 1-1B and Comparative Example 1-1CB, each absorption curve according to wavelength is obtained in the ultraviolet-visible (UV-Vis) region using Cary 5000 UV spectrometer (manufactured by Varian), to measure maximum absorption wavelength ($\lambda_{max}$), full width at half maximum (FWHM) and absorption coefficients. Additionally, external quantum efficiency is evaluated with the Incident Photon to Current Efficiency (IPCE) instrument at wavelengths of 450 nm (blue, B), 530 nm (green, G), and 630 nm (red, R). The results are shown in Table 6.

(Table 6)

| | $\lambda_{max}$ (nm) | FWHM (nm) | Absorption coefficient ($10^4$ $cm^{-1}$) | $EQE_{max}$ (%) | EQE (3V, B/G/R, %) |
|---|---|---|---|---|---|
| Example 1-1B | 530 | 107 | 7.4 | 65 | 19/65/17 |
| Comparative Example 1-1CB | 550 | 110 | 6.4 | 62 | 15/60/27 |

Referring to Table 6, the photoelectric device according to Example 1-1B exhibits a maximum absorption wavelength in the wavelength range of 520 nm to 540 nm, a small FWHM and a high absorption coefficient, and thus has excellent green wavelength selectivity. In addition, the photoelectric device according to Example 1-1B exhibits improved external quantum efficiency (photoelectric conversion efficiency) in the green wavelength spectrum compared to the photoelectric device according to Comparative Example 1-1CB. In addition, the photoelectric device of Example 1-1B exhibits a higher absorption coefficient (absorption intensity) than the photoelectric device of Comparative Example 1-1CB.

**Evaluation V: Evaluation of Remaining Charge Characteristics**

The remaining charges of the photoelectric device according to Example 1-1A are evaluated.

When photoelectrically converted charges are not all used for signal treatment but remain in one frame, the charges in the former frame are overlapped and read with charges in the following frame, and herein, an amount of the charges in the following frame is called to be an amount of remaining charges. The amount of the remaining charges is measured by irradiating light in the green wavelength region of 532 nm where the photoelectric conversion may occur for desired and/or alternatively predetermined time, turning off the light, and integrating the current measured in units of $10^{-6}$ seconds with an oscilloscope equipment, by time. The amount of the remaining charges is evaluated by a $mA/cm^2$ unit based on 5000 lux light. The results are shown in Table 7.

(Table 7)

| | Remaining charge (mA/$cm^2$) | |
|---|---|---|
| | Room temperature (25 °C) | High temperature (85 °C, 1h) |
| Example 1-1A | 8.9 X $10^{-6}$ | 6.4 X $10^{-6}$ |

Referring to Table 7, the photoelectric device according to Example 1-1A has a lower number of remaining charges at room temperature and high temperature.

While the inventive concepts have been described in connection with what is presently considered to be practical example embodiments, it is to be understood that the inventive concepts are not limited to such example embodiments. On the contrary, the inventive concepts are intended to cover various modifications and equivalent arrangements included within the scope of the appended claims.

**<Description of symbols>**

| | | | | | |
|---|---|---|---|---|---|
| 10: | first electrode | 20: | second electrode | | |
| 30: | active layer | 40, 45: | charge auxiliary layer | | |
| 100, 200, 100a, 100b, 100c, 100aa, 100bb, 100cc, 100dd: | photoelectric device | | | | |
| 300, 400, 500, 600, 700, 800: | organic CMOS image sensor | | | | |
| 310: | semiconductor substrate | 70B, 72B: | blue filter | 70R, 72R: | red filter |
| 70, 72: | color filter layer | 85, 85a, 85b, 85c: | through-hole | | |
| 60: | lower insulation layer | 80: | upper insulation layer | | |
| 50B, 50R: | photo-sensing device | 55: | charge storage | | |
| 1000: | digital camera | 1010: | lens | | |
| 1020: | image sensor | 1030: | motor | | |
| 1040: | engine | 1050: | host/ application | | |

## Claims

1. A compound represented by Chemical Formula 1:

[Chemical Formula 1]

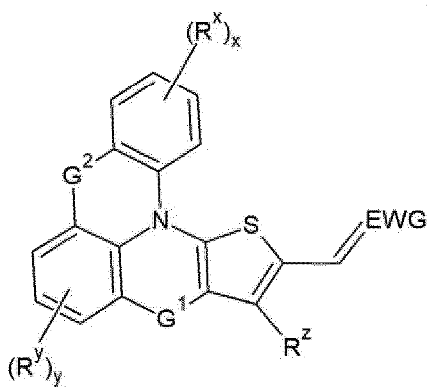

wherein, in Chemical Formula 1,

$G^1$ is a single bond, O, S, Se, Te, S(=O), $S(=O)_2$, $NR^a$, $BR^b$, $-SiR^cR^d-$, $-SiR^{cc}R^{dd}-$, $-GeR^eR^f-$, $-GeR^{ee}R^{ff}-$, $-(CR^gR^h)_{n1}-$, $-(CR^{gg}R^{hh})-$, $-(C(R^i)=C(R^j))-$, or $-(C(R^{ii})=C(R^{jj}))-$, wherein $R^a$, $R^b$, $R^c$, $R^d$, $R^e$, $R^f$, $R^g$, $R^h$, $R^i$ and $R^j$ are each independently hydrogen, deuterium, a halogen, a C1 to C10 alkyl group, a C1 to C10 haloalkyl group, a C1 to C10 alkoxy group, or a C6 to C20 aryl group, $R^{cc}$ and $R^{dd}$, $R^{ee}$ and $R^{ff}$, $R^{gg}$ and $R^{hh}$, or $R^{ii}$ and $R^{jj}$ are linked to each other to form a ring structure, and n1 is 1 or 2,
$G^2$ is O, S, Se, Te, S(=O), $S(=O)_2$, $NR^a$, $BR^b$, $-SiR^cR^d-$, $-SiR^{cc}R^{dd}-$, $-GeR^eR^f-$, $-GeR^{ee}R^{ff}-$, $-(CR^gR^h)n_1-$, $-(CR^{gg}R^{hh})-$, $-(C(R^i)=C(R^j))-$ or $-(C(R^{ii})=C(R^{jj}))-$, wherein $R^a$, $R^b$, $R^c$, $R^d$, $R^e$, $R^f$, $R^g$, $R^h$, $R^i$ and $R^j$ are each independently hydrogen, deuterium, a halogen, a C1 to C10 alkyl group, a C1 to C10 haloalkyl group, a C1 to C10 alkoxy group, or a C6 to C20 aryl group, $R^{cc}$ and $R^{dd}$, $R^{ee}$ and $R^{ff}$, $R^{gg}$ and $R^{hh}$, or $R^{ii}$ and $R^{jj}$ are linked to each other to form a ring structure, and n1 is 1 or 2,
$R^x$, $R^y$, and $R^z$ are each independently hydrogen, deuterium, a halogen, a C1 to C10 alkyl group, a C1 to C10 haloalkyl group, a C1 to C10 alkoxy group, or a C6 to C20 aryl group,
x is an integer of 0 to 4,
y is an integer of 0 to 3, and
EWG is an acceptor moiety including at least one electron withdrawing group,

wherein the compound has a reorganization energy of less than or equal to 0.191 eV, and the compound has a maximum absorption wavelength value calculated based on density functional theory (DFT) of less than or equal to 500 nm.

**2.** The compound of claim 1, wherein in Chemical Formula 1, at least one -CH= of a benzene ring of a $G^2$-containing cyclic group is optionally replaced by -N=; and/or
wherein in Chemical Formula 1, nitrogen (N) is included at a 1st position of a $G^2$-containing cyclic group.

**3.** The compound of claims 1 or 2, wherein in Chemical Formula 1, $R^x$, $R^y$, and $R^z$ are each independently a C1 to C10 alkyl group or a C1 to C10 alkoxy group; and/or
wherein in Chemical Formula 1, the ring structure is a substituted or unsubstituted C5 to C30 hydrocarbon cyclic group or a substituted or unsubstituted C2 to C30 heterocyclic group.

**4.** The compound of any of claims 1-3, wherein
in Chemical Formula 1, the ring structure includes a moiety represented by Chemical Formula 2:

[Chemical Formula 2]

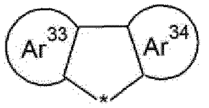

wherein, in Chemical Formula 2,

$Ar^{33}$ and $Ar^{34}$ are each independently a substituted or unsubstituted C6 to C30 arene group, a substituted or unsubstituted C3 to C30 heteroarene group, or a condensed ring thereof, and
* is a point linked to Chemical Formula 1.

**5.** The compound of any of claims 1-4, wherein in Chemical Formula 1, EWG is a substituted or unsubstituted C6 to C30 hydrocarbon ring group including at least one functional group selected from

C=O,
C=S,
C=Se,
C=Te, and
$C=CR^pR^q$, wherein $R^p$ and $R^q$ are each independently hydrogen, deuterium, a substituted or unsubstituted C1 to C10 alkyl group, a cyano group, or a cyano-containing group,
a substituted or unsubstituted C2 to C30 heterocyclic group including at least one functional group selected from C=O, C=S, C=Se, C=Te, and $C=CR^pR^q$, wherein $R^p$ and $R^q$ are each independently hydrogen, deuterium, a substituted or unsubstituted C1 to C10 alkyl group, a cyano group, or a cyano-containing group, or
a fused ring thereof,
$CR^pR^q$, wherein $R^p$ and $R^q$ are each independently hydrogen, deuterium, a substituted or unsubstituted C1 to C10 alkyl group, a cyano group, or a cyano-containing group, or
a C2 to C20 alkyl group including at least one functional group selected from C=O, C=S, C=Se, C=Te, and $C=CR^pR^q$, wherein $R^p$ and $R^q$ are each independently hydrogen, deuterium, a substituted or unsubstituted C1 to C10 alkyl group, a cyano group, or a cyano-containing group.

**6.** The compound of any of claims 1-5, wherein in Chemical Formula 1, EWG is a cyclic group represented by Chemical Formula 4:

[Chemical Formula 4]

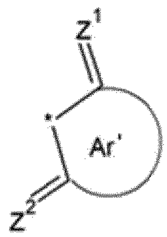

wherein, in Chemical Formula 4,

Ar' is a substituted or unsubstituted C6 to C30 aryl group or a substituted or unsubstituted C3 to C30 heteroaryl group,
$Z^1$ and $Z^2$ are each independently O, S, Se, Te, or $CR^aR^b$, wherein $R^a$ and $R^b$ are each independently hydrogen, deuterium, a substituted or unsubstituted C1 to C10 alkyl group, a cyano group, or a cyano-containing group, and
* is a linking portion linked to Chemical Formula 1.

**7.** The compound of any of claims 1-6, wherein in Chemical Formula 1, EWG is a cyclic group represented by any one of Chemical Formula 5A to Chemical Formula 5H:

[Chemical Formula 5A]

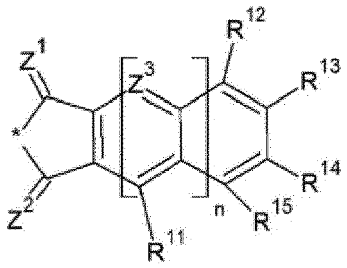

wherein, in Chemical Formula 5A,

$Z^1$ and $Z^2$ are each independently O, S, Se, Te, or $CR^aR^b$, wherein $R^a$ and $R^b$ are each independently hydrogen, deuterium, a substituted or unsubstituted C1 to C10 alkyl group, a cyano group, or a cyano-containing group,
$Z^3$ is N or $CR^c$, wherein $R^c$ is hydrogen, deuterium or a substituted or unsubstituted C1 to C10 alkyl group,
$R^{11}$, $R^{12}$, $R^{13}$, $R^{14}$, and $R^{15}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), a cyano-containing group, or any combination thereof, wherein $R^{12}$ and $R^{13}$ and $R^{14}$ and $R^{15}$ are each independently present or are linked to each other to form a fused aromatic ring,
n is 0 or 1, and
* is a linking portion linked to Chemical Formula 1,

[Chemical Formula 5B]

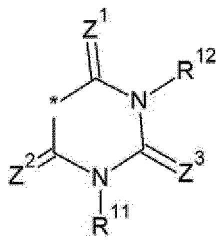

wherein, in Chemical Formula 5B,

$Z^1$ and $Z^2$ are each independently O, S, Se, Te, or $CR^aR^b$, wherein $R^a$ and $R^b$ are each independently hydrogen, deuterium, a substituted or unsubstituted C1 to C10 alkyl group, a cyano group, or a cyano-containing group,
$Z^3$ is O, S, Se, Te, or $CR^aR^b$, wherein $R^a$ and $R^b$ are each independently hydrogen, deuterium, a substituted or unsubstituted C1 to C10 alkyl group, a cyano group, or a cyano-containing group,
$R^{11}$ and $R^{12}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* is a linking portion linked to Chemical Formula 1,

[Chemical Formula 5C]

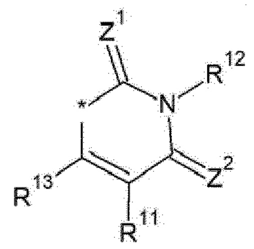

wherein, in Chemical Formula 5C,

$Z^1$ and $Z^2$ are each independently O, S, Se, Te, or $CR^aR^b$, wherein $R^a$ and $R^b$ are each independently hydrogen, deuterium, a substituted or unsubstituted C1 to C10 alkyl group, a cyano group, or a cyano-containing group,
$R^{11}$, $R^{12}$, and $R^{13}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* is a linking portion linked to Chemical Formula 1,

[Chemical Formula 5D]

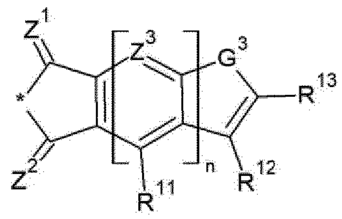

wherein, in Chemical Formula 5D,

$Z^1$ and $Z^2$ are each independently O, S, Se, Te, or $CR^aR^b$, wherein $R^a$ and $R^b$ are each independently hydrogen, deuterium, a substituted or unsubstituted C1 to C10 alkyl group, a cyano group, or a cyano-containing group,
$Z^3$ is N or $CR^c$, wherein $R^c$ is hydrogen or a substituted or unsubstituted C1 to C10 alkyl group,
$G^3$ is O, S, Se, Te, $SiR^xR^y$, or $GeR^zR^w$, wherein $R^x$, $R^y$, $R^z$, and $R^w$ are each independently hydrogen, deuterium, a halogen, a cyano group, a substituted or unsubstituted C1 to C20 alkyl group, or a substituted or unsubstituted C6 to C20 aryl group,
$R^{11}$, $R^{12}$, and $R^{13}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof,
n is 0 or 1, and
* is a linking portion linked to Chemical Formula 1,

[Chemical Formula 5E]

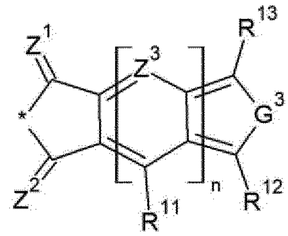

wherein, in Chemical Formula 5E,

$Z^1$ and $Z^2$ are each independently O, S, Se, Te, or $CR^aR^b$, wherein $R^a$ and $R^b$ are each independently hydrogen, deuterium, a substituted or unsubstituted C1 to C10 alkyl group, a cyano group, or a cyano-containing group,
$Z^3$ is N or $CR^c$, wherein $R^c$ is hydrogen or a substituted or unsubstituted C1 to C10 alkyl group,
$G^3$ is O, S, Se, Te, $SiR^xR^y$, or $GeR^zR^w$, wherein $R^x$, $R^y$, $R^z$, and $R^w$ are each independently hydrogen, deuterium, a halogen, a cyano group, a substituted or unsubstituted C1 to C20 alkyl group, or a substituted or unsubstituted C6 to C20 aryl group,
$R^{11}$, $R^{12}$, and $R^{13}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof,
n is 0 or 1, and
* is a linking portion linked to Chemical Formula 1,

[Chemical Formula 5F]

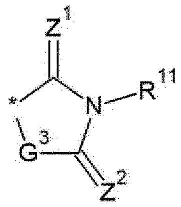

wherein, in Chemical Formula 5F,

$Z^1$ and $Z^2$ are each independently O, S, Se, Te, or $CR^aR^b$, wherein $R^a$ and $R^b$ are each independently hydrogen, deuterium, a substituted or unsubstituted C1 to C10 alkyl group, a cyano group, or a cyano-containing group,
$R^{11}$ is hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), a cyano-containing group, or any combination thereof,
$G^3$ is O, S, Se, Te, $SiR^xR^y$, or $GeR^zR^w$, wherein $R^x$, $R^y$, $R^z$, and $R^w$ are each independently hydrogen, deuterium, a halogen, a cyano group, a substituted or unsubstituted C1 to C20 alkyl group, or a substituted or unsubstituted C6 to C20 aryl group, and
* is a linking portion linked to Chemical Formula 1,

[Chemical Formula 5G]

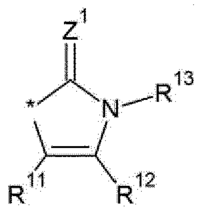

wherein, in Chemical Formula 5G,

$Z^1$ is O, S, Se, Te, or $CR^aR^b$, wherein $R^a$ and $R^b$ are each independently hydrogen, deuterium, a substituted or unsubstituted C1 to C10 alkyl group, a cyano group, or a cyano-containing group,
$R^{11}$, $R^{12}$, and $R^{13}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof, and
* is a linking portion linked to Chemical Formula 1,

[Chemical Formula 5H]

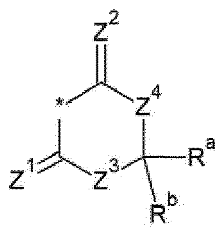

wherein, in Chemical Formula 5H,

$R^a$ and $R^b$ are each independently hydrogen, deuterium, a substituted or unsubstituted C1 to C10 alkyl group, a cyano group, or a cyano-containing group,
$Z^1$ to $Z^4$ are each independently O, S, Se, Te, or $CR^cR^d$, wherein $R^c$ and $R^d$ are each independently hydrogen, deuterium, a substituted or unsubstituted C1 to C10 alkyl group, a cyano group, or a cyano-containing group, and
* is a linking portion linked to Chemical Formula 1.

**8.** The compound of any of claims 1-7, wherein the compound has a polarizability of less than or equal to 450 $Bohr^3$; and/or

wherein the compound has an oscillator strength value of greater than or equal to 0.65; and/or
wherein a dipole moment of the compound is greater than or equal to 5 Debye; and/or
wherein the compound has a maximum absorption wavelength ($\lambda_{max}$) in a wavelength range of 520 nm to 540 nm in a thin film state.

**9.** A photoelectric device, comprising:

a first electrode and a second electrode facing each other, and
a light absorbing layer between the first electrode and the second electrode,
wherein the light absorbing layer includes the compound of any of claims 1-8.

**10.** The photoelectric device of claim 9, wherein

the light absorbing layer includes a p-type semiconductor and an n-type semiconductor,
the p-type semiconductor includes the compound represented by Chemical Formula 1, and
the n-type semiconductor includes fullerene, fullerene derivative, sub-phthalocyanine or a sub-phthalocyanine derivative, thiophene or a thiophene derivative, a compound represented by Chemical Formula 6, or any combination thereof:

[Chemical Formula 6]

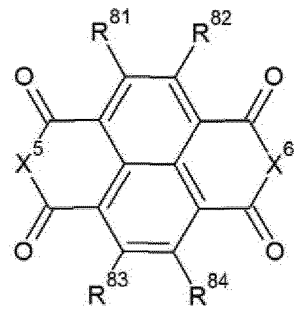

wherein, in Chemical Formula 6,

$X^5$ and $X^6$ are each independently O or $NR^a$, wherein $R^a$ is hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heterocyclic group, a halogen, or a cyano group, and
$R^{81}$ to $R^{84}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heterocyclic group, a halogen, a cyano group, or any combination thereof.

**11.** An image sensor comprising the photoelectric device of claims 9 or 10.

**12.** The image sensor of claim 11, further comprising:

a semiconductor substrate integrated with a plurality of first photo-sensing devices configured to sense light in a blue wavelength region and a plurality of second photo-sensing devices configured to sense light in a red wavelength region,
wherein the photoelectric device is on the semiconductor substrate and is configured to selectively sense light in a green wavelength region.

**13.** The image sensor of claim 12, wherein
each first photo-sensing device of the plurality of first photo-sensing devices is stacked with a separate, respective second photo-sensing device of the plurality of second photo-sensing devices in a vertical direction in the semiconductor substrate.

**14.** The image sensor of any of claims 11-13, wherein

the photoelectric device is a green photoelectric device that is configured to selectively sense light in a green wavelength region, and
the image sensor includes a stack of

the green photoelectric device,
a blue photoelectric device configured to selectively sense light in a blue wavelength region, and
a red photoelectric device configured to selectively sense light in a red wavelength region.

**15.** An electronic device comprising the image sensor of any of claims 11-14.

FIG. 1

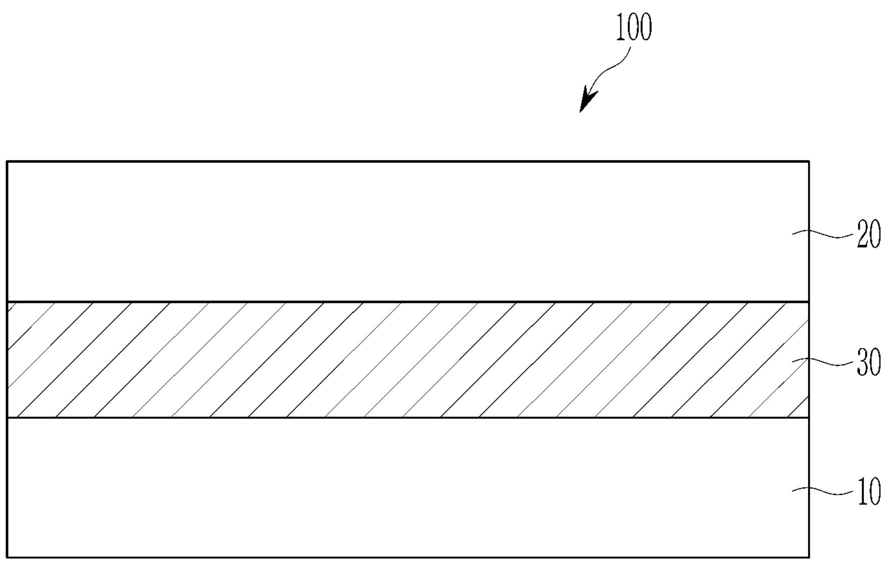

FIG. 2

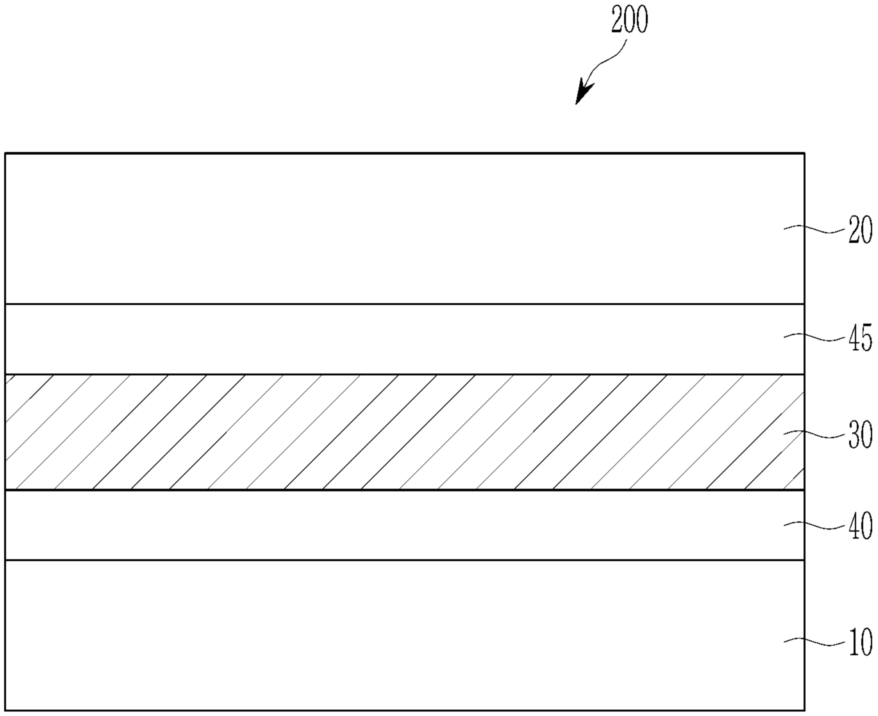

FIG. 3

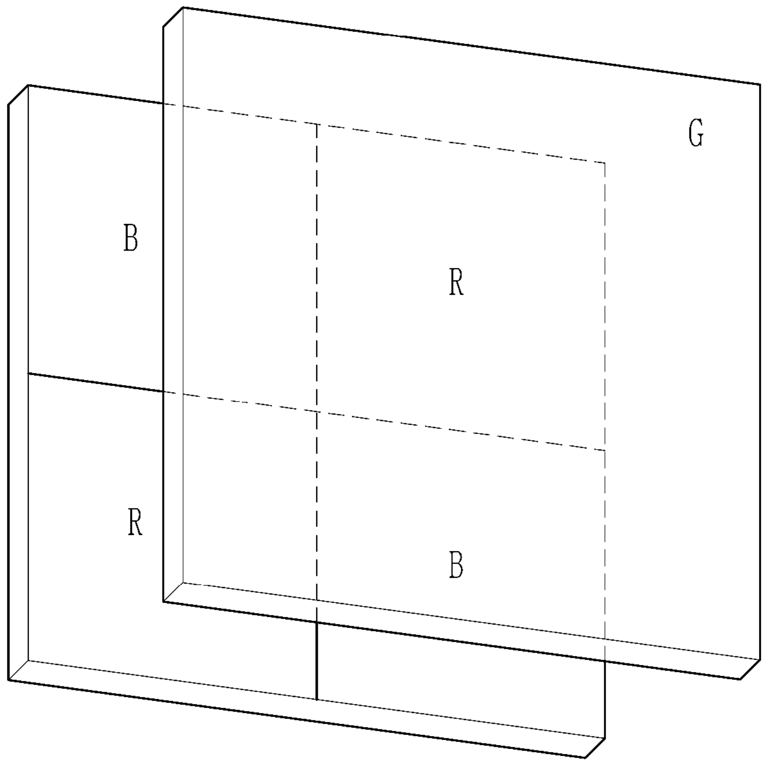

FIG. 4

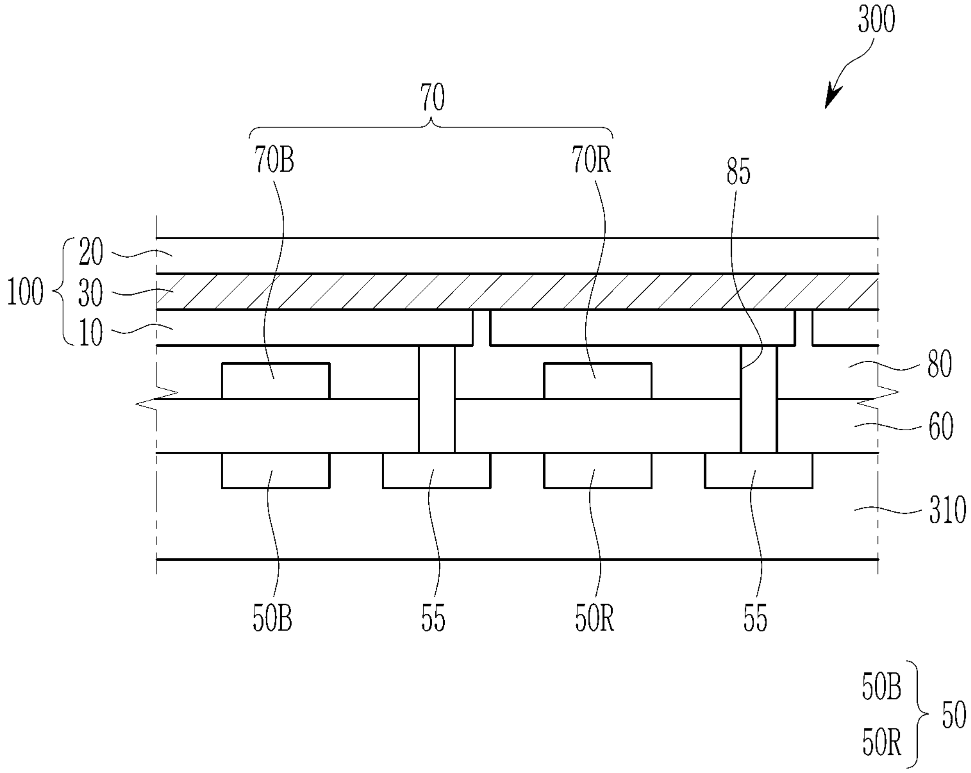

FIG. 5

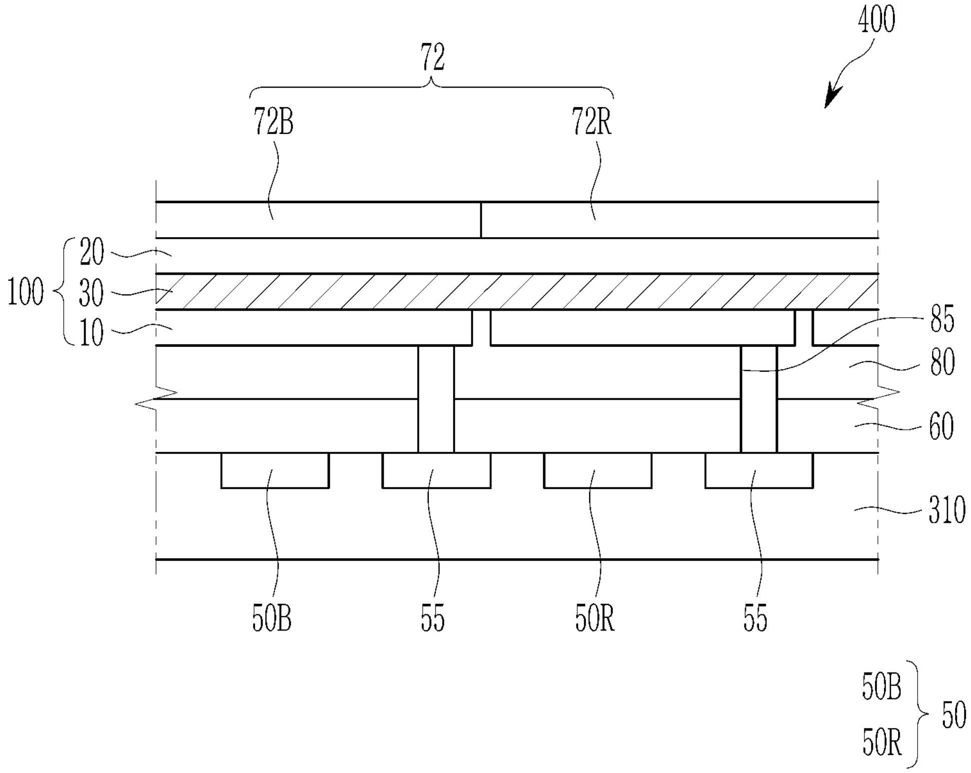

FIG. 6

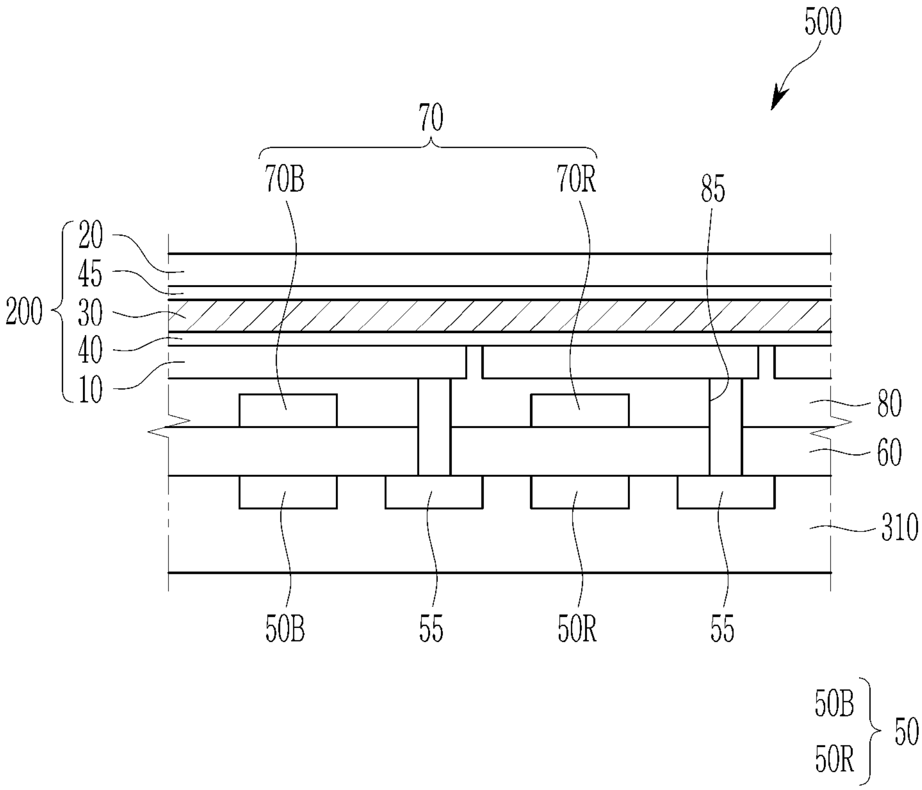
500
70
70B
70R
85
20
45
200
30
40
10
80
60
310
50B
55
50R
55
50B
50R
50

FIG. 7

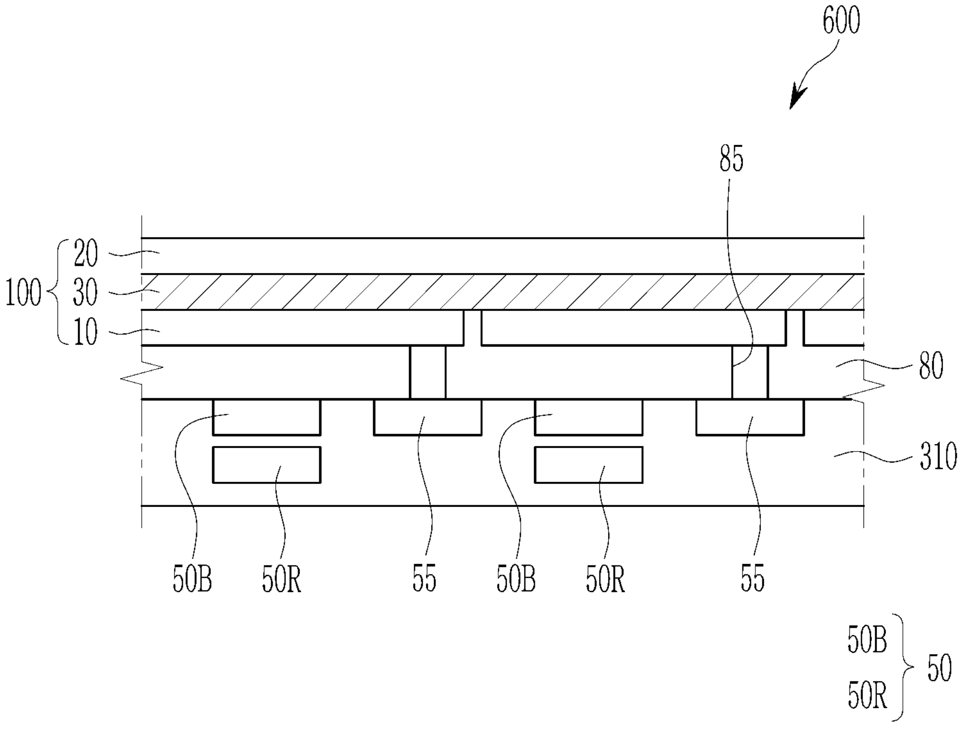

FIG. 8

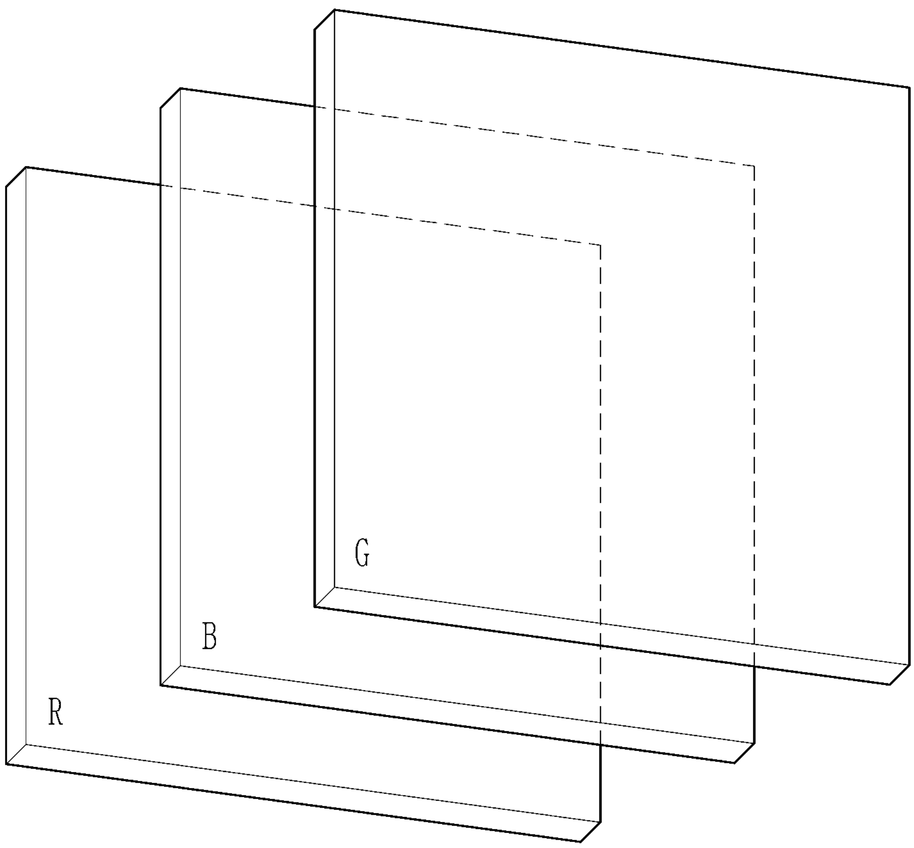

FIG. 9

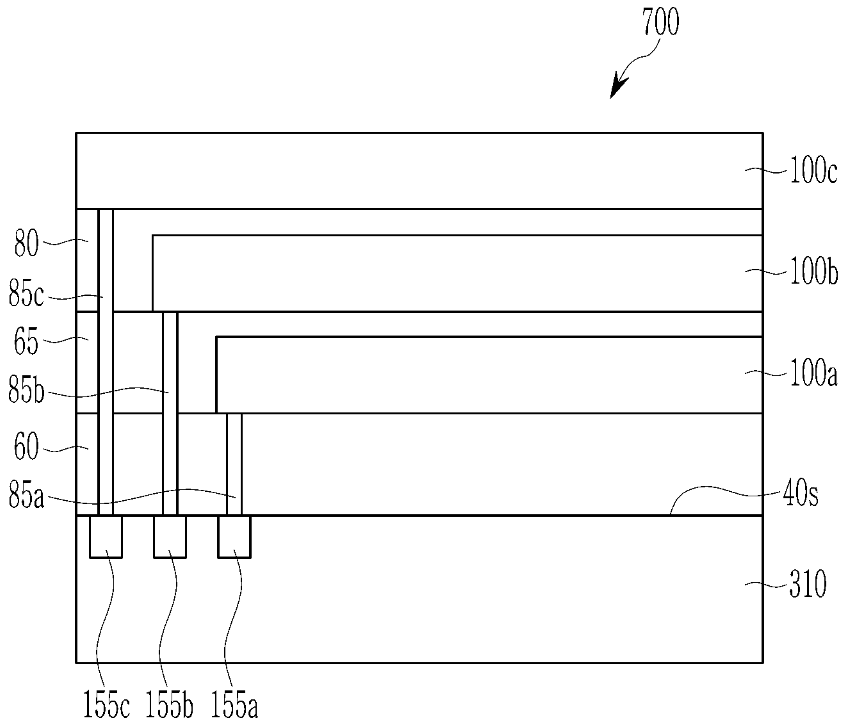

FIG. 10

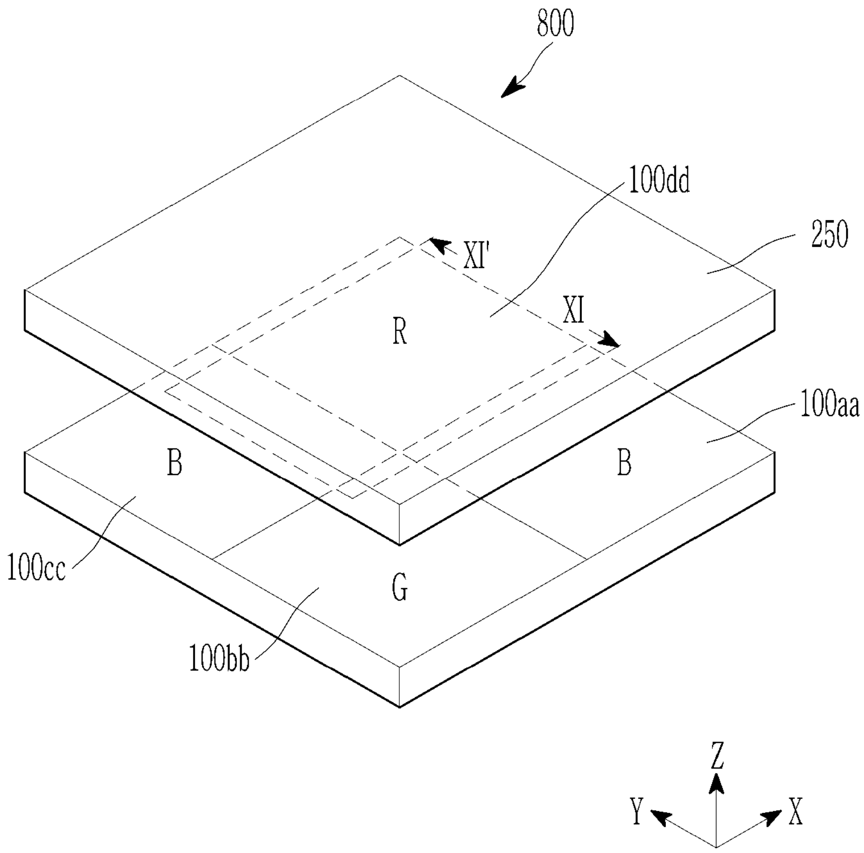

FIG. 11

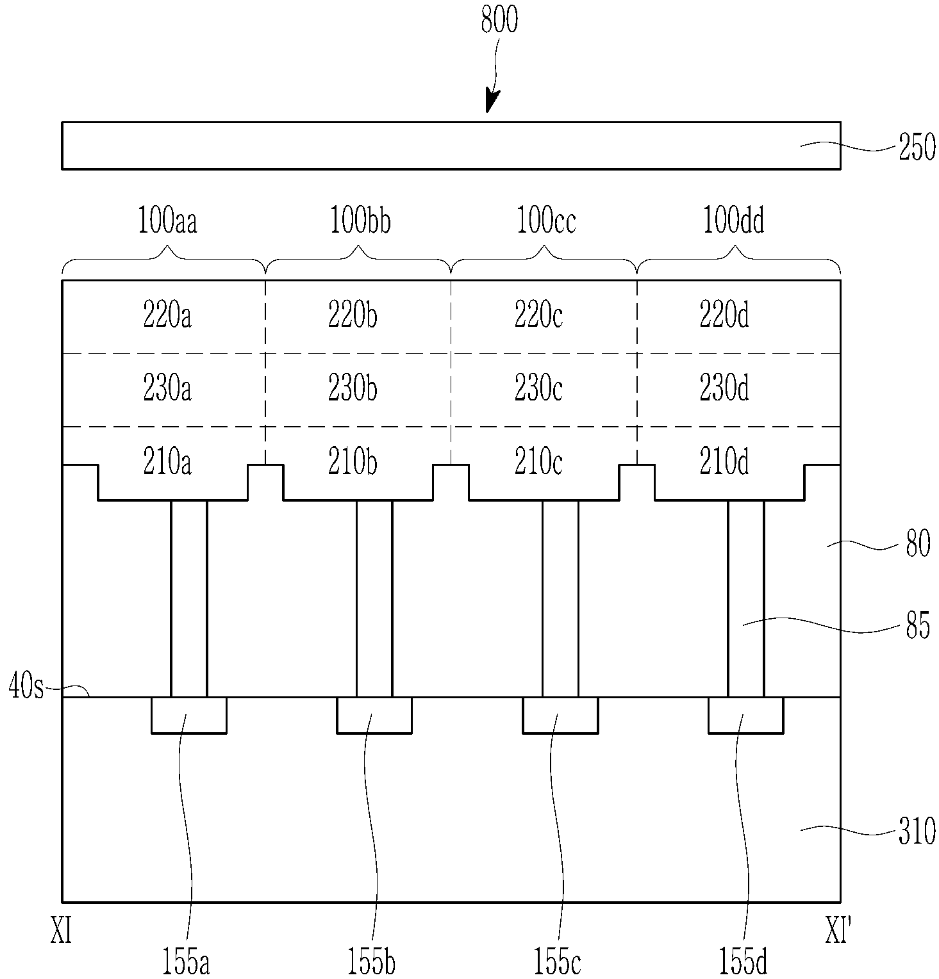
800
250
100aa
100bb
100cc
100dd
220a
220b
220c
220d
230a
230b
230c
230d
210a
210b
210c
210d
80
85
40s
310
XI
XI'
155a
155b
155c
155d

FIG. 12

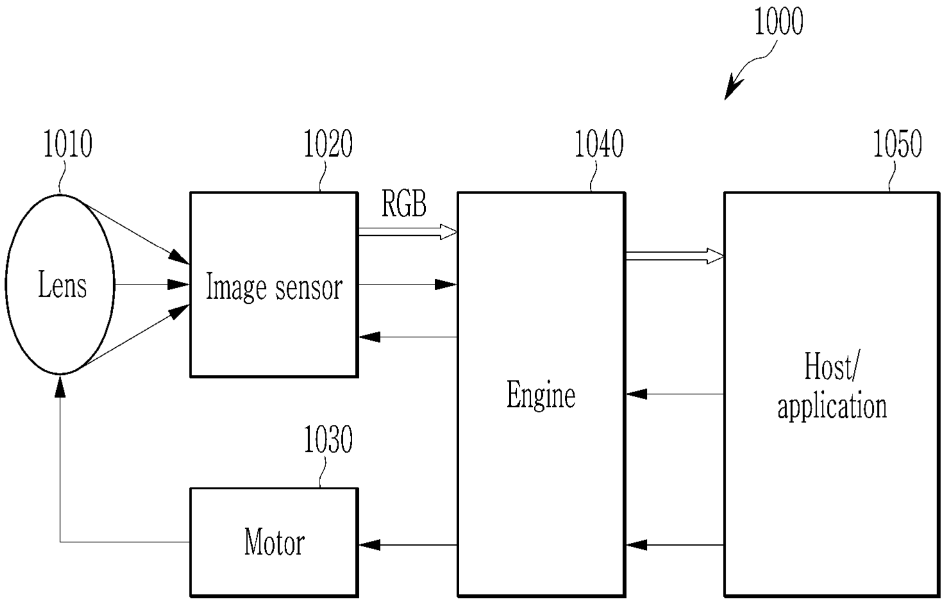
1000
1010
Lens
1020
Image sensor
RGB
1040
Engine
1050
Host/
application
1030
Motor

FIG. 13

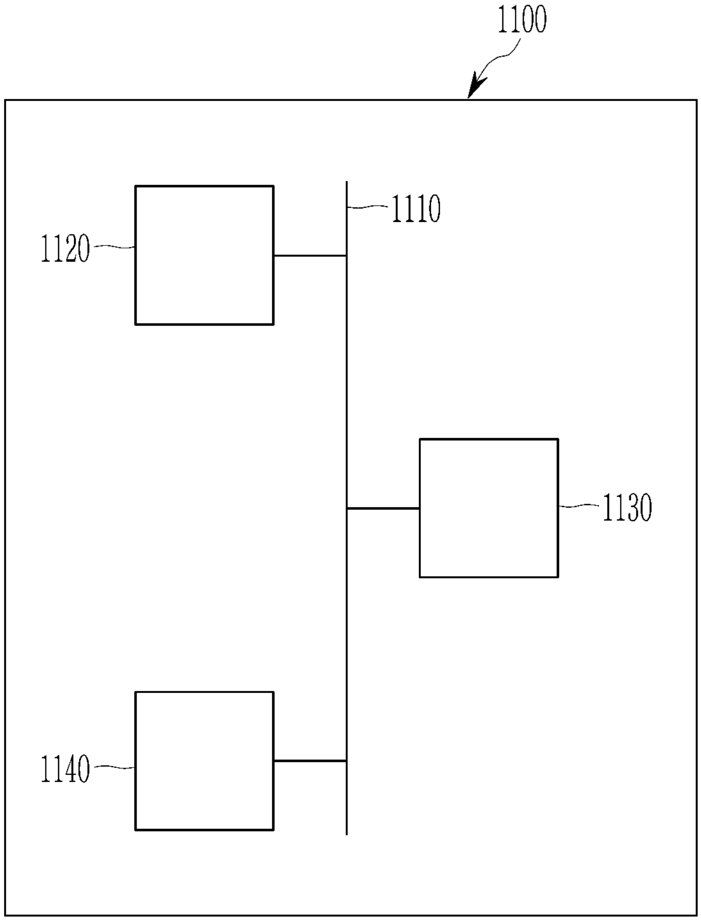

Europäisches Patentamt
European Patent Office
Office européen des brevets

# EUROPEAN SEARCH REPORT

Application Number
EP 24 17 2844

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 024 484 A1 (SAMSUNG ELECTRONICS CO LTD [KR]) 6 July 2022 (2022-07-06)<br>* paragraph [0352] to paragraph [0366], compounds I-1 to I-4 *<br>* paragraph [0384] to paragraph [0403], examples 1 to 4, evaluation 1 to 6 ; tables 1 to 7 *<br>* claims 1 to 15 *<br>----- | 1-15 | INV.<br>C07D495/04<br>H10K30/30<br>H10K39/32<br>H10K85/60 |
| X,P | WO 2023/247338 A1 (MERCK PATENT GMBH [DE]) 28 December 2023 (2023-12-28)<br>* page 66 to page 161, examples, compounds, tables 1 to 5 *<br>* claims 1 to 15 *<br>----- | 1-15 | |
| E | WO 2024/172295 A1 (DUK SAN NEOLUX CO LTD [KR]; SAMSUNG DISPLAY CO LTD [KR]) 22 August 2024 (2024-08-22)<br>* examples, compounds, tables *<br>* claims 1 to 13 *<br>----- | 1-15 | |
| | | | TECHNICAL FIELDS SEARCHED (IPC) |
| | | | C07D<br>H10K |

The present search report has been drawn up for all claims

3

| Place of search | Date of completion of the search | Examiner |
|---|---|---|
| Munich | 23 September 2024 | Cortés Suárez, José |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 24 17 2844

This annex lists the patent family members relating to the patent documents cited in the above-mentioned European search report. The members are as contained in the European Patent Office EDP file on
The European Patent Office is in no way liable for these particulars which are merely given for the purpose of information.

23-09-2024

| Patent document cited in search report | Publication date | Patent family member(s) | Publication date |
|---|---|---|---|
| EP 4024484 A1 | 06-07-2022 | CN 114716457 A | 08-07-2022 |
| | | EP 4024484 A1 | 06-07-2022 |
| | | KR 20220098602 A | 12-07-2022 |
| | | US 2022220127 A1 | 14-07-2022 |
| WO 2023247338 A1 | 28-12-2023 | NONE | |
| WO 2024172295 A1 | 22-08-2024 | KR 20240126879 A | 22-08-2024 |
| | | WO 2024172295 A1 | 22-08-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **JUHA ALAKARHU'S.** Image Sensors and Image Quality in Mobile Phones. *International Image Sensor Workshop (Ogunquit Maine, USA,* 2007